# EUROPEAN PATENT SPECIFICATION

(11) **EP 2 167 469 B1**
(45) Date of publication and mention of the grant of the patent: **15.08.2012**
(21) Application number: 08762671.9
(22) Date of filing: 17.06.2008
(51) Int. Cl.: C07D 215/36, C07D 409/12, A61K 31/4706, A61K 31/4709, A61P 25/00

(54) **SULFONYL-QUINOLINE DERIVATIVES**
SULFONYLCHINOLINDERIVATE
DÉRIVÉS DE LA SULFONYLE QUINOLÉINE

(30) Priority: 18.06.2007 HU 0700417; 12.06.2008 HU 0800376
(43) Date of publication of application: 31.03.2010
(73) Proprietor: Richter Gedeon Nyrt., 1103 Budapest (HU)
(72) Inventor: GALAMBOS, János, H-1162 Budapest (HU); KESERÜ, György, H-2089 Telki (HU); GÁL, Krisztina, H-1193 Budapest (HU); BOBOK, Amrita, Ágnes, H-1025 Budapest (HU); WÉBER, Csaba, H-1142 Budapest (HU); PRAUDA, lbolya, H-1031 Budapest (HU); WÁGNER, Gábor, András, H-1107 Budapest (HU); VASTAG, Mónika, H-1025 Budapest (HU)
(74) Representative: HOFFMANN EITLE
(86) International application number: PCT/HU2008/000068
(87) International publication number: WO 2008/155588

(56) References cited:
- WO-A-02/28837
- WO-A-03/080580
- WO-A-2005/058834
- WO-A-2007/072093

## Description

### FIELD OF THE INVENTION

The present invention relates to new mGluR1 and mGluR5 receptor subtype preferring ligands of formula (I) and/or salts and/or hydrates and/or solvates thereof, to the processes for their preparation, to pharmaceutical compositions containing these compounds and to their use in therapy and/or prevention of a condition which requires modulation of mGluR1 and mGluR5 receptors.

### BACKGROUND OF THE INVENTION

A major excitatory neurotransmitter in the mammalian central nervous system (CNS) is the glutamate molecule, which binds to neurons, thereby activating cell surface receptors. These receptors can be divided into two major classes, ionotropic and metabotropic glutamate receptors, based on the structural features of the receptor proteins, the means by which the receptors transduce signals into the cell, and pharmacological profiles.

The metabotropic glutamate receptors (mGluRs) are G protein-coupled receptors that activate a variety of intracellular second messenger systems following the binding of glutamate. Activation of mGluRs in intact mammalian neurons elicits one or more of the following responses: activation of phospholipase C; increases in phosphoinositide (PI) hydrolysis; intracellular calcium release; activation of phospholipase D; activation or inhibition of adenyl cyclase; increases or decreases in the formation of cyclic adenosine monophosphate (cAMP); activation of guanylyl cyclase; increases in the formation of cyclic guanosine monophosphate (cGMP); activation of phospholipase A2; increases in arachidonic acid release; and increases or decreases in the activity of voltage- and ligand-gated ion channels. (Trends Pharmacol. Sci., 1993, 14, 13; Neurochem. Int., 1994, 24, 439; Neuropharmacology, 1995, 34, 1; Prog. Neurobiol., 1999, 59, 55).

Eight distinct mGluR subtypes, termed mGluR1 through mGluR8, have been identified by molecular cloning *(*Neuron, 1994, 13, 1031; Neuropharmacology, 1995, 34, 1; J. Med. Chem., 1995, 38, 1417). Further receptor diversity occurs via expression of alternatively spliced forms of certain mGluR subtypes (PNAS, 1992, 89, 10331; BBRC,1994, 199,1136; J. Neurosci.,1995, 15, 3970).

Metabotropic glutamate receptor subtypes may be subdivided into three groups, Group I, Group II, and Group III mGluRs, based on amino acid sequence homology, the second messenger systems utilized by the receptors, and by their pharmacological characteristics. Group I mGluR comprises mGluRl, mGluR5 and their alternatively spliced variants.

Attempts at elucidating the physiological roles of Group I mGluRs suggest that activation of these receptors elicits neuronal excitation. Evidence indicates that this excitation is due to direct activation of postsynaptic mGluRs, but it also has been suggested that activation of presynaptic mGluRs occurs, resulting in increased neurotransmitter release *(*Trends Pharmacol. Sci., 1992, 15, 92; Neurochem. Int., 1994, 24, 439; Neuropharmacology, 1995, 34, 1; Trends Pharmacol. Sci., 1994, 15, 33).

Metabotropic glutamate receptors have been implicated in a number of normal processes in the mammalian CNS. Activation of mGluRs has been shown to be required for induction of hippocampal long-term potentiation and cerebellar long-term depression *(*Nature, 1993, 363, 347; Nature, 1994, 368, 740; Cell, 1994, 79, 365; Cell, 1994, 79, 377). A role for mGluR activation in nociception and analgesia also has been demonstrated (Neuroreport, 1993, 4, 879; Brain Res., 1999, 871, 223).

Group I metabotropic glutamate receptors and mGluR5 in particular, have been suggested to play roles in a variety of pathophysiological processes and disorders affecting the CNS. These include stroke, head trauma, anoxic and ischemic injuries, hypoglycemia, epilepsy, neurodegenerative disorders such as Alzheimer's disease, acute and chronic pain, substance abuse and withdrawal, obesity and gastroesophageal reflux disease (GERD) and irritable bowel syndrome *(*Trends Pharmacol. Sci., 1993, 14, 13; Life Sci., 1994, 54, 135; Ann. Rev. Neurosci., 1994, 17, 31; Neuropharmacology, 1995, 34, 1; J. Med. Chem., 1995, 38, 1417; Trends Pharmacol. Sci., 2001, 22, 331; Curr. Opin. Pharmacol., 2002, 2, 43; Pain, 2002, 98, 1, Curr Top Med Chem., 2005; 5(9):897-911). Much of the pathology in these conditions is thought to be due to excessive glutamate-induced excitation of CNS neurons. As Group I mGluRs appear to increase glutamate-mediated neuronal excitation via postsynaptic mechanisms and enhanced presynaptic glutamate release, their activation probably contributes to the pathology. Accordingly, selective antagonists of Group I mGluR receptors could be therapeutically beneficial, specifically as neuroprotective agents, analgesics or anticonvulsants.

WO 2006 120573 relates to a method for inhibiting the proliferation of cancer cells in mammal, comprising administering to said mammal a therapeutically effective amount of heterocyclic mono-N-oxides, among them N-oxides of compounds of formula (I) of the present invention.

The preparation of quinolines was stated by the application of general method that can be found in WO 2005 070890, J. Med. Chem,, 2003, 46, 49 and J. Med. Chem., 2005, 48, 1107. In the citated publications 4-amino-3-cyano-quinoline derivatives are prepared by condensation of anilines with 2-cyano-3-ethoxy-acrylic acid ethyl ester followed by the ring closure of the obtained intermediates. Thermical ring closure afforded 3-cyano-4-hydroxyquinoline derivatives that was converted into 3-cyano-4-chloro-quinoline derivatives by phosphorous(V) oxychloride. Ring closure with phosphorous(V) oxyhalogenides directly resulted in 3-cyano-4-halogen-quinoline derivatives. From these intermediates no 3-arylsulfonyl-4-aryl-quinoline derivatives of formula (I) of the present invention were prepared or chracterized by physical properties either in WO 2006 120573 or in the cited publications.

WO 2005 58834 provides novel quinoline derivatives for use in treating liver X receptor (LXR) mediated diseases particularly multiple sclerosis, rheumatoid arthritis, inflammatory bowel disease and atherosclerosis, which compounds suppress Th-1 type lymphokine production, resulting in increased HDL levels, and cholesterol metabolism. The general formula of WO 2005058834 covers some of the compounds of formula (I) of the present invention, but only 3-benzenesulfonyl-4-phenyl-8-trifluoromethyl-quinoline was prepared by the reaction of the appropriate aniline derivative (described in Scheme 9 of WO 2005 58834) with 1,2-bis(benzenesulfonyl)-ethylen. This compound is not covered by the general formula of the present invention and proved to be inactive on mGluRl and mGluR5 receptors.

WO 2005 30129 relates to compounds useful as potassium channel inhibitors. Compounds in this class may be useful as Kv1.5 antagonists for treating and preventing cardiac arrhythmias, and the like, and as Kv1.3 inhibitors for treatment of immunosuppression, autoimmune diseases, and the like. The general formula of WO 2005 30129 covers some of the compounds of formula (I) of the present invention, but no 3-arylsulfonyl-4-aryl-quinoline derivatives of formula (I) of the present invention only 2-substitutcd-6-methoxy-quinolin-3-carbonitrile derivatives were prepared or chracterized by physical properties.

The compounds mentioned in the above publications are not declared or even not suggested having activity on the mGluR1 and mGluR5 receptors.

Quinoline derivates useful in the treatment of mGluR5 receptor-mediated disorders are described in WO 2007/072093 A1. The use of quinoline derivatives as 5-HT6 ligands is described in WO 2003/080580 A3. WO 02/28837 A1 relates to metabotropic glutamate receptor antagonists.

### SUMMARY OF THE INVENTION

The present invention relates to new mGluR1 and mGluR5 receptor subtype preferring ligands of formula (I): 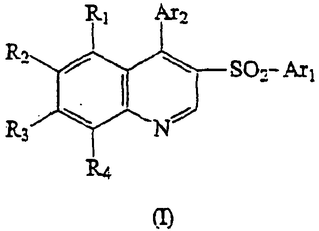 wherein
Ar₁ represents phenyl or thienyl group, optionally substituted with one or more substituent(s) selected from hydrogen, fluoro, chloro, cyano, methyl, methoxy;
Ar₂ represents phenyl, substituted with one or more substituent(s) selected from fluoro, chloro, cyano, methyl, methoxy;
R₁, R₂, R₃ and R₄ represent independently a substituent selected from hydrogen, fluoro, chloro, cyano, methyl, methoxy, hydroxy, trifluoromethyl, amino, methylamino, dimethylamino, aminomethyl, methylaminomethyl, dimethylaminomethyl,
and/or salts and/or hydrates and/or solvates thereof, to the processes for producing the same, to pharmaceutical compositions containing the same and to their use in therapy and/or prevention of pathological conditions which require the modulation of mGluR1 and mGluR5 receptors such as neurological disorders, psychiatric disorders, acute and chronic pain and neuromuscular dysfunctions of the lower urinary tract.

### DETAILED DESCRIPTION OF THE INVENTION

The present invention relates to new mGluR1 and mGluR5 receptor subtype preferring ligands of formula (I): 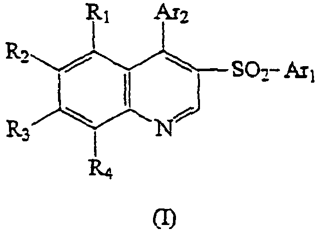 wherein
Ar₁ represents phenyl or thienyl group optionally substituted with one or more substituent(s) selected from hydrogen, fluoro, chloro, cyano, methyl, methoxy;
Ar₂ represents a substituted phenyl group;
R₁, R₂, R₃ and R₄ represent independently a substituent selected from hydrogen, fluoro, chloro, cyano, methyl, methoxy, hydroxy, trifluoromethyl, amino, methylamino, dimethylamino, aminomethyl, methylaminomethyl, dimethylaminomethyl,
and/or salts and/or hydrates and/or solvates thereof.

The term "halogen" includes fluorine, chlorine, bromine and iodine atoms.

In this specification the term "halo" may be fluoro, chloro, bromo or iodo. Compounds of formula (I) contain basic function(s) so may form salts with acids. The invention relates also to the salts of compounds of formula (I) formed with acids, especially the salts formed with pharmaceutically acceptable acids. The meaning of compound of formula (I) is either the free base or the salt even if it is not referred separately.

Both organic and inorganic acids can be used for the formation of acid addition salts.

Suitable inorganic acids can be for example hydrochloric acid, sulfuric acid, nitric acid and phosphoric acid. Representatives of monovalent organic acids can be for example formic acid, acetic acid, propionic acid, and different butyric acids, valeric acids and capric acids. Representatives of bivalent organic acids can be for example oxalic acid, malonic acid, maleic acid, fumaric acid and succinic acid. Other organic acids can also be used, such as hydroxy acids for example citric acid, tartaric acid, or aromatic carboxylic acids for example benzoic acid or salicylic acid, as well as aliphatic and aromatic sulfonic acids for example methanesulfonic acid, naphthalenesulfonic acid and p-toluenesulfonic acid. Especially valuable group of the acid addition salts is in which the acid component itself is physiologically acceptable and does not have therapeutical effect in the applied dose or it does not have unfavourable influence on the effect of the active ingredient These acid addition salts are pharmaceutically acceptable acid addition salts. The reason why acid addition salts, which do not belong to the pharmaceutically acceptable acid addition salts belong to the present invention is, that in given case they can be advantageous in the purification and isolation of the desired compounds.

Solvates and/or hydrates of compounds of formula (I) are also included within the scope of the invention.

The compounds of the invention are those compounds of formula (I),
wherein
Ar₁ represents phenyl or thienyl, optionally substituted with one or more substituent(s) selected from hydrogen, fluoro, chloro, cyano, methyl, methoxy;
Ar₂ represents phenyl, substituted with one or more substituent(s) selected from fluoro, chloro, cyano, methyl, methoxy,
R₁, R₂, R₃ and R₄ represent independently a substituent selected from hydrogen, fluoro, chloro, cyano, methyl, methoxy, hydroxy, trifluoromethyl, amino, methylamino, dimethylamino, aminomethyl, methylaminomethyl, dimethylaminomethyl,
and/or salts and/or hydrates and/or solvates thereof.

Especially important compounds of formula (I) of the present invention are the following:
4-(4-chloro-phenyl)-3-(4-methyl-benzenesulfonyl)-quinoline,
7-chloro-3-(4-chloro-benzenesulfonyl)-4-(4-fluoro-phenyl)-quinoline,
8-chloro-4-(3-chloro-phenyl)-3-(3,4-dichloro-benzenesulfonyl)-quinoline,
7-fluoro-3-(4-fluoro-benzenesulfonyl)-4-(3-fluoro-phenyl)-quinoline,
4-(4-chloro-phenyl)-7-fluoro-3-(4-methoxy-benzenesulfonyl)-quinoline,
7-fluoro-3-(4-methoxy-benzenesulfonyl)-4-(4-methoxy-phenyl)-quinoline,
7-fluoro-3-(3-fluoro-benzenesulfonyl)-4-(3-fluoro-phenyl)-quinoline,
7-fluoro-3-(3-fluoro-benzenesulfonyl)-4-(4-fluoro-phenyl)-quinoline,
4-(3-chloro-phenyl)-3-(3,4-dimethyl-benzenesulfonyl)-7-fluoro-quinoline,
3-(3,4-dimethyl-benzenesulfonyl)-7-fluoro-4-(3-fluoro-phenyl)-quinoline,
3-(3,4-dimethyl-benzenesulfonyl)-7-fluoro-4-(3-methoxy-phenyl)-quinoline,
4-(3-chloro-phenyl)-8-fluoro-3-(4-fluoro-benzenesulfonyl)-quinoline,
4-(4-chloro-phenyl)-8-fluoro-3-(4-fluoro-benzenesulfonyl)-quinoline,
8-fluoro-3-(4-fluoro-benzenesulfonyl)-4-(3-fluoro-phenyl)-quinoline,
8-fluoro-3-(4-fluoro-benzenesulfonyl)-4-(3-methoxy-phenyl)-quinoline,
4-(4-chloro-phenyl)-6-fluoro-3-(4-methoxy-benzenesulfonyl)-quinoline,
4-(4-chloro-phenyl)-3-(3,4-dimethyl-benzenesulfonyl)-6-fluoro-quinoline,
4-(4-chloro-phenyl)-3-(3,5-difluoro-benzenesulfonyl)-7-fluoro-quinoline,
3-(3,5-difluoro-benzenesulfonyl)-7-fluoro-4-(4-fluoro-phenyl)-quinoline,
4-(4-chloro-phenyl)-3-(3-cyano-benzenesulfonyl)-7-fluoro-quinoline,
3-(3-cyano-benzenesulfonyl)-7-fluoro-4-(3-fluoro-phenyl)-quinoline,
3-(3-cyano-benzenesulfonyl)-7-fluoro-4-(4-fluoro-phenyl)-quinoline,
7-fluoro-3-(4-fluoro-benzenesulfonyl)-4-(4-fluoro-phenyl)-quinoline,
4-(4-chloro-phenyl)-7-fluoro-3-(3-fluoro-benzenesulfonyl)-quinoline,
4-(3-chloro-phenyl)-7-fluoro-3-(3-methoxy-benzenesulfonyl)-quinoline,
7-fluoro-4-(4-fluoro-phenyl)-3-(3-methoxy-benzenesulfonyl)-quinoline,
4-(4-chloro-phenyl)-3-(3,4-dimethyl-benzenesulfonyl)-7-fluoro-quinoline,
3-(3,4-dimethyl-benzenesulfonyl)-7-fluoro-4-(4-fluoro-phenyl)-quinoline,
4-(3-chloro-phenyl)-3-(3-chloro-4-methoxy-benzenesulfonyl)-7-fluoro-quinoline,
3-(3-chloro-4-methoxy-benzenesulfonyl)-7-fluoro-4-(3-fluoro-phenyl)-quinoline,
3-(3-chloro-4-methoxy-benzenesulfonyl)-7-fluoro-4-(4-fluoro-phenyl)-quinoline,
3-(3,5-dichloro-benzenesulfonyl)-8-fluoro-4-(4-fluoro-phenyl)-quinoline,
3-(3,5-difluoro-benzenesulfonyl)-8-fluoro-4-(3-fluoro-phenyl)-quinoline,
8-fluoro-3-(4-fluoro-benzenesulfonyl)-4-(4-methoxy phenyl)-quinoline,
4-(3-chloro-phenyl)-8-fluoro-3-(4-methoxy-benzenesulfonyl)-quinoline,
4-(4-chloro-phenyl)-8-fluoro-3-(4-methoxy-benzenesulfonyl)-quinoline,
8-fluoro-4-(4-fluoro-phenyl)-3-(4-methoxy-benzenesulfonyl)-quinoline,
8-fluoro-3-(4-methoxy-benzenesulfonyl)-4-(4-methoxy-phenyl)-quinoline,
8-ffuoro-3-(4-methoxy-benzenesulfonyl)-4-(3-methoxy-phenyl)-quinoline,
4-(3-chloro-phenyl)-8-fluoro-3-(3-fluoro-benzenesulfonyl)-quinoline,
4-(4-chloro-phenyl)-8-fluoro-3-(3-fluoro-benzenesulfonyl)-quinoline,
8-ffuoro-3-(3-fluoro-benzenesulfonyl)-4-(3-fluoro-phenyl)-quinoline,
8-fluoro-3-(3-fluoro-benzenesulfonyl)-4-(4-methoxy-phenyl)-quinoline,
8-fluoro-3-(3-fluoro-benzenesulfonyl)-4-(3 -methoxy-phenyl)-quinoline,
8-fluoro-3-(3-fluoro-benzenesulfonyl)-4-(4-fluoro-phenyl)-quinoline,
3-(3-cyano-benzenesulfonyl)-6-fluoro-4-(3-fluoro-phenyl)-quinoline,
3-(3-cyano-benzenesulfonyl)-6-fluoro-4-(3-methoxy-phenyl)-quinoline,
3-(3-cyano-benzenesulfonyl)-6-fluoro-4-(4-fluoro-phenyl)-quinoline,
4-(3-chloro-phenyl)-6-fluoro-3-(3-methoxy-benzenesulfonyl)-quinoline,
6-fluoro-4-(4-fluoro-phenyl)-3-(3-methoxy-benzenesulfonyl)-quinoline,
3-(3-chloro-4-methoxy-benzenesulfonyl)-6-fluoro-4-(4-methoxy-phenyl)-quinoline,
3-(3-chloro-4-methoxy-benzenesulfonyl)-6-fluoro-4-(3-methoxy-phenyl)-quinoline,
4-(4-chloro-phenyl)-7-fluoro-3-(4-fluoro-benzenesulfonyl)-quinoline,
3-(3,4-dimethyl-benzenesulfonyl)-7-fluoro-4-(4-methoxy-phenyl)-quinoline,
3-(3-chloro-4-methoxy-benzenesulfonyl)-4-(4-chloro-phenyl)-6-fluoro-quinoline,
3-(3-chloro-4-methoxy-benzenesulfonyl)-6-fluoro-4-(3-fluoro-phenyl)-quinoline,
3-(3,5-dichloro-benzenesulfonyl)-7-fluoro-4-(4-methoxy-phenyl)-quinoline,
3-(3,5-dichloro-benzenesulfonyl)-7-fluoro-4-(3-methoxy-phenyl)-quinoline,
3-(3,5-difluoro-benzenesulfonyl)-7-fluoro-4-(4-fluoro-phenyl)-quinoline,
4-(3-chloro-phenyl)-7-fluoro-3-(3-fluoro-4-methyl-benzenesulfonyl)-quinoline,
7-fluoro-3-(3-fluoro-4-methyl-benzenesulfonyl)-4-(3-fluoro-phenyl)-quinoline,
7-fluoro-3-(3-fluoro-4-methyl-benzenesulfonyl)-4-(4-fluoro-phenyl)-quinoline,
3-(3, 5-difluoro-benzenesulfonyl)-7-fluoro-4-(3-fluoro-phenyl)-quinoline,
3-(3,5-difluoro-benzenesulfonyl)-7-fluoro-4-(4-methoxy-phenyl)-quinoline,
3-(3,5-difluoro-benzenesulfonyl)-7-fluoro-4-(3-methoxy-phenyl)-quinoline,
4-(3-chloro-phenyl)-3-(3-cyano-benzenesulfonyl)-7-fluoro-quinoline,
3-(3-cyano-benzenesulfonyl)-7-fluoro-4-(4-methoxy-phenyl)-quinoline,
3-(3-cyano-benzenesulfonyl)-7-fluoro-4-(3-methoxy-phenyl)-quinoline,
3-(3-chloro-4-methyl-benzenesulfonyl)-7-fluoro-4-(3-fluoro-phenyl)-quinoline,
3-(3,4-difluoro-benzenesulfonyl)-7-fluoro-4-(3-fluoro-phenyl)-quinoline,
3-(3,4-difluoro-benzenesulfonyl)-7-fluoro-4-(4-methoxy-phenyl)-quinoline,
3-(3,4-difluoro-benzenesulfonyl)-7-fluoro-4-(3-methoxy-phenyl)-quinoline,
3-(3,4-difluoro-benzenesulfonyl)-7-fluoro-4-(4-fluoro-phenyl)-quinoline,
3-(3-chloro-4-fluoro-benzenesulfonyl)-4-(4-chloro-phenyl)-7-fluoro-quinoline,
3-(3-chloro-4-fluoro-benzenesulfonyl)-7-fluoro-4-(3-fluoro-phenyl)-quinoline,
3-(3-chloro-4-fluoro-benzenesulfonyl)-7-fluoro-4-(4-methoxy-phenyl)-quinoline,
3-(3-chloro-4-fluoro-benzenesulfonyl)-7-fluoro-4-(4-fluoro-phenyl)-quinoline,
3-(3,5-dichloro-benzenesulfonyl)-8-fluoro-4-(3-fluoro-phenyl)-quinoline,
3-(3,5-dichloro-benzenesulfonyl)-8-fluoro-4-(4-methoxy-phenyl)-quinoline,
3-(3,5-dichloro-benzenesulfonyl)-8-fluoro-4-(3-methoxy-phenyl)-quinoline,
4-(3-chloro-phenyl)-8-fluoro-3-(3-fluoro-4-methyl-benzenesulfonyl)-quinoline,
4-(4-chloro-phenyl)-8-fluoro-3-(3-fluoro-4-methyl-benzenesulfonyl)-quinoline,
8-ffuoro-3-(3-fluoro-4-methyl-benzenesulfonyl)-4-(3-fluoro-phenyl)-quinoline,
8-fluoro-3-(3-fluoro-4-methyl-benzenesulfonyl)-4-(4-methoxy-phenyl)-quinoline,
8-fluoro-3-(3-fluoro-4-methyl-benzenesulfonyl)-4-(3-methoxy-phenyl)-quinoline,
8-fluoro-3-(3-fluoro-4-methyl-benzenesulfonyl)-4-(4-fluoro-phenyl)-quinoline,
4-(3-chloro-phenyl)-3-(3,5-difluoro-benzenesulfonyl)-8-fluoro-quinoline,
4-(4-chloro-phenyl)-3-(3,5-difluoro-benzenesulfonyl)-8-fluoro-quinoline,
3-(3,5-difluoro-benzenesulfonyl)-8-fluoro-4-(4-methoxy-phenyl)-quinoline,
3-(3,5-difluoro-benzenesulfonyl)-8-fluoro-4-(3-methoxy-phenyl)-quinoline,
3-(3,5-difluoro-benzenesulfonyl)-8-fluoro-4-(4-fluoro-phenyl)-quinoline,
4-(4-chloro-phenyl)-3-(3,4-difluoro-benzenesulfonyl)-8-fluoro-quinoline,
3-(3,4-difluoro-benzenesulfonyl)-8-fluoro-4-(3-fluoro-phenyl)-quinoline,
3-(3,4-difluoro-benzenesulfonyl)-8-fluoro-4-(4-methoxy-phenyl)-quinoline,
3-(3,4-difluoro-benzenesulfonyl)-8-fluoro-4-(4-fluoro-phenyl)-quinoline,
3-(3,5-dichloro-benzenesulfonyl)-6-fluoro-4-(3-fluoro-phenyl)-quinoline,
3-(3,5-dichloro-benzenesulfonyl)-6-fluoro-4-(4-methoxy-phenyl)-quinoline,
3-(3,5-dichloro-benzenesulfonyl)-6-fluoro-4-(3-methoxy-phenyl)-quinoline,
4-(3-chloro-phenyl)-6-fluoro-3-(3-fluoro-4-methyl-benzenesulfonyl)-quinoline,
6-fluoro-3-(3-fluoro-4-methyl-benzenesulfonyl)-4-(3-fluoro-phenyl)-quinoline,
6-fluoro-3-(3-fluoro-4-methyl-benzenesulfonyl)-4-(4-methoxy-phenyl)-quinoline,
6-fluoro-3-(3-fluoro-4-methyl-benzenesulfonyl)-4-(3-methoxy-phenyl)-quinoline,
6-fluoro-3-(3-fluoro-4-methyl-benzenesulfonyl)-4-(4-fluoro-phenyl)-quinoline,
4-(3-chloro-phenyl)-3-(3-cyano-benzenesulfonyl)-6-fluoro-quinoline,
4-(4-chloro-phenyl)-3-(3-cyano-benzenesulfonyl)-6-fluoro-quinoline,
3-(3-cyano-benzenesulfonyl)-6-fluoro-4-(4-methoxy-phenyl)-quinoline,
3-(3-chloro-4-methyl-benzenesulfonyl)-4-(3-chloro-phenyl)-6-fluoro-quinoline,
3-(3-chloro-4-methyl-benzenesulfonyl)-4-(4-chloro-phenyl)-6-fluoro-quinoline,
3-(3-chloro-4-methyl-benzenesulfonyl)-6-fluoro-4-(3-fluoro-phenyl)-quinoline,
3-(3-chloro-4-methyl-benzenesulfonyl)-6-fluoro-4-(3-methoxy-phenyl)-quinoline,
3-(3-chloro-4-methyl-benzenesulfonyl)-6-fluoro-4-(4-fluoro-phenyl)-quinoline,
4-(4-chloro-phenyl)-6-fluoro-3-(3-methoxy-benzenesulfonyl)-quinoline,
6-fluoro-3-(3-methoxy-benzenesulfonyl)-4-(3-methoxy-phenyl)-quinoline,
3-(3,4-dimethyl-benzenesulfonyl)-6-fluoro-4-(3-fluoro-phenyl)-quinoline,
3-(3,4-dimethyl-benzenesulfonyl)-6-fluoro-4-(4-methoxy-phenyl)-quinoline,
3-(3,4-dimethyl-benzenesulfonyl)-6-fluoro-4-(4-fluoro-phenyl)-quinoline,
3-(3,4-difluoro-benzenesulfonyl)-6-fluoro-4-(3-fluoro-phenyl)-quinoline,
3-(3,4-difluoro-benzenesulfonyl)-6-fluoro-4-(4-fluoro-phenyl)-quinoline,
3-(3-chloro-4-fluoro-benzenesulfonyl)-4-(4-chloro-phenyl)-6-fluoro-quinoline,
3-(3-chloro-4-fluoro-benzenesulfonyl)-6-fluoro-4-(3-methoxy-phenyl)-quinoline,
3-(3-chloro-4-fluoro-benzenesulfonyl)-6-fluoro-4-(4-fluoro-phenyl)-quinoline,
4-(3-chloro-phenyl)-3-(3,5-dichloro-benzenesulfonyl)-7-fluoro-quinoline,
4-(4-chloro-phenyl)-3-(3,5-dichloro-benzenesulfonyl)-7-fluoro-quinoline,
4-(3-chloro-phenyl)-3-(3,5-dichloro-benzenesulfonyl)-8-fluoro-quinoline,
4-(4-chloro-phenyl)-3-(3,5-dichloro-benzenesulfonyl)-8-fluoro-quinoline,
4-(3-chloro-phenyl)-3-(3,5-dichloro-benzenesulfonyl)-6-fluoro-quinoline,
4-(4-chloro-phenyl)-3-(3,5-dichloro-benzenesulfonyl)-6-fluoro-quinoline,
6-fluoro-3-(4-methoxy-benzenesulfonyl)-4-(4-methoxy-phenyl)-quinoline,
7-chloro-4-(4-chloro-phenyl)-3-(3,5-difluoro-benzenesulfonyl)-quinoline,
7-chloro-4-(4-chloro-phenyl)-3-(3,4-difluoro-benzenesulfonyl)-quinoline,
7-chloro-4-(4-chloro-phenyl)-3-(3-cyano-benzenesulfonyl)-quinoline,
7-chloro-3-(3,5-dichloro-benzenesulfonyl)-4-(4-fluoro-phenyl)-quinoline,
7-chloro-3-(4-fluoro-benzenesulfonyl)-4-(4-fluoro-phenyl)-quinoline,
7-chloro-3-(3-fluoro-benzenesulfonyl)-4-(4-fluoro-phenyl)-quinoline,
7-chloro-3-(3,4-difluoro-benzenesulfonyl)-4-(4-fluoro-phenyl)-quinoline,
7-chloro-3-(3-chloro-4-fluoro-benzenesulfonyl)-4-(4-fluoro-phenyl)-quinoline,
7-chloro-3-(3-cyano-5-fluoro-benzenesulfonyl)-4-(4-fluoro-phenyl)-quinoline,
6-chloro-3-(3-chloro-4-fluoro-benzenesulfonyl)-4-(4-chloro-phenyl)-7-fluoro-quinoline,
6-chloro-3-(3-chloro-4-fluoro-benzenesulfonyl)-7-fluoro-4-(4-fluoro-phenyl)-quinoline,
3-(3-chloro-4-fluoro-benzenesulfonyl)-7-cyano-4-(2-fluoro-phenyl)-quinoline,
7-chloro-3-(3,4-difluoro-benzenesulfonyl)-8-fluoro-4-(3-fluoro-phenyl)-quinoline,
7-chloro-3-(3-chloro-4-fluoro-benzenesulfonyl)-4-(4-fluoro-phenyl)-8-fluoro-quinoline,
7-chloro-3-(3-chloro-4-fluoro-benzenesulfonyl)-8-fluoro-4-(3-fluoro-phenyl)-quinoline,
7-chloro-4-(4-chloro-phenyl)-3-(3,4-difluoro-benzenesulfonyl)-8-fluoro-quinoline,
7-chloro-3-(3-chloro-4-fluoro-benzenesulfonyl)-8-fluoro-4-(4-fluoro-phenyl)-quinoline.

3-(3-cyano-4-fluoro-benzenesulfonyl)-4-(3-fluoro-phenyl)-quinoline,
3-(3-cyano-5-fluoro-benzenesulfonyl)-4-(3-fluoro-phenyl)-quinoline,
3-(3-chloro-4-fluoro-benzenesulfonyl)-4-(4-chloro-phenyl)-8-fluoro-quinoline,
3-(3-chloro-4-fluoro-benzenesulfonyl)-8-fluoro-4-(2-fluoro-phenyl)-quinoline,
3-(3-chloro-4-fluoro-benzenesulfonyl)-8-fluoro-4-(3-fluoro-phenyl)-quinoline,
3-(3-cyano-4-fluoro-benzenesulfonyl)-4-(4-fluoro-phenyl)-quinoline,
3-(3-chloro-4-fluoro-benzenesulfonyl)-8-fluoro-4-(4-fluoro-phenyl)-quinoline,
3-(3-cyano-benzenesulfonyl)-8-fluoro-4-(2-fluoro-phenyl)-quinoline,
3-(3-cyano-benzenesulfonyl)-4-(3-fluoro-phenyl)-quinoline,
3-(3-cyano-benzenesulfonyl)-8-fluoro-4-(3-fluoro-phenyl)-quinoline,
3-(3-cyano-4-fluoro-benzenesulfonyl)-7-fluoro-4-(3-fluoro-phenyl)-quinoline,
4-(3-chloro-phenyl)-3-(3-cyano-4-fluoro-benzenesulfonyl)-quinoline,
3-(3-cyano-5-fluoro-benzenesulfonyl)-7-fluoro-4-(4-fluoro-phenyl)-quinoline,
7-chloro-3-(3-cyano-4-fluoro-benzenesulfonyl)-4-(3-fluoro-phenyl)-quinoline,
3-(3,5-dichloro-benzenesulfonyl)-4-(3,4-difluoro-phenyl)-8-fluoro-quinoline,
7-chloro-3-(3-cyano-5-fluoro-benzenesulfonyl)-4-(3-fluoro-phenyl)-quinoline,
3-(3-cyano-benzenesulfonyl)-8-fluoro-4-(4-ffuoro-phenyl)-quinoline,
3-(3,5-dichloro-benzenesulfonyl)-8-fluoro-4-(2-fluoro-phenyl)-quinoline,
3-(3-chloro-4-fluoro-benzenesulfonyl)-7-fluoro-4-(2-fluoro-phenyl)-quinoline,
4-(4-chloro-phenyl)-3-(3-cyano-benzenesulfonyl)-8-fluoro-quinoline,
4-(3-chloro-phenyl)-3-(3-cyano-5-fluoro-benzenesulfonyl)-quinoline,
7-chloro-3-(3-cyano-benzenesulfonyl)-4-(2-fluoro-phenyl)-quinoline,
3-(3-cyano-5-fluoro-benzenesulfonyl)-7-fluoro-4-(3-fluoro-phenyl)-quinoline,
3-(3-chloro-4-fluoro-benzenesulfonyl)-4-(3-chloro-phenyl)-8-fluoro-quinoline,
3-(3-cyano-benzenesulfonyl)-7-fluoro-4-(2-fluoro-phenyl)-quinoline,
3-(3-cyano-5-fluoro-benzenesulfonyl)-4-(3,4-difluoro-phenyl)-7-fluoro-quinoline,
3-(3-cyano-4-fluoro-benzenesulfonyl)-4-(3,4-dichloro-phenyl)-quinoline,
7-chloro-3-(3-chloro-4-fluoro-benzenesulfonyl)-4-(2-fluoro-phenyl)-quinoline,
7-chloro-3-(3-cyano-benzenesulfonyl)-4-(3-fluoro-phenyl)-quinoline,
4-(3-chloro-phenyl)-3-(3-cyano-4-fluoro-benzenesulfonyl)-7-fluoro-quinoline,
3-(3,4-difluoro-benzenesulfonyl)-4-(3,5-difluoro-phenyl)-8-fluoro-quinoline,
3-(3,4-difluoro-benzenesulfonyl)-4-(4-fluoro-phenyl)-quinoline,
3-(3,4-difluoro-benzenesulfonyl)-4-(3,4-difluoro-phenyl)-8-fluoro-quinoline,
3-(3,5-dichloro-benzenesulfonyl)-4-(3,4-difluoro-phenyl)-7-fluoro-quinoline,
3-(3,4-difluoro-benzenesulfonyl)-4-(3-fluoro-phenyl)-quinoline,
7-chloro-3-(3-cyano-benzenesulfonyl)-4-(4-fluoro-phenyl)-quinoline,
7-chloro-4-(3-chloro-phenyl)-3-(3-cyano-4-fluoro-benzenesulfonyl)-quinoline,
3-(3,4-difluoro-benzenesulfonyl)-8-fluoro-4-(2-fluoro-phenyl)-quinoline,
4-(3-chloro-phenyl)-3-(3-cyano-5-fluoro-benzenesulfonyl)-7-fluoro-quinoline,
3-(3-cyano-benzenesulfonyl)-4-(3,5-difluoro-phenyl)-7-fluoro-quinoline,
4-(3-chloro-phenyl)-3-(3-cyano-benzenesulfonyl)-8-fluoro-quinoline,
4-(4-chloro-phenyl)-3-(3,4-difluoro-benzenesulfonyl)-7-fluoro-quinoline,
7-chloro-3-(3,4-dichloro-benzenesulfonyl)-4-(3,4-difluoro-phenyl)-quinoline,
3-(3,5-dicyano-benzenesulfonyl)-7-fluoro-4-(4-fluoro-phenyl)-quinoline,
7-chloro-3-(3-chloro-5-fluoro-benzenesulfonyl)-4-(3-chloro-phenyl)-quinoline,
3-(3,4-difluoro-benzenesulfonyl)-4-(3,4-difluoro-phenyl)-7-fluoro-quinoline,
7-chloro-4-(3-chloro-phenyl)-3-(3,5-dichloro-benzenesulfonyl)-8-fluoro-quinoline,
7-chloro-4-(3-chloro-phenyl)-3-(3-cyano-benzenesulfonyl)-8-fluoro-quinoline,
7-chloro-4-(3-chloro-phenyl)-3-(3-cyano-benzenesulfonyl)-quinoline,
7-chloro-3-(3,5-dichloro-benzenesulfonyl)-4-(2-fluoro-phenyl)-quinoline,
3-(3,5-dicyano-benzenesulfonyl)-4-(3,4-difluoro-phenyl)-7-fluoro-quinoline,
3-(3,5-dichloro-benzenesulfonyl)-7-fluoro-4-(2-fluoro-phenyl)-quinoline,
3-(3-chloro-4-fluoro-benzenesulfonyl)-4-(3-chloro-phenyl)-7-fluoro-quinoline,
4-(3-chloro-phenyl)-3-(3,4-difluoro-benzenesulfonyl)-8-fluoro-quinoline,
4-(3,4-dichloro-phenyl)-3-(3,4-difluoro-benzenesulfonyl)-quinoline,
3-(3,4-difluoro-benzenesulfonyl)-7-fluoro-4-(2-fluoro-phenyl)-quinoline,
7-chloro-3-(3-chloro-4-fluoro-benzenesulfonyl)-4-(3,5-difluoro-phenyl)-quinoline,
7-chloro-4-(3-chloro-phenyl)-3-(3,5-dichloro-benzenesulfonyl)-quinoline,
7-amino-3-(3,4-difluoro-benzenesulfonyl)-4-(3-fluoro-phenyl)-quinoline,
4-(3-chloro-phenyl)-3-(3-cyano-benzenesulfonyl)-quinoline,
3-(3-cyano-5-fluoro-benzenesulfonyl)-4-(3,4-difluoro-phenyl)-quinoline,
3-(3-cyano-5-fluoro-benzenesulfonyl)-7-fluoro-4-(4-fluoro-phenyl)-quinoline,
4-(4-chloro-phenyl)-3-(3-cyano-5-fluoro-benzenesulfonyl)-7-fluoro-quinoline,
7-chloro-3-(3-chloro-5-fluoro-benzenesulfonyl)-4-(4-chloro-phenyl)-quinoline,
7-chloro-3-(3-chloro-5-fluoro-benzenesulfonyl)-4-(4-fluoro-phenyl)-quinoline,
3-(3-chloro-5-fluoro-benzenesulfonyl)-4-(4-fluoro-phenyl)-quinoline,
3-(3-chloro-5-fluoro-benzenesulfonyl)-4-(4-chloro-phenyl)-quinoline,
3-(3-chloro-4-fluoro-benzenesulfonyl)-4-(4-chloro-phenyl)-quinoline,
3-(3-chloro-4-fluoro-benzenesulfonyl)-4-(4-chloro-phenyl)-7-fluoro-quinoline,
3-(3-chloro-4-fluoro-benzenesulfonyl)-7-fluoro-4-(4-fluoro-phenyl)-quinoline,
7-chloro-3-(3-chloro-4-fluoro-benzenesulfonyl)-4-(4-fluoro-phenyl)-quinoline,
7-chloro-3-(3-chloro-4-fluoro-benzenesulfonyl)-4-(4-chloro-phenyl)-quinoline,
7-amino-3-(3-chloro-5-fluoro-benzenesulfonyl)-4-(4-fluoro-phenyl)-quinoline,
7-amino-3-(3-chloro-5-fluoro-benzenesulfonyl)-4-(3-fluoro-phenyl)-quinoline.

### Pharmaceutical formulations

The invention also relates to the pharmaceutical compositions containing the compounds of formula (I) and/or physiologically acceptable salts and/or hydrates and/or solvates thereof as active ingredient and one or more physiologically acceptable carriers.

The compounds of formula (I) and/or physiologically acceptable salts and/or hydrates and/or solvates thereof may be administered by any convenient method, for example by oral, parenteral (including subcutaneous, intramuscular, and intravenous), buccal, sublingual, nasal, rectal or transdermal administration and the pharmaceutical compositions adapted accordingly.

The compounds of formula (I) and/or physiologically acceptable salts and/or hydrates and/or solvates thereof which are active when given orally can be formulated as liquids or solids, for example syrups, suspensions or emulsions, tablets, capsules and lozenges.

A liquid formulation of the compounds of formula (I) and/or physiologically acceptable salts and/or hydrates and/or solvates thereof generally consist of a suspension or solution of the compound of formula (I) and/or physiologically acceptable salts and/or hydrates and/or solvates thereof in a suitable liquid carrier(s) for example an aqueous solvent, such as water and ethanol or glycerine, or a non-aqueous solvent, such as polyethylene glycol or an oil. The formulation may also contain a suspending agent, preservative, flavouring or colouring agent.

A composition in the solid form of a tablet can be prepared using any suitable pharmaceutical carrier(s) routinely used for preparing solid formulations. Examples of solid carriers include lactose, terra alba, sucrose, talc, gelatine, agar, pectin, acacia, magnesium stearate, stearic acid etc. Optionally, tablets may be coated by standard aqueous or nonaqueous techniques.

A composition in the solid form of a capsule can be prepared using routine encapsulation procedures. For example, pellets containing the active ingredient can be prepared using standard carriers and then these are filled into a hard gelatine capsule; alternatively, a dispersion or suspension can be prepared using any suitable pharmaceutical carrier(s), for example aqueous gums, celluloses, silicates or oils and the dispersion or suspension then is filled into a soft gelatine capsule.

Typical parenteral compositions consist of a solution or suspension of the compound of formula (I) and/or physiologically acceptable salts and/or hydrates and/or solvates thereof in a sterile aqueous carrier or parenterally acceptable oil, for example polyethylene glycol, polyvinyl pyrrolidone, lecithin, arachis oil or sesame oil. Alternatively, the solution can be lyophilised and then reconstituted with a suitable solvent just prior to administration.

Compositions of the present invention for nasal administration containing a compound of formula (I) and/or physiologically acceptable salts and/or hydrates and/or solvates thereof may conveniently be formulated as aerosols, drops, gels and powders. Aerosol formulations of the present invention typically comprise a solution or fine suspension of the compound of formula (I) and/or physiologically acceptable salts and/or hydrates and/or solvates in a physiologically acceptable aqueous or non-aqueous solvent and are usually presented in a single or multidose quantities in sterile form in a sealed container, which can take the form of a cartridge or refill for use with an atomizing device. Alternatively, the sealed container may be a unitary dispensing device, such as a single dose nasal inhaler or an aerosol dispenser fitted with a metering valve which is intended for disposal once the contents of the container have been exhausted. If the dosage form comprises an aerosol dispenser, it will contain a propellant which can be a compressed gas, such as compressed air or an organic propellant, such as a fluorochlorohydrocarbon. The aerosol dosages form can also take the form of a pump-atomiser.

Compositions of the present invention containing a compound of formula (I) and/or physiologically acceptable salts and/or hydrates and/or solvates are suitable for buccal or sublingual administration including tablets, lozenges and pastilles, wherein the active ingredient is formulated with a carrier, such as sugar and acacia, tragacanth, or gelatine, glycerin etc.

Compositions of the present invention containing a compound of formula (I) and/or physiologically acceptable salts and/or hydrates and/or solvates thereof for rectal administration are conveniently in the form of suppositories containing a conventional suppository base, such as cocoa butter and other materials commonly used in the art. The suppositories may be conveniently formed by first admixing the composition with the softened or melted carrier(s) followed by chilling and shaping in moulds.

Compositions of the present invention containing a compound of formula (I) and/or physiologically acceptable salts and/or hydrates and/or solvates thereof for transdermal administration include ointments, gels and patches.

The compositions of the present invention containing a compound of formula (I) and/or physiologically acceptable salts and/or hydrates and/or solvates thereof is preferably in the unit dose form, such as tablet, capsule or ampoule.

Each dosage unit of the present invention for oral administration contains preferably from 0.1 to 500 mg of a compound of formula (I) and/or physiologically acceptable salts and/or hydrates and/or solvates thereof calculated as a free base.

Each dosage unit of the present invention for parenteral administration contains preferably from 0.1 to 500 mg of a compound of formula (I) and/or physiologically acceptable salts and/or hydrates and/or solvates thereof calculated as a free base.

The compounds of formula (I) and/or physiologically acceptable salts and/or hydrates and/or solvates thereof can normally be administered in a daily dosage regimen. In the treatment of mGluR1 and mGluR5 mediated disorders, such as schizophrenia, anxiety, depression, panic, bipolar disorders, and circadian disorders or chronic and acute pain disorders the dosage levels from about 0,01 mg/kg to about 140 mg/kg of body weight per day are useful or alternatively about 0.5 mg to about 7 g per patient per day.

The amount of active ingredient that may be combined with the carrier materials to produce a single dosage form will vary depending upon the host treated and the particular mode of administration. For example, a formulation intended for the oral administration to humans may conveniently contain from about 0.5 mg to about 5 g of active agent, compounded with an appropriate and convenient amount of carrier material which may vary from about 5 to about 95 percent of the total composition. Unit dosage forms will generally contain between from about 1 mg to about 1000 mg of the active ingredient, typically 25 mg, 50 mg, 100 mg, 200 mg, 250-300 mg, 400 mg, 500 mg, 600 mg, 800 mg or 1000 mg.

It is understood, however, that the specific dose level for any particular patient will depend upon a variety of factors including the age, body weight, general health, sex, diet, time of administration, route of administration, rate of excretion, drug combination and the severity of the particular disease undergoing therapy.

### Medical use

The compounds of formula (I) and/or physiologically acceptable salts and/or hydrates and/or solvates of the present invention have been found to exhibit biological activity at mGluR1 and mGluR5 receptors and are expected to be useful in the treatment of mGluR1 and mGluR5 mediated disorders.

It has been found that the compounds according to the present invention or salts thereof, exhibit a high degree of potency and selectivity for individual metabotropic glutamate receptor (mGluR) subtypes. In particular there are compounds according to the present invention that are potent and selective for mGluR1 and mGluR5 receptors. Accordingly, the compounds of the present invention are expected to be useful in the prevention and/or treatment of conditions associated with excitatory activation of mGluR1 and mGluR5 receptor and for inhibiting neuronal damage caused by excitatory activation of mGluR1 and mGluR5 receptor. The compounds may be used to produce an inhibitory effect of mGluR1 and mGluR5, in mammals, including human.

Thus, it is expected that the compounds of the invention are well suited for the prevention and/or treatment of mGluR1 and mGluR5 receptor-mediated disorders such as acute and chronic neurological and psychiatric disorders, chronic and acute pain disorders and neuromuscular dysfunctions of the lower urinary tract.

The dose required for the therapeutic or preventive treatment of a particular disorder will necessarily be varied depending on the host treated and the route of administration.

The invention relates to compounds of formula (I) as defined hereinbefore, for use in therapy.

The invention relates to compounds of formula (I) as defined hereinbefore, for use in prevention and/or treatment of mGluR1 and mGluR5 receptor-mediated disorders.

The invention relates to compounds of formula (I) as defined hereinbefore, for use in prevention and/or treatment of neurological disorders.

The invention relates to compounds of formula (I) as defined hereinbefore, for use in prevention and/or treatment of psychiatric disorders.

The invention relates to compounds of formula (I) as defined hereinbefore, for use in prevention and/or treatment of chronic and acute pain disorders.

The invention relates to compounds of formula (I) as defined hereinbefore, for use in prevention and/or treatment of neuromuscular dysfunctions of the lower urinary tract.

The invention relates to compounds of formula (I) as defined hereinbefore, for use in prevention and/or treatment of pain related to migraine, inflammatory pain, neuropathic pain disorders such as diabetic neuropathies, arthritis and rheumatoid diseases, low back pain, post-operative pain and pain associated with various conditions including angina, in renal or biliary colic, menstruation, migraine and gout.

The invention relates to compounds of formula (I) as defined hereinbefore, for use in prevention and/or treatment of Alzheimer's disease senile dementia, AIDS-induced dementia Parkinson's disease, amyotrophic lateral sclerosis, Huntington's Chorea, migraine, epilepsy, schizophrenia, depression, anxiety, acute anxiety, obesity, obsessive compulsive disorder, ophthalmological disorders such as retinopathies, diabetic retinopathies, glaucoma, auditory neuropathic disorders such as tinnitus, chemotherapy induced neuropathies, post-herpetic neuralgia and trigeminal neuralgia, tolerance, substance abuse and withdrawal, Fragile X, autism, mental retardation, schizophrenia and Down's Syndrome.

The invention relates to compounds of formula (I) as defined hereinbefore, for use in prevention and/or treatment of stroke, head trauma, anoxic and ischemic injuries, hypoglycemia, cardiovascular diseases and epilepsy.

The compounds are also well suited for the treatment of neuromuscular dysfunction of the lower urinary tract, such as urinary urgency, overactive bladder, greater urinary frequency, reduced urinary compliance, cystitis, incontinence, enuresis and dysuria.

The compounds are also well suited for the treatment of gastrointestinal disorders, such as transient lower esophageal sphincter relaxation (TLESR), gastrointestinal reflux disease and irritable bowel syndrome.

The present invention relates also to the use of a compound of formula (I) as defined hereinbefore, in the manufacture of a medicament for the prevention and/or treatment of mGluR1 and mGluR5 receptor-mediated disorders and any disorder listed above.

The invention also provides compound of formula (I), as herein before defined, for use in a method of treatment and/or prevention of mGluR1 and mGluR5 receptor mediated disorders and any disorder listed above, in a patient suffering from, or at risk of, said condition, which use comprises administering to the patient an effective amount of a compound of formula (I), as hereinbefore defined.

In the context of the present specification, the term "therapy" includes treatment as well as prevention, unless there are specific indications to the contrary. The terms "therapeutic" and "therapeutically" should be construed accordingly.

In this specification, unless stated otherwise, the term "antagonist" means a compound that by any means, partly or completely blocks the transduction pathway leading to the production of a response by the ligand.

The term "disorder", unless stated otherwise, means any condition and disease associated with metabotropic glutamate receptor activity.

### Methods of preparation

The reactions are carried out by conventional methods. If necessary, protecting groups can be used for protect functional groups as it apparent to one skilled in the art.

According to the present invention a process for the preparation of compounds of formula (I): 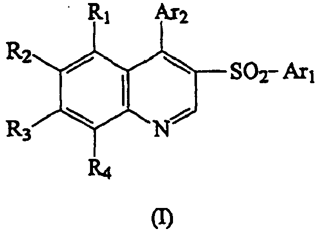 wherein Ar₁, Ar₂, R₁, R₂, R₃ and R₄ are as defined above, reacting a compound of formula (II): 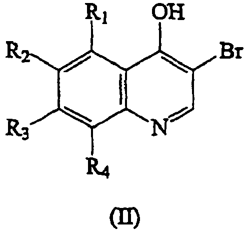 wherein R₁, R₂, R₃ and R₄ are as defined above for a compound of formula (I), with a compound of formula (III):

Ar₁-S⁻M⁺ (III)

wherein M is selected from alkali metals or alkaline-earth metals, Ar₁ is as defined above for compounds of formula (I), to give a compound of formula (IV): 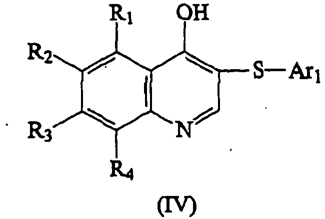 wherein R₁, R₂, R₃, R₄ and Ar₁ are as defined above for compounds of formula (I), thereafter oxidizing a compound of formula (IV) to obtain a compound of formula (V): 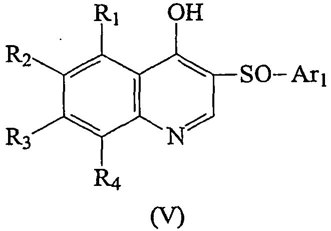 wherein R₁, R₂, R₃, R₄ and Ar₁ are as defined above for compounds of formula (I), thereafter oxidizing a compound of formula (V) to obtain a compound of formula (VI): 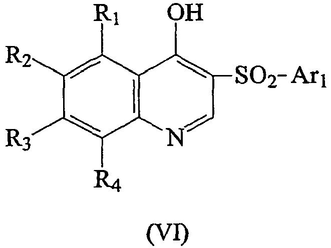 wherein R₁, R₂, R₃, R₄ and Ar₁ are as defined above for compounds of formula (I), thereafter converting the obtained compound of formula (VI) to a compound of formula (VII): 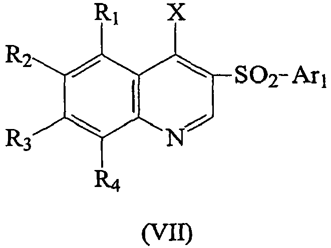 wherein X is selected from chloro, bromo, benzenesulfonyloxy, 4-fluoro-benzenesulfonyloxy, 4-methyl-benzenesulfonyloxy, methanesulfonyloxy or trifluoromethanesulfonyloxy groups and R₁, R₂, R₃, R₄ and Ar₁ are as defined above for compounds of formula (I), thereafter reacting the obtained compound of formula (VII) with a boronic acid derivative of formula (VIII):

Ar₂-B(OH)₂ (VIII)

in the presence of base (e.g. sodium carbonate) and catalyst (e.g. tetrakis(triphenylphosphine)-palladium(O)) in a solvent,
and optionally thereafter forming salts and/or hydrates and/or solvates of compounds of formula (I).

According to Scheme 1, 3-Bromo-quinolin-4-ol derivatives of formula (II) can be reacted by alkali or alkaline-earth metal salts (e.g. sodium salt) of thiophenols of formula (III) to provide a compound of formula (IV) (e.g. Bioorg. Med. Chem. Lett., 2001, 9, 1141). Advantageously the reaction can be carried out in the presence of palladium catalyst and under microwave conditions. 3-Bromo-quinolin-4-ol derivatives of formula (II) are known (e.g. 3-bromo-6-chloro-quinolin-4-ol: J. Chem. Soc., 1950, 384; 3-bromo-7-trifluoromethyl-quinolin-4-ol: Synthesis, 1977, 865) or can be synthesised by conventional methods. Alkali or alkaline-earth metal salts of thiophenols of formula (III) can be purchased or can be prepared by conventional methods.

Oxidation of 3-arenesulfanyl-quinolin-4-ol derivatives of formula (IV) can be accomplished in a suitable acid (e.g. trifluoroacetic acid) with hydrogen peroxid to give sulfoxides of formula (V) and sulfones of formula (VI), respectively.

Conversion of 3-arenesulfonyl-quinolin-4-ol derivatives of formula (VI) to compounds of formula (VII) can be carried out by known halogenation methods with suitable reagents (e.g. POCl₃, SOCl₂, PCl₅, POBr₃, PBr₃), or by acylation with the appropriate sulfonic acid halogenid or sulfonic acid anhydride derivatives.

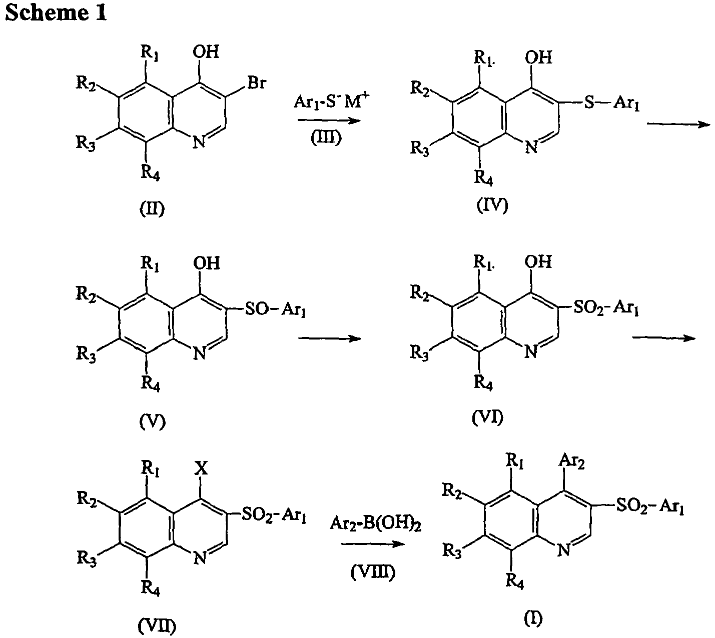

Another method to prepare intermediates of formula (VI) comprises reacting a compound of formula (IX):

Ar₁-SO₂Na (IX)

whererein Ar₁ is as defined above for a compound of formula (I), with α-halogen-acetic acid esters of formula (X):

Hlg-CH₂-COOR₅ (X)

wherein Hlg is halogen and R₅ is an ethyl or methyl group, to obtain a compound of formula (XI):

Ar₁-SO₂-CH₂-COOR₅ (XI)

wherein Ar₁ is as defined above for a compound of formula (I) and R₅ is as defined above for compounds of formula (X); reacting a compound of formula (XI) with a trialkyl orthoformate of formula (XII):

CH(OR₆)₃ (XII)

wherein R₆ is an ethyl or methyl group, to obtain a compound of formula (XIII): 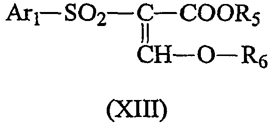 wherein Ar₁ is as defined above for a compound of formula (I), R₅ is as defined above for compounds of formula (X) and R₆ is as defined above for compounds of formula (XII); reacting a compound of formula (XIII) with an aniline derivative of formula (XIV): 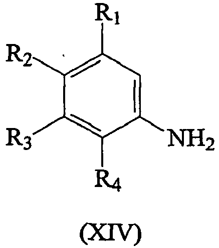 wherein R₁, R₂, R₃ and R₄ are as defined above for a compound of formula (I), to obtain a compound of formula (VI). 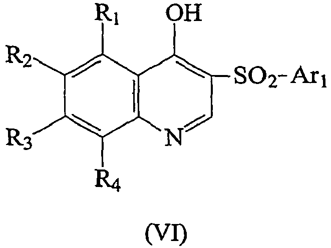 wherein R₁, R₂, R₃ and R₄ are as defined above for a compound of formula (1), thereafter converting the obtained compound of formula (VI) to a compound of formula (I) as described above.

According to Scheme 2 compounds of formula (IX) are reacted with α-halogen-acetic acid esters of formula (X) in a suitable solvent (e.g. DMF, water). Compounds of formula (IX) can be purchased or can be prepared from the appropriate benzenesulfonyl chloride derivatives by known methods (e.g. Org. Lett., 2003, 5(21), 3895). Compounds of formula (XIII) can be prepared by the reaction of compounds of formula (XI) and compounds of formula (XII) in the presence of acetic anhydride [J. Org. Chem. USSR (Engl. Transl., 1980, 16(7), 1275; Zh. Org. Khim., 1980, 16(7), 1483]. Reaction of compounds of formula (XIII) with aniline derivatives of compounds of formula (XIV) gives benzenesulfonyl-phenylamino-acrylic acid esters [e.g. J. Org. Chem. USSR (Engl. Transl., 1980, 16(7), 1275; Zh. Org. Khim., 1980, 16(7), 1483-1487] that can *be in situ* convert into quinolin-4-ol derivatives of formula (VI) (see analogous reaction: J. Chem. Soc. Perkin Trans., 1, 1994, 4, 387-392).

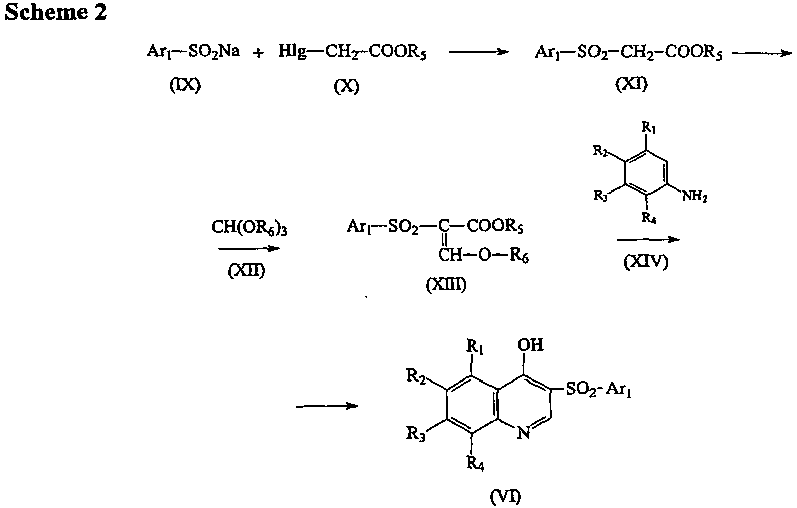

Compounds of formula (IV), compounds of formula (V), compounds of formula (VI) and compounds of formula (VII) and/or enantiomers and/or racemates and/or diastereomers and/or pharmaceutically acceptable salts thereof formed with acids or bases are new.

Compounds of formula (I) contain basic function(s) so can be transformed into the salts thereof with acids and/or can be liberated from the obtained acid addition salts by treatment with a base.

Compounds of formula (I) can be transformed into hydrates and/or solvates.

The compounds of formula (I) can optionally be intercoverted to a different compound of formula (1) by conventional synthetic methods.

### Biological test methods

### MGluR1 receptor binding test

MGluR1 receptor binding testes were performed according to modified method of Lavreysen et al. (Mol.Pharm., 2003, 63, 1082). Based on the high homology between the human and rat mGluR1 receptors, rat cerebellar membrane preaparation was used to determine the binding characteristics of reference compounds and novel compounds to the rat mGluRl. As radioligand [3H]R214127 (3 nM) was used and the nonspecific binding was determined in the presence of 1 µM of R214127.

IC-50 values were determined from displacement curves by nonlinear regression analysis and were converted by equation method of Cheng and Prusoff (Biochem. Pharmacol., 1973, 22, 3099) to Ki values.

### MGluR5 receptor binding tests

MGluR5 receptor binding was determined according to Gasparini et.al. (Bioorg. Med. Chem. Lett. 2000, 12:407-409) with modifications. Rat cerebro-cortical membrane preparation was used to determine the binding characteristics of reference compounds and novel compounds to the rat mGluR5. The A18 cell line expressing hmGluR5a (purchased from Euroscreen) was used to determine binding characteristics of the chemical compounds to the human mGluR5a receptor. As radioligand [3H]-M-MPEP (2 nM) was used. The nonspecific binding was determined in the presence of 10 µM M-MPEP.

### Assessment of functional activity

### Cell cultures for native rat mGluR5 and mGluR1 receptors

Functional potency at native rat mGluR5 and mGluR1 receptors was estimated using primary neocortical cell cultures derived from 17 day old Charles River rat embryos and primary cerebellar cell cultures derived from 4-day old Wistar rats, respectively (for the details on the preparation of neural cell cultures see Johnson, M.I.; Bunge, R.P. (1992): Primary cell cultures of peripheral and central neurons and glia. In: Protocols for Neural Cell Culture, eds: Fedoroff, S.; Richardson A., The Humana Press Inc., 51-77). After isolation the cells were plated onto standard 96-well microplates and the cultures were maintained in an atmosphere of 95% air-5% CO₂ at 37 °C. The neocortical and cerebellar cultures were used for the calcium measurements after 5-7 and 3-4 days in vitro, respectively.

### Cell cultures for recombinant human mGluR5a receptors

Chinese hamster ovary (CHO) cells stably expressing recombinant human mGluR5a (CHO-mGluR5a, purchased from Euroscreen) receptors were cultured in F12 medium containing 10% FCS, 1% antibiotic antimycotic solution, 400 µg/ml G418, 250 µg/ml zeocin, 5 µg/ml puromycin. Cells were kept at 37 °C in a humidified incubator in an atmosphere of 5% CO₂/95% air and were passaged three times a week. Cells were plated at 2.5-3.5×104 cell/well on standard 96-well microplates, receptor expression was induced by adding 600 ng/ml doxycycline on the next day. The calcium measurements were carried out 16-24 hours after the addition of the inducing agent.

### Fluorimetric measurement of cytosolic calcium concentration

Measurements of cytosolic calcium concentration ([Ca²⁺]ᵢ ) were carried out on primary neocortical and cerebellar cultures, and on CHO-mGluR5a cells stably expressing human mGluR5a receptors. Cells were grown in standard 96-well microplates and before the measurement were loaded with a fluorescent Ca²⁺-sensitive dye, fluo-4/AM (2 µM): the neural cultures were loaded in their growth medium, CHO-mGluR5a cells were loaded in assay buffer (145 mM NaCl, 5 mM KCl, 2 mM MgCl₂, 2 mM CaCl₂, 10 mM HEPES, 20 mM D-glucose, 2 mM probenecid, pH=7.4) supplemented with 2 mM Na-pyruvate and 30 µg/ml glutamate-pyruvate transaminase (in case of CHO-mGluR5a cells these supplements were also present during the course of the [Ca²]ᵢ measurements). Loading was done by incubating the cells with 100 µl/well dye solution at 37 °C in a humidified incubator in an atmosphere of 5% CO2/95% air for 40-120 min. To stop dye loading cells were washed twice with assay buffer. After washing, various concentrations of the test compounds (diluted in assay buffer from a DMSO or a dimethylformamide (DMF) stock solution, final DMSO/DMF concentration was <0.1%) or buffer were added to each well depending on the experimental setup. In the case of neocortical cultures the assay buffer also contained TTX (0.5 µM, to suppress spontaneous oscillations of [Ca2+]i, in the case of cerebellar cultures probenecid was substituted with sulfinpyrazone (0.25 mM).

After incubation at 37 °C for 10-20 min. baseline and agonist-evoked changes of [Ca2+]i were measured column by column with a plate reader fluorimeter (FlexStation II, Molecular Devices). Excitation and detection of emission was carried out from the bottom of the plate. The whole measurement process was performed at 37 °C and was controlled by custom software. Inhibitory potency of the test compounds was assessed by measuring the reduction in the agonist-evoked [Ca²⁺]ᵢ -elevation in the presence of different concentrations of the compounds. DHPG was used as agonist for all three cultures, the concentration was 20 and 100 µM for neocortical and cerebellar cultures, respectively. In the case of CHO-mGluR5a cells DHPG was applied at an EC80 concentration, the EC80-values were derived from daily determined dose-response curves. Fluorescence data were expressed as ΔF/F (fluorescence change normalized to baseline).

All treatments on a single plate were measured in multiple wells. Data from all wells with the same treatment were averaged and the average values were used for analysis. Inhibitory potency of a compound at a single concentration point was expressed as percent inhibition of the control agonist response. Sigmoidal concentration-inhibition curves were fitted to the data (derived from at least three independent experiments) and IC50-values were determined as the concentration that produces half of the maximal inhibition caused by the compound. Raw fluorescence data were analyzed using Soft Max Pro (Molecular Devices), curve fitting was done with GraphPad Prism.

### Results

Compounds of formula (I) of the present invention showed affinity for both rat and human mGluR1 and mGluR5 receptors and proved to be functional antagonists that are they inhibited functional responses elicited by stimulation of mGluR5 receptors.

The invention is further illustrated by the following non-limiting examples.

Unless specifically stated otherwise, all operation were carried out at room temperature, that is at a temperature range of 18-25 °C. The course of reactions was followed by thin layer chromatography (TLC) and reaction times are given for illustration only. The structure of all intermediates and end products were elucidated by IR, NMR and MS spectroscopy. When given yields are for illustration only. When given, NMR data are in the form of delta (δ) values for major diagnostic protons, given in parts per million (ppm) relative to tetramethylsilane (TMS) as internal standard, using the indicated solvent. Conventional abbreviations are used for signal shape.

### Examples

All starting materials are either commercially available or can be synthesized by different known methods described in the literature.

### Example 1

### 4-(4-chloro-phenyl)-3-(4-methyl-benzenesulfonyl)-quinoline; Table I, compound 1

### 3-(4-Methyl benzenesulfanyl)-quinolin-4-ol

A mixture of 3-bromo-quinolin-4-ol (0.448 g, 2 mmol; J. Am. Chem. Soc. 1946, 68, 1229-1231), 4-methylbenzenethiol (0.30 g, 2.4 mmol), tetrakis-(triphenylphosphin)palladium(O) (0.115 g, 0.1 mmol), sodium-*tert*-butylate (0.23 g, 2.4 mmol) and DMF (2.0 ml) was stirred and irradiated at 142 °C for 3 hours in a 8-ml microwave vial. The solvent was evaporated *in vacuo* and the residue was purified by gradient silica gel flashchromatography (80 g silica gel, eluent A: chloroform, eluent B: chloroform: methanol = 95:5) to give 0.33 g of the title compound in 62% yield.
MS (EI) M⁺ = 268.2

### 3-(4-Methyl-benzenesulfonyl)-quinolin-4-ol

To a mixture of 3-(4-Methyl-benzenesulfanyl)-quinolin-4-ol (0.25 g, 0.936 mmol) and trifluoroacetic acid (5.0 ml) a solution of hydrogen peroxide in trifluoroacetic acid (c = 3.0M, 3.7 ml) was added dropwise. The solution was stirred for 8 hours at room temperature. To the reaction mixture 6ml of water was added dropwise. The precipitate was filtered off, washed with water and dried *in vacuo* to give 0.24 g of the title compound in 86%.
MS (EI) M⁺ = 300.1

### 4-Chloro-3-(4-methyl-benzenesulfonyl)-quinoline

A mixture of 3-(4-methyl-benzenesulfonyl)-quinolin-4-ol (0.24 g, 0.8 mmol) and phosphorus(V) oxychloride (15 ml) was refluxed for 5 hours. Phosphorus(V) oxychloride was distilled off, and the residue was poured onto ice. The slurry was stirred for 2 hours at 0-5 °C , neutralized with sodium carbonate and extracted with chloroform (50 ml). The organic layer was dried over anhydrous sodium sulfate, filtered and the solvent was removed *in vacuo* to give 0.23 g of the title compound in 90%yield..
MS (EI) M⁺ = 318.2

### 4-(4-chloro-phenyl)-3-(4-methyl-benzenesulfonyl)-quinoline

A mixture of 4-chloro-3-(4-methyl-benzenesulfonyl)-quinolin-4-ol (0.2 g, 0.67 mmol) and 4-chlorophenylboronic acid (0.16 g, 1.0 mmol) in 8 ml dioxane was stirred for 20 hours at 90 °C with potassium carbonate (0.5 g, 3.6 mmol) and tetrakis-(triphenylphosphin)palladium(0) (0.04 g, 0.035 mmol). After cooling the mixture was concentrated *in vacuo.* The crude residue was purified by column chromatography on silica gel (Kieselgel 60, eluent: chloroform) to obtain 0.21 g of the title compound in 80% yield.
¹H NMR (500 MHz, DMSO-*d₆*): 9.61 (s, 1H); 8.21 (dm, *J* = 8.6 Hz, 1H); 7.98 (ddd, *J* = 8.5, 6.8, 1.4 Hz, 1H); 7.64 (ddd, *J* = 8.5, 6.8, 1.2 Hz, 1H); 7.49-7.44 (m, 2H); 7.37-7.32 (m, 2H); 7.30-7.24 (m, 3H); 7.04-6.98 (m, 2H), 2.36 (s, 3H).
MS (EI) M⁺ = 393.8

### 4-(4-chloro-phenyl)-3-(4-methyl-benzenesulfonyl)-quinoline hydrochloride

4-(4-chloro-phenyl)-3-(4-methyl-benzenesulfonyl)-quinoline (40 mg, 0.102 mmol) was dissolved in ethyl acetate (1.5 ml) and a solution of hydrogen chloride in ethyl acetate (c = 1.6M, 0.14 ml, 0.224 mmol) was added dropwise to the solution. The solid was filtered off, washed with ethyl acetate and dried in vacuo to give 35 mg of the title compound in 80% yield.
¹H NMR (500 MHz, DMSO-*d₆*): 9.61 (s, 1H); 8.22 (dm, *J* = 8.6 Hz, 1H); 7.98 (ddd, *J* = 8.6, 6.8, 1.4 Hz, 1H); 7.64 (ddd, *J* = 8.6, 6.8, 1.2 Hz, 1H); 7.50-7.43 (m, 2H); 7.37-7.31 (m, 2H); 7.31-7.23 (m, 3H); 7.05-6.98 (m, 2H),2.36 (s, 3H).
MS (EI) M⁺ = 393.8

### Example 2

### (4-Chloro-benzenesulfonyl)-acetic acid methyl ester (intermediate)

A mixture of methyl bromoacetate (11.25 ml, 116 mmol) and sodium 4-chloro-benzenesulfinate (25.2g, 116 mmol) in DMF (120 ml) was stirred and heated at 80°C for 2 h. The solution was diluted with water (360 ml). The separated oil was extracted with chloroform (200 ml) and washed with water (3 X 80 ml). The organic phase was evaporated *in vacuo* to obtain 22.4 g of the title compound in 77.7% yield. MS (EI) M⁺= 249.1.

Applying the above procedure the following compounds were prepared: e.g. (3-chloro-benzenesulfonyl)-acetic acid methyl ester (MS (EI) M⁺ = 249.1); (3,5-dichloro-benzenesulfonyl)-acetic acid methyl ester (MS (EI) M⁺ = 283.1); (4-methoxy-benzenesulfonyl)-acetic acid methyl ester (MS (EI) M⁺ = 245.1); (3-chloro-4-fluoro-benzenesulfonyl)-acetic acid methyl ester (MS (EI) M⁺ = 267.1); (3,5-difluoro-benzenesulfonyl)-acetic acid methyl ester (MS (EI) M⁺ = 251.1); (3-fluoro-benzenesulfonyl)-acetic acid methyl ester (MS (EI) M⁺= 233.2).

### Example 3

### 2-(4-Chloro-benzenesulfonyl)-3-ethoxy-acrylic acid methyl ester (intermediate)

The mixture of (4-chloro-benzenesulfonyl)-acetic acid methyl ester (22.4 g, 90 mmol), triethyl orthoformate (33.2 ml, 216 mmol) and acetic anhydride (19.1 ml, 203 mmol) was refluxed for 3 h with simultaneous distillation of ethanol, triethyl orthoformate and acetic anhydride, and then evaporated to dryness. The crude material (22.7 g, 82.8%) was used in the next step without purification. MS (EI) M⁺= 305.1.

Applying the above procedure the following compounds were prepared: e.g. 2-(3-chloro-benzenesulfonyl)-3-ethoxy-acrylic acid methyl ester (MS (EI) M⁺ = 305.1); 2-(3-chloro-4-fluoro-benzenesulfonyl)-3-ethoxy-acrylic acid methyl ester (MS (EI) M⁺= 323.1); 2-(3,5-dichloro-benzenesulfonyl)-3-ethoxy-acrylic acid methyl ester (MS (EI) M⁺ = 340.1); 2-(4-fluoro-benzenesulfonyl)-3-ethoxy-acrylic acid methyl ester (MS (EI) M⁺ = 289.1); 2-(3-cyano-5 fluoro-benzenesulfonyl)-3-ethoxy-acrylic acid methyl ester (MS (EI) M⁺= 314.2).

### Example 4

### 7-Chloro-3-(4-chloro-benzenesulfonyl)-quinolin-4-ol (intermediate)

The mixture of 2-(4-chloro-benzenesulfonyl)-3-ethoxy-acrylic acid methyl ester (7.62 g, 25 mmol) and 3-chloroaniline (3.19 g, 25 mmol) in diphenyl ether (20 ml) was heated at near reflux for 1 h. After cooling the precipitate was filtered and washed with ether to obtain 4.25g of the title compound in 48.0% yield. MS (EI) M⁺= 355.1.

Applying the above procedure the following compounds were prepared: e.g. 8-chloro-3-(4-chloro-benzenesulfonyl)-quinolin-4-ol (MS (EI) M⁺ = 355.1); 6-chloro-3-(4-fluoro-benzenesulfonyl)-quinolin-4-ol (MS (EI) M⁺= 338.1); 6-cyano-3-(4-fluoro-benzenesulfonyl)-quinolin-4-ol (MS (EI) M⁺ = 329.2); 8-chloro-3-(3,4-dichloro-benzenesulfonyl)-quinolin-4-ol (MS (EI) M⁺ = 390.1); 7-chloro-3-(3,5-difluoro-benzenesulfonyl)-quinolin-4-ol (MS (EI) M⁺= 356.1).

### Example 5

### 3-(4-chloro-benzenesulfonyl)-4,7-dichloroquinoline (intermediate)

7-Chloro-3-(4-chloro-benzenesulfonyl)-4-hydroxyquinoline (4.25 g, 12 mmol) in phosphorus(V) oxychloride (5.6 ml, 60 mmol) was refluxed for 3 h. The reaction mixture was poured into water (50 ml) and was alkalized with 5M sodium hydroxyde solution. After cooling the precipitate was filtered and washed with water to obtain 3.8 g of the title compound in 85.0% yield. MS (EI) M⁺ = 373.2.

Applying the above procedure the following compounds were prepared: e.g. 3-(3-chloro-benzenesulfonyl)-4,6-dichloroquinoline (MS (EI) M⁺ = 373.2); 3-(4-chloro-benzenesulfonyl)-4,8-dichloroquinoline (MS (EI) M⁺ = 373.2); 4-chloro-6-cyano-3-(4-fluoro-benzenesulfonyl)-quinoline (MS (EI) M⁺ = 347.1); 4-chloro-3-(4-chloro-benzenesulfonyl)-6-fluoro-quinoline (MS (EI) M⁺ = 357.1).

### Example 6

### 4-bromo-7-chloro-3-(4-chloro-benzenesulfonyl)-quinoline (intermediate)

A mixture of 7-chloro-3-(4-chloro-benzenesulfonyl)-quinolin-4-ol (0.5 g, 1.41 mmol) and phosphorus(V) oxybromide (1.2 g, 4.2 mmol) in chloroform (30 ml) and triethylamine (1 ml) was refluxed for 6 hours. The reaction mixture was diluted with water (30 ml) and the pH was adjusted to 10 with aqueous sodium hydroxide solution. The organic layer was separated, dried over sodium sulfate, filtered, and concentrated *in vacuo.* The obtained crude product was purified by crystallization from diethyl ether to obtain 0.45 g of the title compound in 77% yield. MS (EI) M⁺= 418.1.

Applying the above procedure the following compounds were prepared: e.g. 4-bromo-3-(4-chloro-benzenesulfonyl)-6-fluoro-quinoline (MS (EI) M⁺ = 347.1); 4-bromo-7-chloro-3-(3,5-difluoro-benzenesulfonyl)-quinoline (MS (EI) M⁺ = 420.1); 4-bromo-7-chloro-3-(4-chloro-benzenesulfonyl)-6-fluoro-quinoline (MS (EI) M⁺ = 436.1); 4-bromo-7-chloro-3-(4-fluoro-benzenesulfonyl)-quinoline (MS (EI) M⁺ = 402.1); 4-bromo-7-chloro-3-(3-fluoro-benzenesulfonyl)-quinoline (MS (EI) M⁺ = 402.1); 4-bromo-7-chloro-3-(3-cyano-benzenesulfonyl)-quinoline (MS (EI) M⁺ = 409.2); 4-bromo-7-chloro-3-(3-cyano-5-fluoro-benzenesulfonyl)-quinoline (MS (EI) M⁺= 427.1).

### Example 7

### 7-chloro-3-(4-chloro-benzenesulfonyl)-4-(4-fluoro-phenyl)-quinoline

### Table I compound 4

A mixture of 4-bromo-7-chloro-3-(4-chloro-benzenesulfonyl)-quinoline (0.42 g, 1 mmol), 4-fluorophenylboronic acid (0.21 g, 1.5 mmol) and tetrakis-(triphenylphosphin)palladium(0) (0.08 g, 0.07 mmol) in 30 ml 1,2-dimethoxyethane and 8 ml of 2M aqueous sodium carbonate solution was stirred for 2 hours at 70 °C. After cooling the mixture was concentrated *in vacuo.* The crude residue was purified by column chromatography on silica gel (Kieselgel 60, eluent: chloroform) and crystallized from methanol to obtain 0.29 g of the title compound in 67% yield..
¹H NMR (400 MHz, DMSO-*d₆*): 6.92-6.98 m (2H) [H-14,18]; 7.06-7.13 m (2H) [H-15,17]; 7.25-7.32 m (5H) [H-6, 22,23,25, 26]; 7.44 dd (1H) [H-7]; 9.23 d (1H) [H-9]; 9.77 s (1H) [H-2]
MS (EI) M⁺ = 433.2

Applying the above procedure the following compounds were prepared: e.g. 7-fluoro-3-(4-fluoro-benzenesulfonyl)-4-(3-fluoro-phenyl)-quinoline (Table I compound 9, MS (EI) M⁺ = 400.2),
4-(3-chloro-phenyl)-3-(3,4-dimethyl-benzenesulfonyl)-7-fluoro-quinoline (Table I compound 17, MS (EI) M⁺ = 426.2),
4-(4-chloro-phenyl)-3-(3,5-difluoro-benzenesulfonyl)-7-fluoro-quinoline (Table I compound 35, MS (EI) M⁺ = 434.2),
4-(3-chloro-phenyl)-8-fluoro-3-(4-methoxy-benzenesulfonyl)-quinoline (Table I compound 53, MS (EI) M⁺ = 428.2),
3-(3-cyano-benzenesulfonyl)-6-fluoro-4-(3-methoxy phenyl)-quinoline (Table I compound 68, MS (EI) M⁺ = 419.3),
3-(3,4-difluoro-benzenesulfonyl)-7-fluoro-4-(4-fluoro-phenyl)-quinoline (Table I compound 101, MS (EI) M⁺ = 418.2),
7-chloro-3-(3,4-difluoro-benzenesulfonyl)-4-(3-fluoro-phenyl)-quinoline (Table I compound 170, MS (EI) M⁺ = 434.2),
7-chloro-3-(3-cyano-5-fluoro-benzenesulfonyl)-4-(4-fluoro-phenyl)-quinoline (Table I compound 186, MS (EI) M⁺ = 441.2).

Examples of compounds of formula (I) and their affinity for rat mGluR5 and mGluR5 receptors are given in the table below.
**Table**

| **Comp. No.** | **Structure** | **(M+H)⁺ or M⁺** | **mGlu5 Kᵢ (nM)** | **mGlu1 Kᵢ (nM)** |
|---|---|---|---|---|
| 1. | 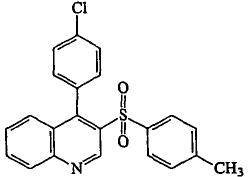 | 393.8 | *** | ** |
| 2. | 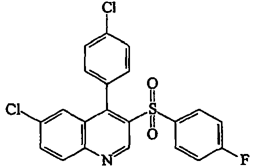 | 433.2 | * | * |
| 3. | 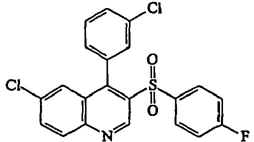 | 433.2 | * | * |
| 4. | 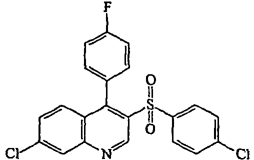 | 433.2 | *** | ** |
| 5. | 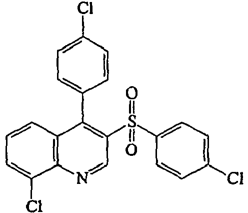 | 450.1 | ** | * |
| 6. | 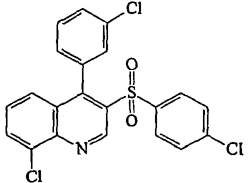 | 450.1 | ** | * |
| 7. | 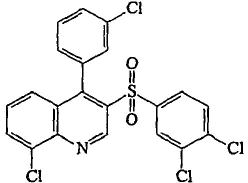 | 484.1 | *** | *** |
| 8. | 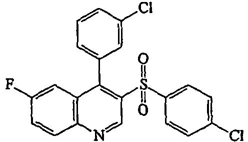 | 433.2 | * | * |
| 9. | 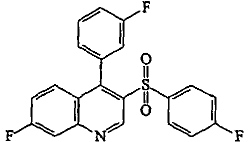 | 400.2 | *** | ** |
| 10. | 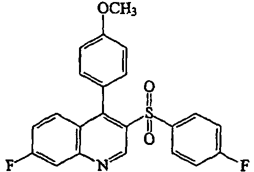 | 412.2 | ** | * |
| 11. | 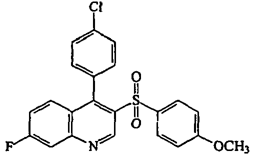 | 428.2 | *** | * |
| 12. | 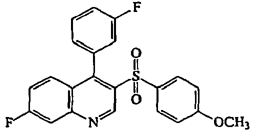 | 412.2 | ** | * |
| 13. | 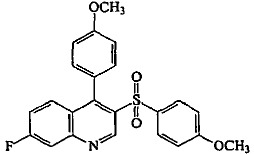 | 424.2 | *** | * |
| 14. | 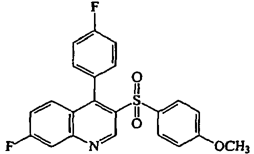 | 412.2 | ** | * |
| 15. | 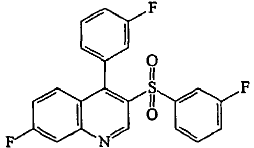 | 400.2 | *** | ** |
| 16. | 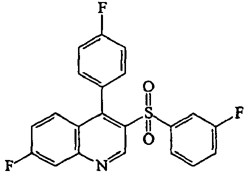 | 400.2 | *** | * |
| 17. | 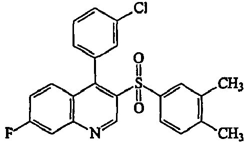 | 426.2 | *** | * |
| 18. | 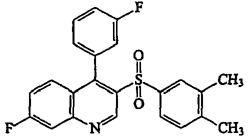 | 410.2 | *** | * |
| 19. | 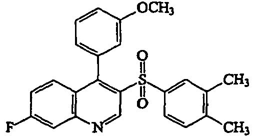 | 422.3 | *** | * |
| 20. | 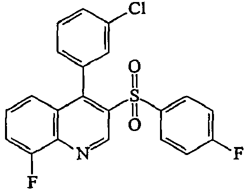 | 416.2 | *** | *** |
| 21. | 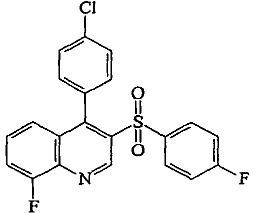 | 416.2 | *** | ** |
| 22. | 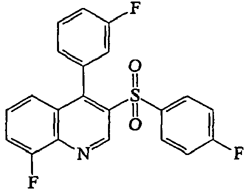 | 400.2 | *** | ** |
| 23. | 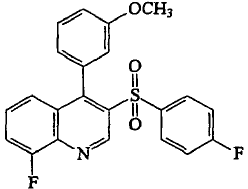 | 412.2 | *** | * |
| 24. | 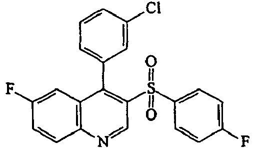 | 416.2 | * | * |
| 25. | 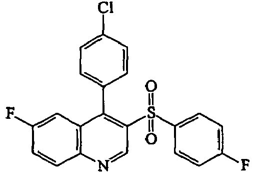 | 416.2 | ** | * |
| 26. | 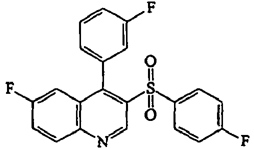 | 400.2 | ** | * |
| 27. | 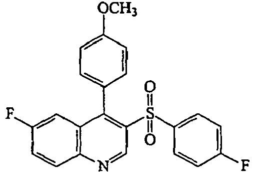 | 412.2 | * | * |
| 28. | 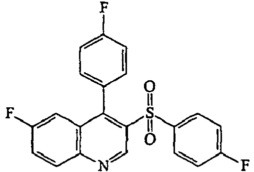 | 400.2 | * | * |
| 29. | 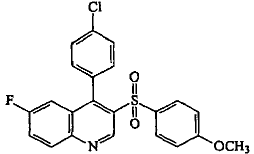 | 428.2 | *** | * |
| 30. | 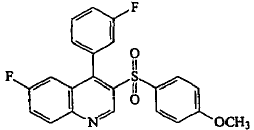 | 412.2 | ** | * |
| 31. | 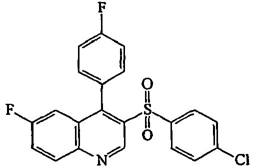 | 416.2 | * | * |
| 32. | 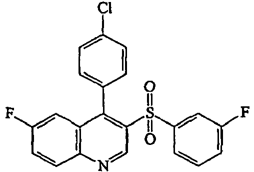 | 416.2 | * | * |
| 33. | 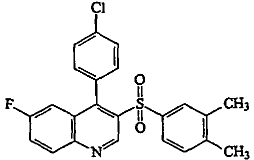 | 426.2 | *** | * |
| 34. | 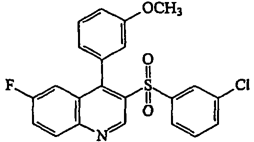 | 428.2 | * | * |
| 35. | 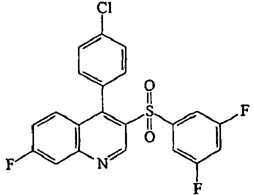 | 434.2 | *** | * |
| 36. | 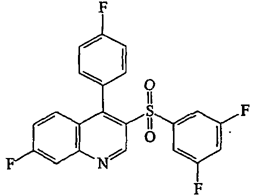 | 418.2 | *** | * |
| 37. | 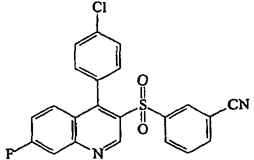 | 423.2 | *** | ** |
| 38. | 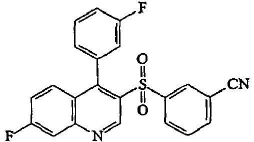 | 407.2 | *** | ** |
| 39. | 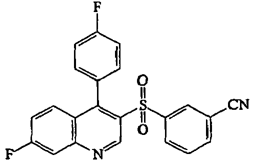 | 407.2 | *** | ** |
| 40. | 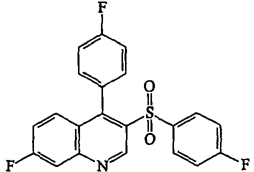 | 400.2 | *** | * |
| 41. | 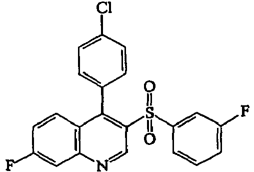 | 416.2 | *** | * |
| 42. | 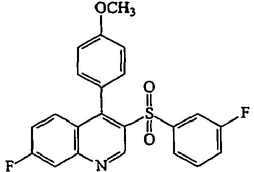 | 412.2 | * | * |
| 43. | 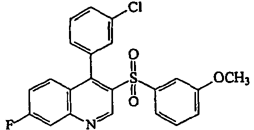 | 428.2 | *** | * |
| 44. | 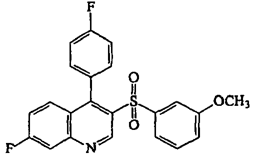 | 412.2 | *** | * |
| 45. | 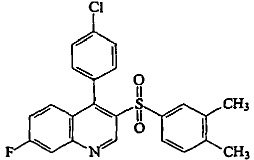 | 426.2 | *** | * |
| 46. | 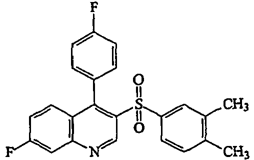 | 410.2 | *** | * |
| 47. | 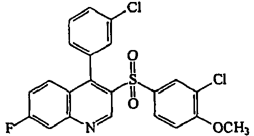 | 463.2 | *** | ** |
| 48. | 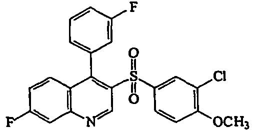 | 446.2 | *** | * |
| 49. | 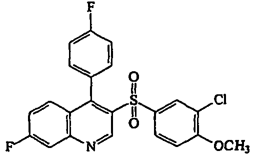 | 446.2 | *** | * |
| 50. | 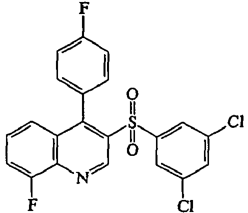 | 451.2 | *** | *** |
| 51. | 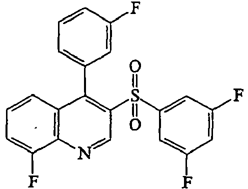 | 418.2 | *** | * |
| 52. | 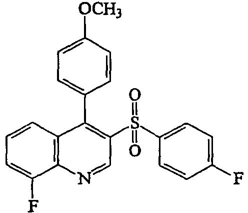 | 412.2 | *** | * |
| 53. | 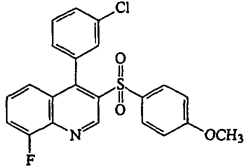 | 428.2 | *** | * |
| 54. | 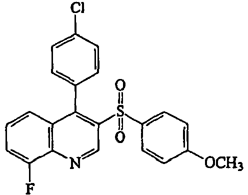 | 428.2 | *** | *** |
| 55. | 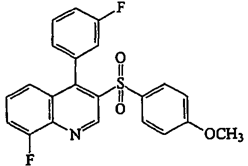 | 412.2 | *** | * |
| 56. | 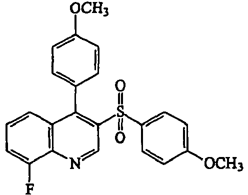 | 424.2 | *** | * |
| 57. | 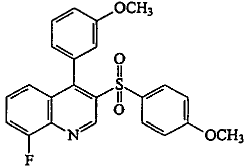 | 424.2 | *** | * |
| 58. | 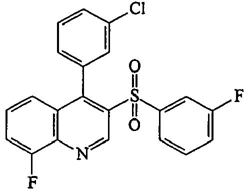 | 416.2 | *** | ** |
| 59. | 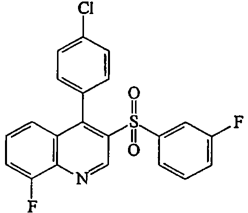 | 416.2 | *** | ** |
| 60. | 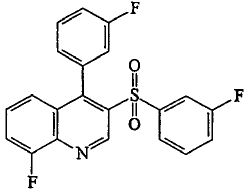 | 400.2 | *** | ** |
| 61. | 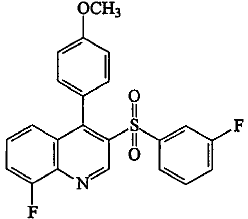 | 412.2 | *** | * |
| 62. | 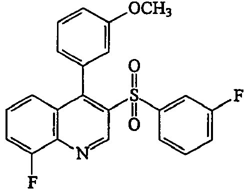 | 412.2 | *** | * |
| 63. | 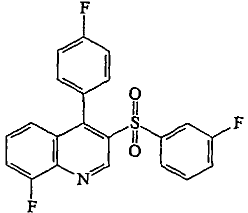 | 400.2 | *** | ** |
| 64. | 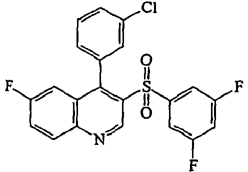 | 434.2 | * | * |
| 65. | 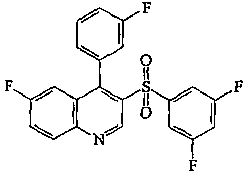 | 418.2 | ** | ** |
| 66. | 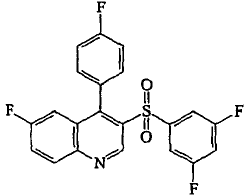 | 418.2 | ** | * |
| 67. | 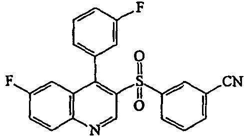 | 407.2 | *** | ** |
| 68. | 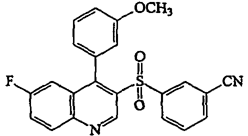 | 419.3 | *** | * |
| 69. | 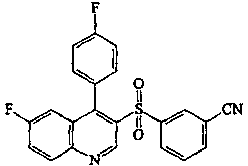 | 407.2 | *** | ** |
| 70. | 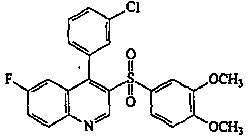 | 458.3 | * | * |
| 71. | 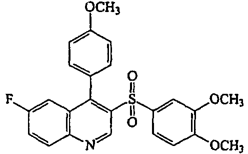 | 454.3 | * | * |
| 72. | 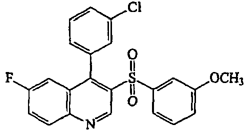 | 428.2 | *** | * |
| 73. | 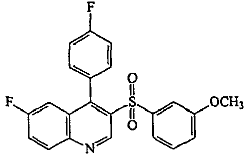 | 412.2 | *** | * |
| 74. | 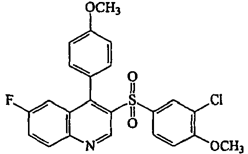 | 458.3 | *** | * |
| 75. | 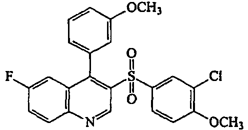 | 458.3 | *** | * |
| 76. | 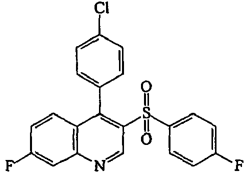 | 416.2 | *** | * |
| 77. | 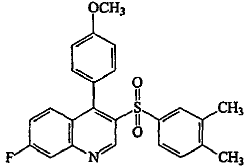 | 422.3 | *** | * |
| 78. | 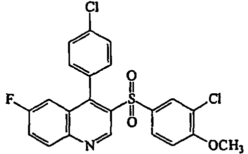 | 463.2 | *** | * |
| 79. | 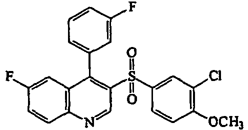 | 446.2 | *** | * |
| 80. | 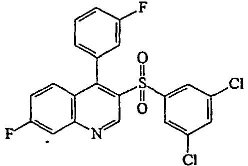 | 451.2 | *** | ** |
| 81. | 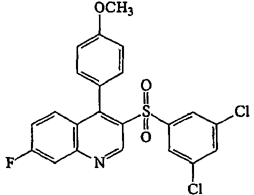 | 463.2 | *** | * |
| 82. | 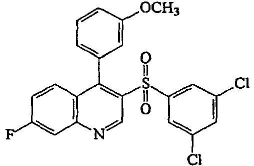 | 463.2 | *** | * |
| 83. | 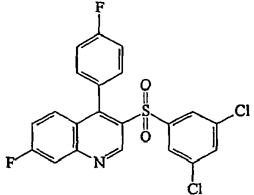 | 451.2 | *** | ** |
| 84. | 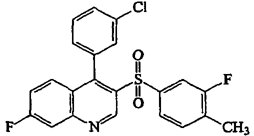 | 430.2 | *** | * |
| 85. | 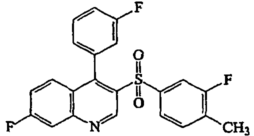 | 414.2 | *** | * |
| 86. | 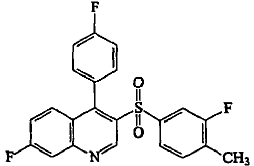 | 414.2 | *** | * |
| 87. | 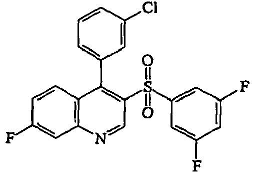 | 434.2 | ** | * |
| 88. | 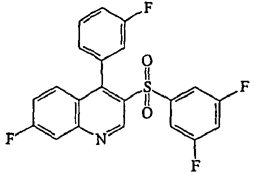 | 418.2 | *** | ** |
| 89. | 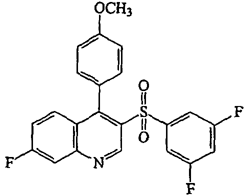 | 430.3 | *** | * |
| 90. | 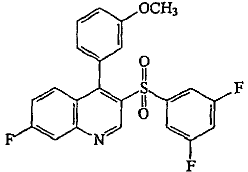 | 430.3 | *** | * |
| 91. | 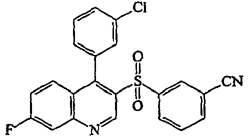 | 423.2 | *** | *** |
| 92. | 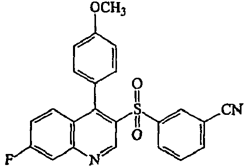 | 419.3 | *** | ** |
| 93. | 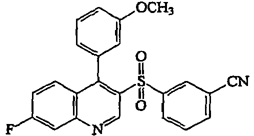 | 419.3 | *** | * |
| 94. | 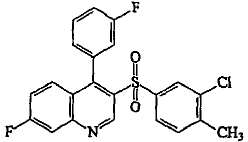 | 430.2 | *** | * |
| 95. | 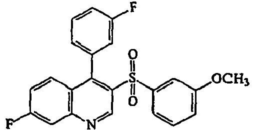 | 412.2 | ** | * |
| 96. | 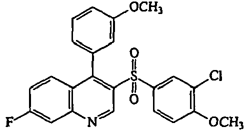 | 458.3 | ** | * |
| 97. | 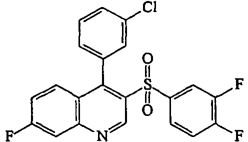 | 434.2 | ** | ** |
| 98. | 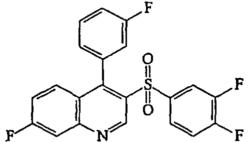 | 418.2 | *** | ** |
| 99. | 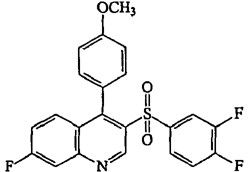 | 430.3 | *** | * |
| 100. | 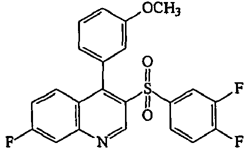 | 430.3 | *** | * |
| 101. | 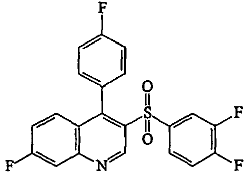 | 418.2 | *** | ** |
| 102. | 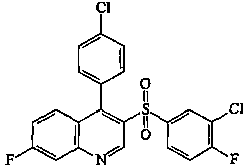 | 451.2 | *** | ** |
| 103. | 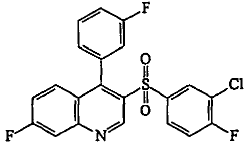 | 434.2 | *** | *** |
| 104. | 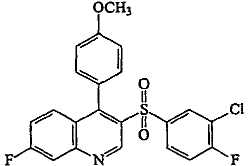 | 446.2 | *** | ** |
| 105. | 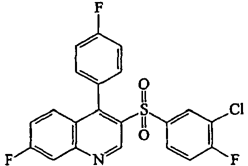 | 434.2 | *** | *** |
| 106. | 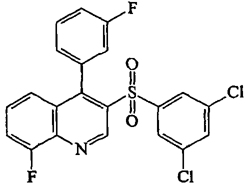 | 451.2 | *** | * |
| 107. | 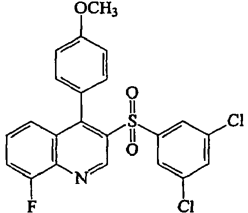 | 463.2 | *** | * |
| 108. | 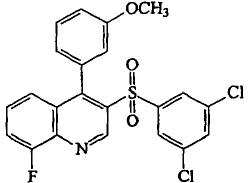 | 463.2 | *** | * |
| 109. | 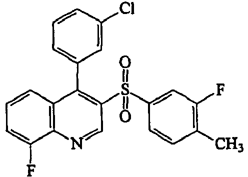 | 430.2 | *** | * |
| 110. | 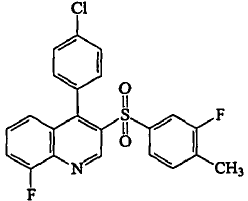 | 430.2 | *** | * |
| 111. | 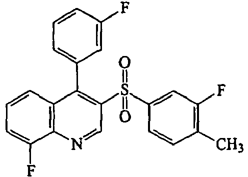 | 414.2 | *** | * |
| 112. | 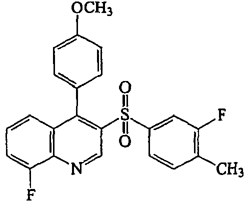 | 426.3 | *** | * |
| 113. | 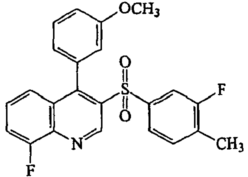 | 426.3 | *** | * |
| 114. | 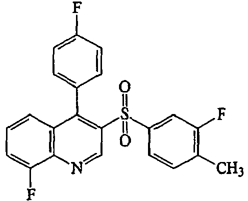 | 414.2 | *** | * |
| 115. | 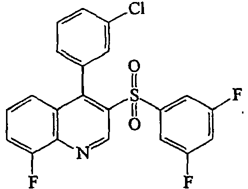 | 434.2 | *** | ** |
| 116. | 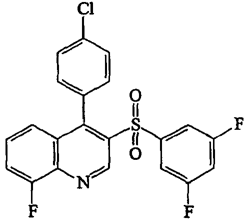 | 434.2 | *** | ** |
| 117. | 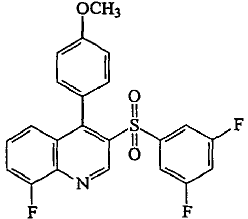 | 430.3 | *** | * |
| 118. | 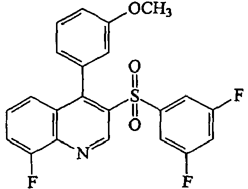 | 430.3 | *** | * |
| 119. | 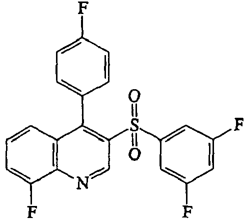 | 418.2 | *** | *** |
| 120. | 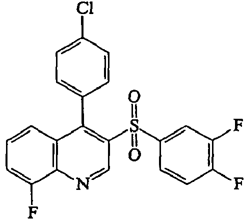 | 434.2 | *** | ** |
| 121. | 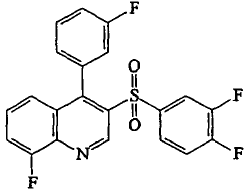 | 418.2 | *** | *** |
| 122. | 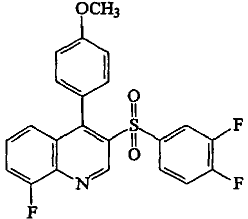 | 430.3 | *** | * |
| 123. | 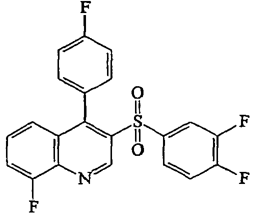 | 418.2 | *** | *** |
| 124. | 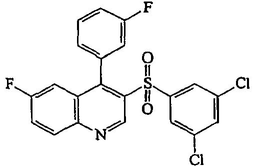 | 451.2 | *** | ** |
| 125. | 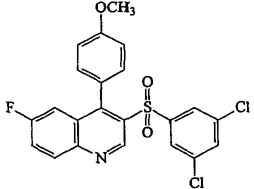 | 463.2 | *** | * |
| 126. | 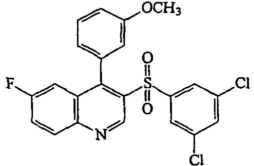 | 463.2 | *** | * |
| 127. | 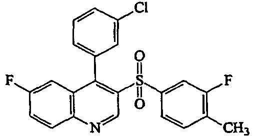 | 430.2 | *** | * |
| 128. | 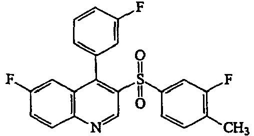 | 414.2 | *** | * |
| 129. | 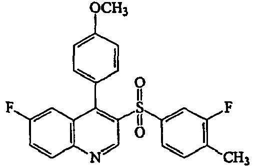 | 426.3 | *** | * |
| 130. | 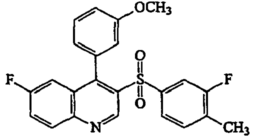 | 426.3 | *** | * |
| 131. | 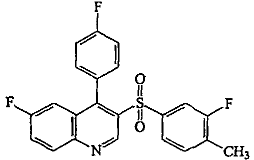 | 414.2 | *** | * |
| 132. | 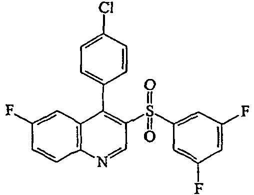 | 434.2 | ** | * |
| 133. | 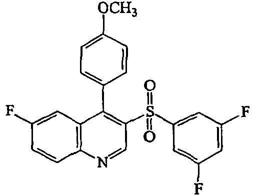 | 430.3 | ** | * |
| 134. | 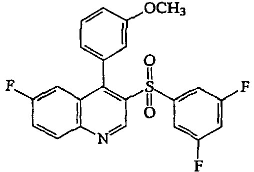 | 430.3 | ** | * |
| 135. | 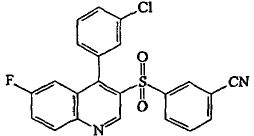 | 423.2 | *** | ** |
| 136. | 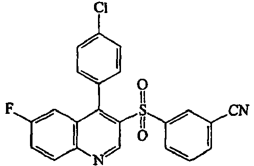 | 423.2 | *** | ** |
| 137. | 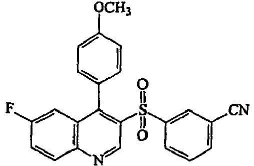 | 419.3 | *** | * |
| 138. | 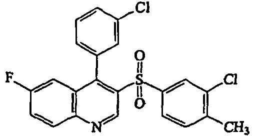 | 447.2 | *** | * |
| 139. | 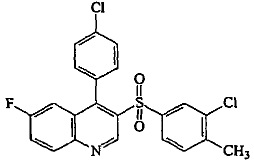 | 447.2 | *** | * |
| 140. | 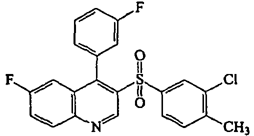 | 430.2 | *** | * |
| 141. | 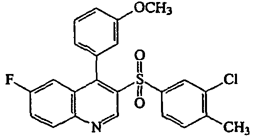 | 442.2 | *** | * |
| 142. | 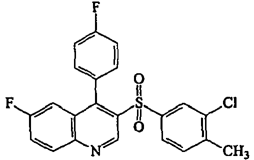 | 430.2 | *** | * |
| 143. | 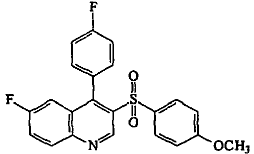 | 412.2 | ** | * |
| 144. | 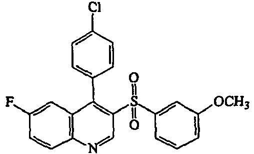 | 428.2 | *** | * |
| 145. | 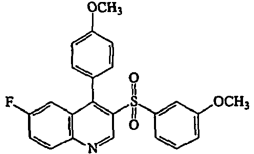 | 424.2 | *** | * |
| 146. | 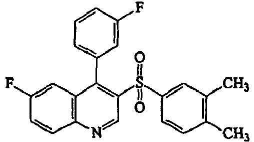 | 410.2 | *** | * |
| 147. | 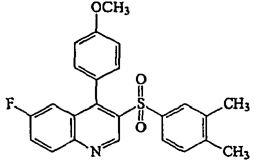 | 422.3 | *** | * |
| 148. | 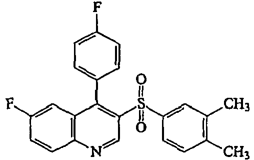 | 410.2 | *** | * |
| 149. | 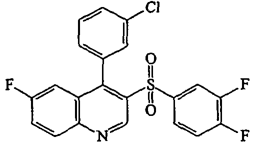 | 434.2 | ** | ** |
| 150. | 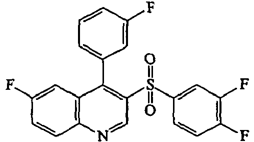 | 418.2 | *** | ** |
| 151. | 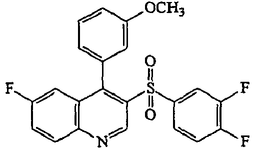 | 430.3 | ** | * |
| 152. | 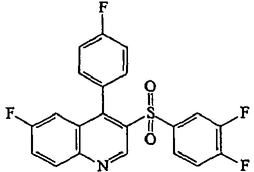 | 418.2 | *** | ** |
| 153. | 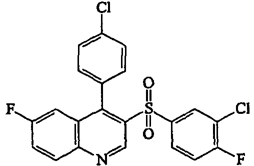 | 451.2 | *** | ** |
| 154. | 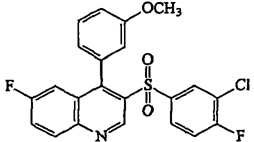 | 446.2 | *** | * |
| 155. | 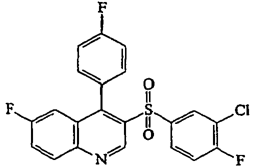 | 434.2 | *** | ** |
| 156. | 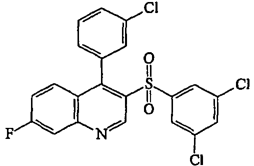 | 468.1 | *** | ** |
| 157. | 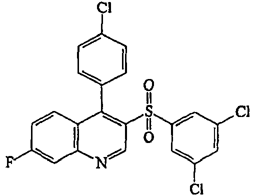 | 468.1 | *** | ** |
| 158. | 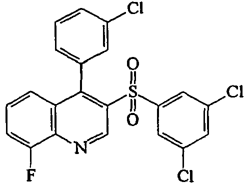 | 468.1 | *** | ** |
| 159. | 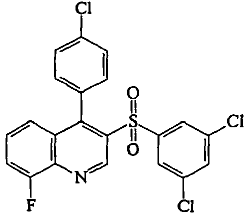 | 468.1 | *** | ** |
| 160. | 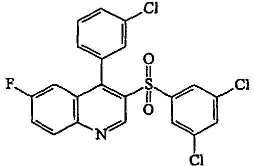 | 468.1 | *** | * |
| 161. | 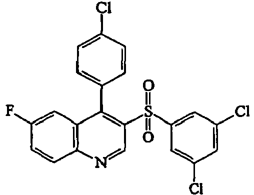 | 468.1 | *** | * |
| 162. | 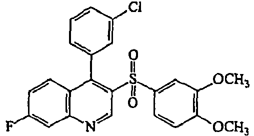 | 458.3 | * | * |
| 163. | 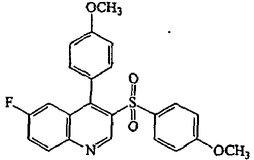 | 424.2 | *** | * |
| 164. | 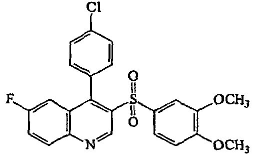 | 458.3 | * | * |
| 165. | 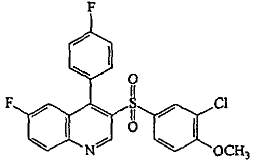 | 446.2 | * | * |
| 166. | 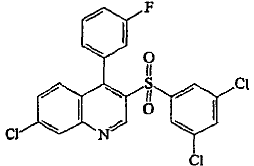 | 468.1 | *** | * |
| 167. | 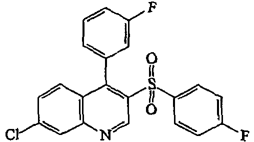 | 416.2 | *** | * |
| 168. | 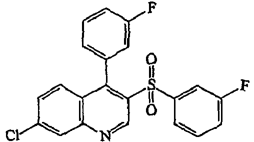 | 416.2 | *** | * |
| 169. | 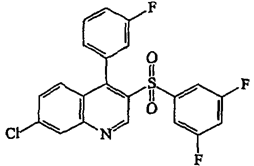 | 434.2 | *** | * |
| 170. | 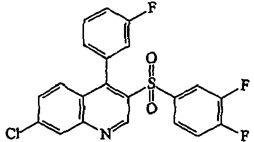 | 434.2 | *** | * |
| 171. | 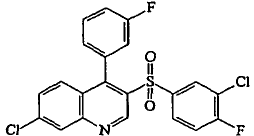 | 451.2 | *** | * |
| 172. | 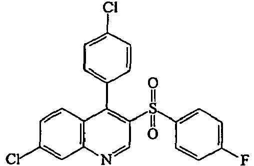 | 433.2 | *** | * |
| 173. | 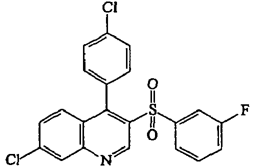 | 433.2 | *** | * |
| 174. | 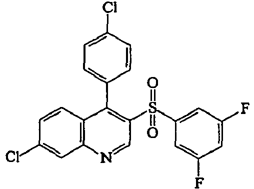 | 451.2 | *** | * |
| 175. | 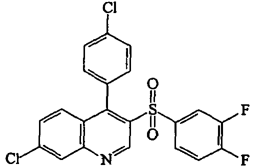 | 451.2 | *** | * |
| 176. | 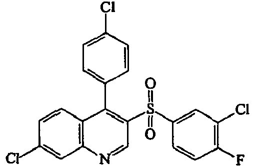 | 468.1 | *** | * |
| 177. | 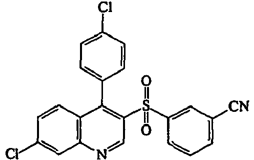 | 440.2 | *** | * |
| 178. | 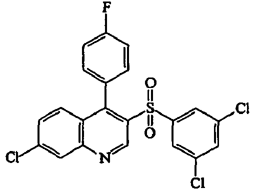 | 468.1 | *** | * |
| 179. | 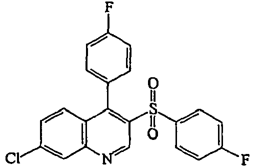 | 416.2 | *** | * |
| 180. | 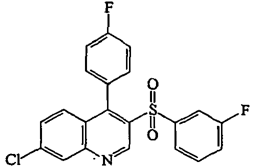 | 416.2 | *** | * |
| 181. | 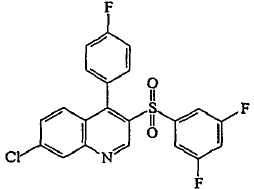 | 434.2 | *** | * |
| 182. | 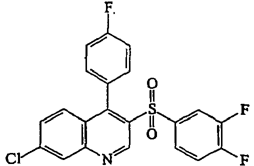 | 434.2 | *** | * |
| 183. | 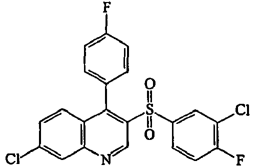 | 451.2 | *** | * |
| 184. | 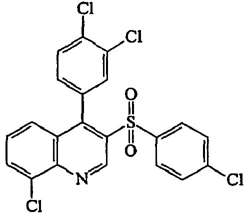 | 484.1 | * | * |
| 185. | 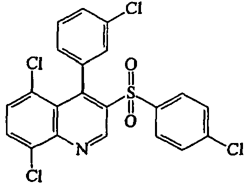 | 484.1 | * | * |
| 186. | 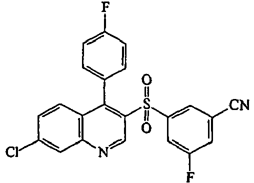 | 441.2 | *** | * |
| 187. | 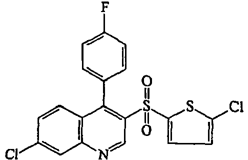 | 439.2 | ** | * |
| 188. | 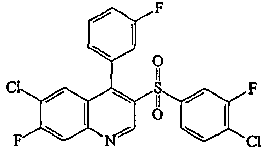 | 469.2 | ** | * |
| 189. | 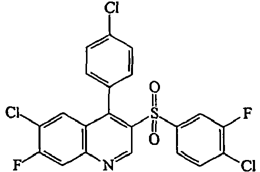 | 485.2 | *** | * |
| 190. | 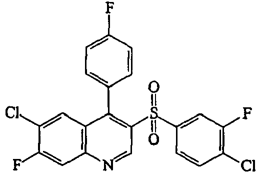 | 469.2 | *** | * |
| 191. | 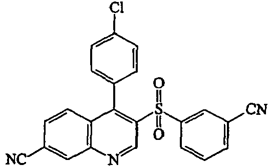 | 430.2 | ** | * |
| 192. | 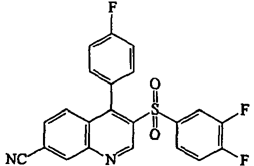 | 425.2 | * | * |
| 193. | 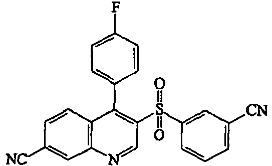 | 414.3 | ** | * |
| 194. | 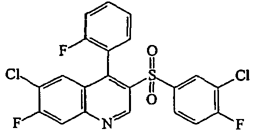 | 469.2 | * | * |
| 195. | 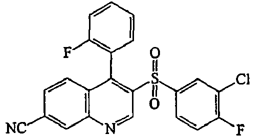 | 441.2 | *** | * |
| 196. | 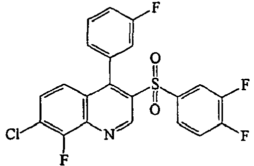 | 452.2 | *** | * |
| 197. | 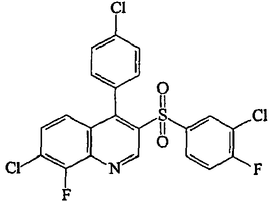 | 485.2 | *** | * |
| 198. | 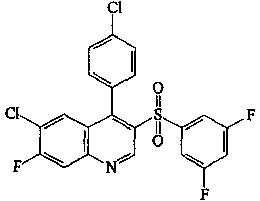 | 469.2 | * | * |
| 199. | 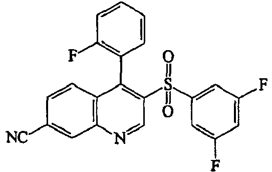 | 425.3 | ** | * |
| 200. | 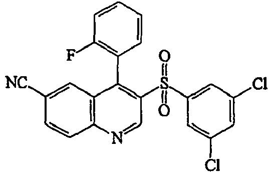 | 458.2 | * | * |
| 201. | 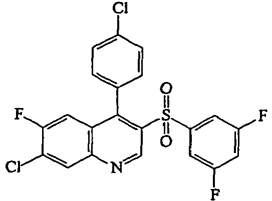 | 469.2 | * | * |
| 202. | 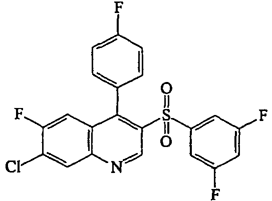 | 452.2 | ** | * |
| 203. | 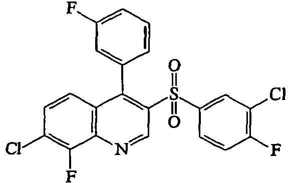 | 469.2 | *** | * |
| 204. | 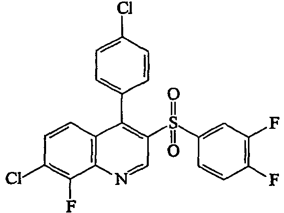 | 469.2 | *** | * |
| 205. | 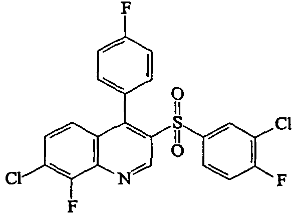 | 469.2 | *** | * |
| | | | | |
| 206. | 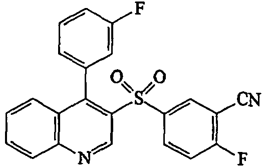 | 407.3 | *** | * |
| 207. | 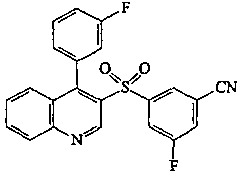 | 407.3 | *** | * |
| 208. | 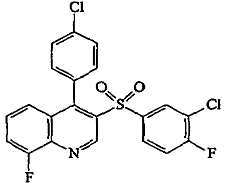 | 451.1 | *** | ** |
| 209. | 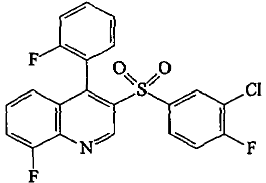 | 434.2 | *** | ** |
| 210. | 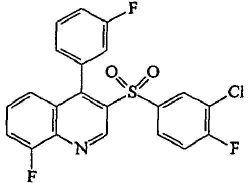 | 434.2 | *** | ** |
| 211. | 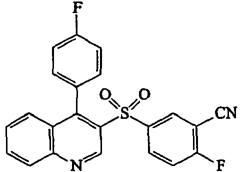 | 407.3 | *** | * |
| 212. | 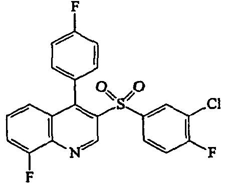 | 434.2 | *** | ** |
| 213. | 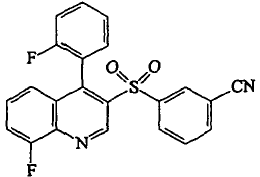 | 407.2 | *** | * |
| 214. | 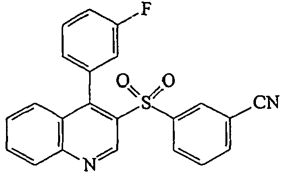 | 389.1 | *** | * |
| 215. | 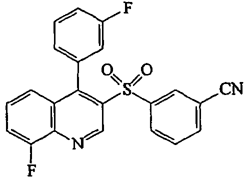 | 407.2 | *** | * |
| 216. | 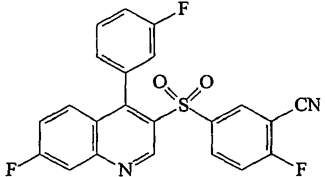 | 425.1 | *** | * |
| 217. | 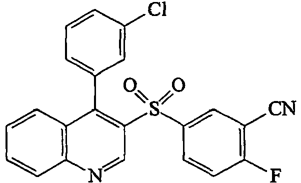 | 423.2 | *** | * |
| 218. | 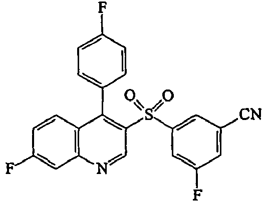 | 425.1 | *** | * |
| 219. | 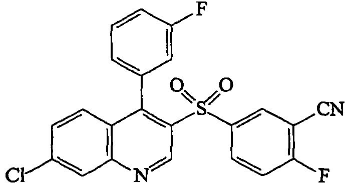 | 441.0 | *** | *** |
| 220. | 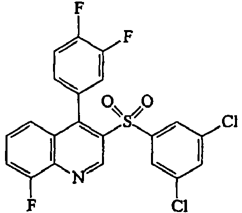 | 469.4 | *** | * |
| 221. | 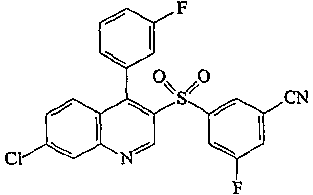 | 441.0 | *** | ** |
| 222. | 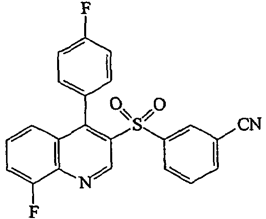 | 407.2 | *** | * |
| 223. | 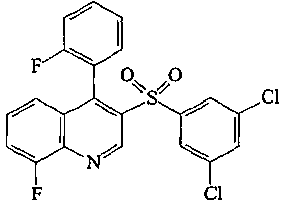 | 451.2 | *** | * |
| 224. | 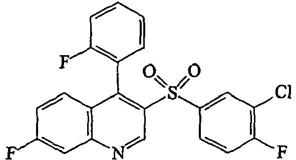 | 434.2 | *** | ** |
| 225. | 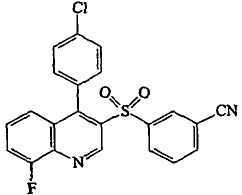 | 423.2 | *** | * |
| 226. | 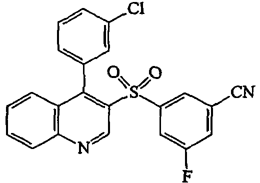 | 423.2 | *** | * |
| 227. | 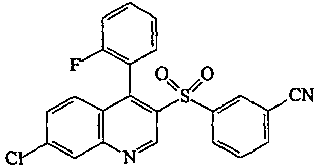 | 423.2 | *** | *** |
| 228. | 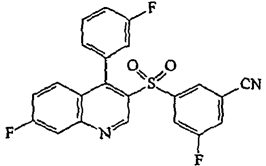 | 425.1 | *** | * |
| 229. | 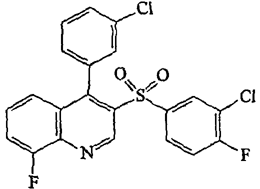 | 451.2 | *** | ** |
| 230. | 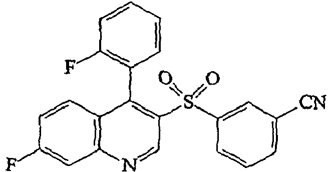 | 407.2 | *** | * |
| 231. | 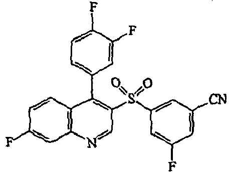 | 443.4 | *** | * |
| 232. | 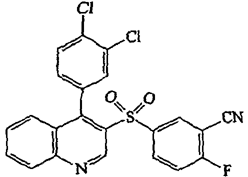 | 457.3 | *** | * |
| 33. | 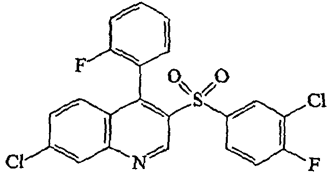 | 451.2 | *** | *** |
| 234. | 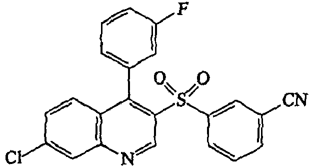 | 423.3 | *** | *** |
| 235. | 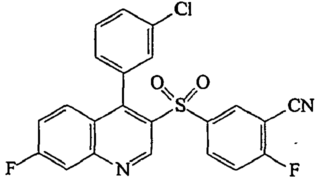 | 441.2 | *** | * |
| 236. | 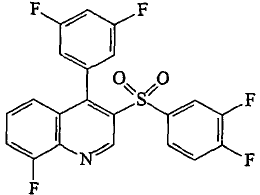 | 436.2 | *** | ** |
| 237. | 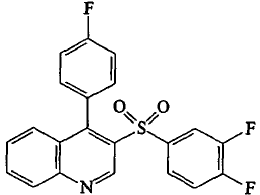 | 400.3 | *** | * |
| 238. | 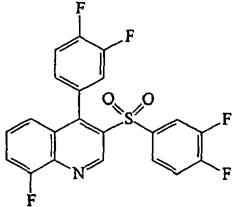 | 436.2 | *** | * |
| 239. | 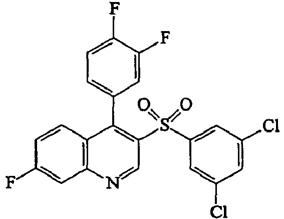 | 468.8 | *** | * |
| 240. | 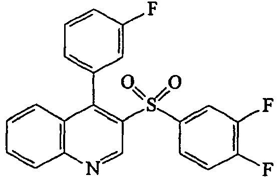 | 400.1 | *** | * |
| 241. | 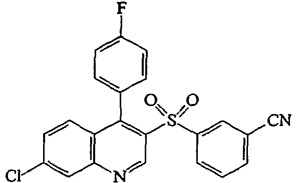 | 423.3 | *** | ** |
| 242. | 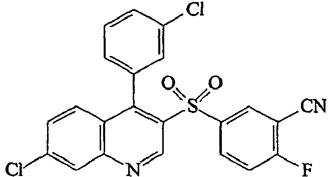 | 458.0 | *** | *** |
| 243. | 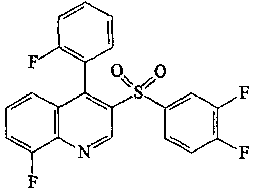 | 418.2 | *** | * |
| 244. | 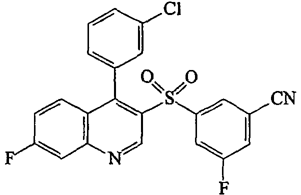 | 441.2 | *** | * |
| 245. | 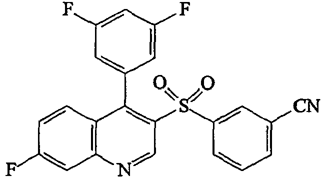 | 425.2 | *** | ** |
| 246. | 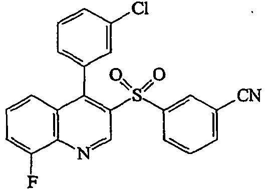 | 423.2 | *** | * |
| 247. | 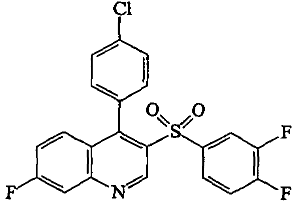 | 434.2 | *** | * |
| 248. | 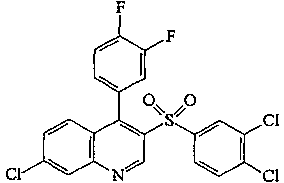 | 486.2 | *** | ** |
| 249. | 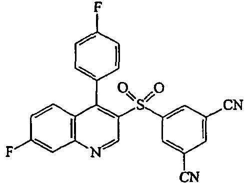 | 432.3 | *** | * |
| 250. | 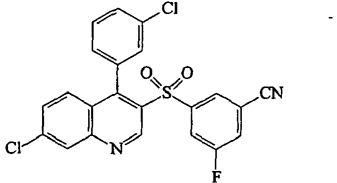 | 458.0 | *** | ** |
| 251. | 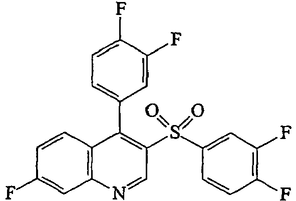 | 436.2 | *** | * |
| 252. | 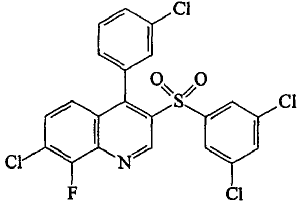 | 503.8 | *** | ** |
| 253. | 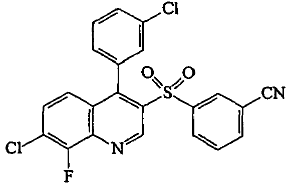 | 458.2 | *** | ** |
| 254. | 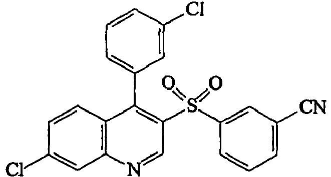 | 440.1 | *** | ** |
| 255. | 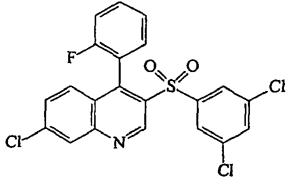 | 468.1 | *** | ** |
| 256. | 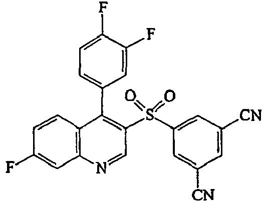 | 450.3 | *** | * |
| 257. | 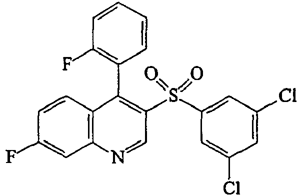 | 450.8 | *** | * |
| 258. | 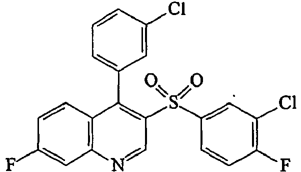 | 451.1 | *** | ** |
| 259. | 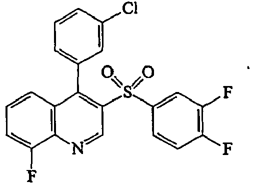 | 434.2 | *** | * |
| 260. | 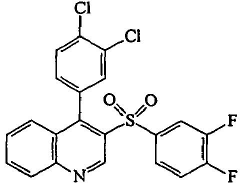 | 451.3 | *** | * |
| 261. | 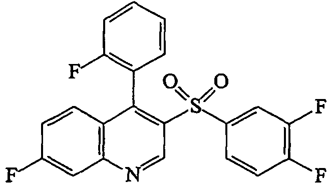 | 417.9 | *** | * |
| 262. | 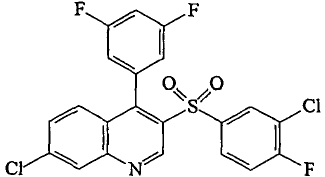 | 469.4 | *** | *** |
| 263. | 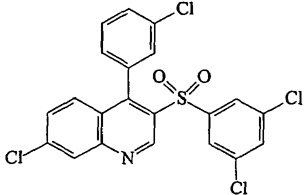 | 484.2 | *** | ** |
| 264. | 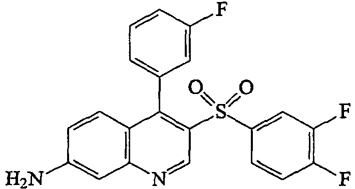 | 415.1 | *** | ** |
| 265. | 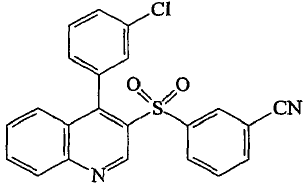 | 405.0 | *** | * |
| 266. | 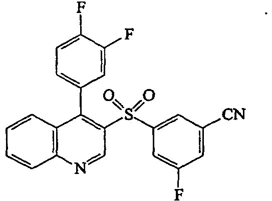 | 425.1 | *** | * |
| 267. | 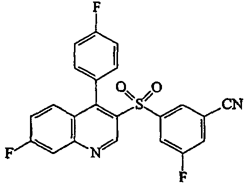 | 425.2 | *** | * |
| 268. | 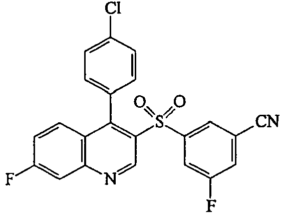 | 441.2 | *** | * |
| 269. | 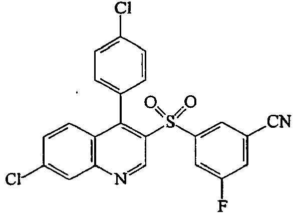 | 458.2 | *** | * |
| 270. | 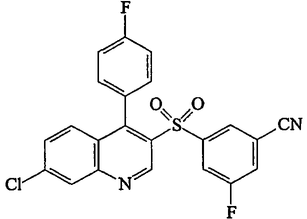 | 441.2 | *** | * |
| 271. | 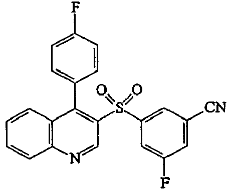 | 407.3 | *** | * |
| 272. | 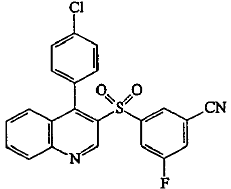 | 423.2 | *** | * |
| 273. | 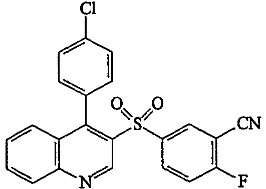 | 423.2 | *** | * |
| 274. | 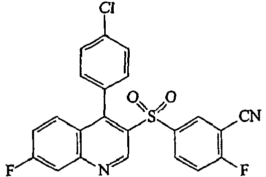 | 441.2 | *** | * |
| 275. | 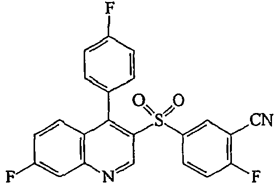 | 425.2 | *** | * |
| 276. | 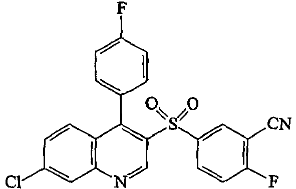 | 441.2 | *** | ** |
| 277. | 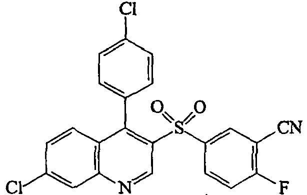 | 458.2 | *** | ** |
| 278. | 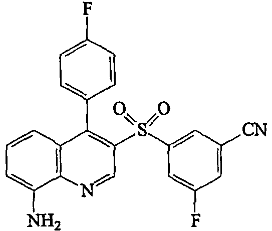 | 422.1 | *** | * |
| 279. | 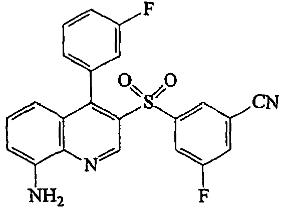 | 422.1 | *** | * |

| | | | | |
|---|---|---|---|---|
| *** Kᵢ < 200 nM ** 200nM < Kᵢ < 500 nM * 500 nM < Kᵢ | | | | |

### Example 8

### Preparation of pharmaceutical compositions:

### a) Tablets:

0.01-50 % of active ingredient of formula (I), 15-50 % of lactose, 15-50 % of potato starch, 5-15 % of polyvinyl pyrrolidone, 1-5 % of talc, 0.01-3 % of magnesium stearate, 1-3 % of colloid silicon dioxide and 2-7 % of ultraamylopectin were mixed, then granulated by wet granulation and pressed to tablets.

### b) Dragées, film coated tablets:

The tablets made according to the method described above were coated by a layer consisting of entero- or gastrosolvent film, or of sugar and talc. The dragées were polished by a mixture of beeswax and carnuba wax.

### c) Capsules:

0.01-50 % of active ingredient of formula (I), 1-5 % of sodium lauryl sulfate, 15-50 % of starch, 15-50 % of lactose, 1-3 % of colloid silicon dioxide and 0.01-3 % of magnesium stearate were thoroughly mixed, the mixture was passed through a sieve and filled in hard gelatin capsules.

### d) Suspensions:

Ingredients: 0.01-15 % of active ingredient of formula (I), 0.1-2 % of sodium hydroxide, 0.1-3 % of citric acid, 0.05-0.2 % of nipagin (sodium methyl 4-hydroxybenzoate), 0.005-0.02 % of nipasol, 0.01-0.5 % of carbopol (polyacrilic acid), 0.1-5 % of 96 % ethanol, 0.1-1 % of flavoring agent, 20-70 % of sorbitol (70 % aqueous solution) and 30-50 % of distilled water.

To solution of nipagin and citric acid in 20 ml of distilled water, carbopol was added in small portions under vigorous stirring, and the solution was left to stand for 10-12 h. Then the sodium hydroxide in 1 ml of distilled water, the aqueous solution of sorbitol and finally the ethanolic raspberry flavor were added with stirring. To this carrier the active ingredient was added in small portions and suspended with an immersing homogenizator. Finally the suspension was filled up to the desired final volume with distilled water and the suspension syrup was passed through a colloid milling equipment.

### e) Suppositories:

For each suppository 0.01-15% of active ingredient of formula (I) and 1-20% of lactose were thoroughly mixed, then 50-95% of adeps pro suppository (for example Witepsol 4) was melted, cooled to 35 °C and the mixture of active ingredient and lactose was mixed in it with homogenizator. The obtained mixture was mould in cooled forms.

### f) Lyophilized powder ampoule compositions:

A 5 % solution of mannitol or lactose was made with bidistilled water for injection use, and the solution was filtered so as to have sterile solution. A 0.01-5 % solution of the active ingredient of formula (I) was also made with bidistilled water for injection use, and this solution was filtered so as to have sterile solution. These two solutions were mixed under aseptic conditions, filled in 1 ml portions into ampoules, the content of the ampoules was lyophilized, and the ampoules were sealed under nitrogen. The contents of the ampoules were dissolved in sterile water or 0.9 % (physiological) sterile aqueous sodium chloride solution before administration.

### Example 9

### 3-(3,4-Difluoro-benzenesulfonyl)-7-nitro-quinolin-4-ol (intermediate)

The title compound was prepared applying the procedure described in Example 4 from 2-(3,4-difluoro-benzenesulfonyl)-3-ethoxy-acrylic acid methyl ester (3.49 g, 11.4 mmol) and 3-nitroaniline (1.57 g, 11.4 mmol). The yield was 1.6 g (38.3%).

MS (EI) M⁺= 367.2.

In the same way was prepared: e.g. 3-(3-cyano-5-fluoro-benzenesulfonyl)-8-nitro-quinolin-4-ol (MS (EI) M⁺= 374.3).

### Example 10

### 4-Bromo-3-(3,4-difluoro-benzenesulfonyl)-7-nitro-quinoline (intermediate)

A mixture of 3-(3,4-difluoro-benzenesulfonyl)-7-nitro-quinolin-4-ol (1.6 g, 4.37 mmol) and phosphorus(V) oxybromide (2.5 g, 8.72 mmol) in DMF (16 ml) was stirred at 65°C for 1 hour. The reaction mixture was diluted with water (100 ml) and the pH was adjusted to 10 with aqueous sodium hydroxide solution. After cooling the precipitate was filtered and washed with water. The obtained crude product was purified by crystallization from ethanol to obtain 1.25 g of the title compound in 67% yield.

MS (EI) M⁺= 430.9.

Applying the above procedure the following compound was prepared: e.g. 4-bromo-3-(3-cyano-5-fluoro-benzenesulfonyl)-8-nitro-quinoline (MS (EI) M⁺ = 437.2).

### Example 11

### 3-(3,4-Difluoro-benzenesulfonyl)-4-(3-fluoro-phenyl)-7-nitro-quinoline (intermediate)

The title compound was prepared applying the procedure described in Example 7 from 4-Bromo-3-(3,4-difluoro-benzenesulfonyl)-7-nitro-quinoline (0.53 g, 1.23 mmol) and 3-fluorophenylboronic acid (0.21 g, 1.5 mmol). The crude product was purified by column chromatography on silica gel (Kieselgel 60, eluent: chloroform) and crystallized from methanol to obtain 0.31 g of the title compound in 57% yield.

MS (EI) M⁺ = 445.3.

In the same way were prepared: e.g. 3-(3-cyano-5-fluoro-benzenesulfonyl)-4-(4-fluoro-phenyl)-8-nitro-quinoline (MS (EI) M⁺ = 452.3); 3-(3-cyano-5-fluoro-benzenesulfonyl)-4-(3-fluoro-phenyl)-8-nitro-quinoline (MS (EI) M⁺ = 452.3).

### Example 12

### 7-Amino-3-(3,4-difluoro-benzenesulfonyl)-4-(3-fluoro-phenyl)-quinoline

### Table I compound 264

A mixture of 3-(3,4-difluoro-benzenesulfonyl)-4-(3-fluoro-phenyl)-7-nitro-quinoline (0.31 g, 0.69 mmol) and iron powder (0.15 g, 2.6 mmol) in acetic acid was stirred for 30 minutes at 60°C. The reaction mixture was diluted with water (5 ml). The precipitate was filtered and washed with water (2x5 ml). The obtained crude product was purified by crystallization from methanol to obtain 0.12 g of the title compound in 42% yield.

MS (EI) M⁺= 415.1.

Applying the above procedure the following compounds were prepared: e.g. 7-amino-3-(3-chloro-5-fluoro-benzenesulfonyl)-4-(4-fluoro-phenyl)-quinoline (MS (EI) M⁺ = 422.1); 7-amino-3-(3-chloro-5-fluoro-benzenesulfonyl)-4-(3-fluoro-phenyl)-quinoline (MS (EI) M⁺ = 422.1).

## Claims

1. A compound having the formula (I): 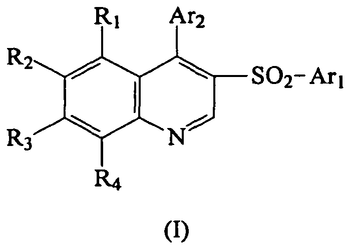 wherein
Ar₁ represents phenyl or thienyl group, optionally substituted with one or more substituent(s) selected from hydrogen, fluoro, chloro, cyano, methyl, methoxy;
Ar₂ represents phenyl, substituted with one or more substituent(s) selected from fluoro, chloro, cyano, methyl, methoxy;
R₁, R₂, R₃ and R₄ represent independently a substituent selected from hydrogen,
fluoro, chloro, cyano, methyl, methoxy, hydroxy, trifluoromethyl, amino, methylamino, dimethylamino, aminomethyl, methylaminomethyl, dimethylaminomethyl,
and/or salts and/or hydrates and/or solvates thereof.

2. A compound according to claim 1 selected from the group consisting of
4-(4-chloro-phenyl)-3-(4-methyl-benzenesulfonyl)-quinoline,
7-chloro-3-(4-chloro-benzenesulfonyl)-4-(4-fluoro-phenyl)-quinoline,
8-chloro-4-(3-chloro-phenyl)-3-(3,4-dichloro-benzenesulfonyl)-quinoline,
7-fluoro-3-(4-fluoro-benzenesulfonyl)-4-(3 -fluoro-phenyl)-quinoline,
4-(4-chloro-phenyl)-7-fluoro-3-(4-methoxy-benzenesulfonyl)-quinoline,
7-fluoro-3-(4-methoxy-benzenesulfonyl)-4-(4-methoxy-phenyl)-quinoline,
7-fluoro-3-(3-fluoro-benzenesulfonyl)-4-(3-fluoro-phenyl)-quinoline,
7-fluoro-3-(3-fluoro-benzenesulfonyl)-4-(4-fluoro-phenyl)-quinoline,
4-(3-chloro-phenyl)-3-(3,4-dimethyl-benzenesulfonyl)-7-fluoro-quinoline,
3-(3,4-dimethyl-benzenesulfonyl)-7-fluoro-4-(3-fluoro-phenyl)-quinoline,
3-(3,4-dimethyl-benzenesulfonyl)-7-fluoro-4-(3-methoxy-phenyl)-quinoline,
4-(3-chloro-phenyl)-8-fluoro-3-(4-fluoro-benzenesulfonyl)-quinoline,
4-(4-chloro-phenyl)-8-fluoro-3-(4-fluoro-benzenesulfonyl)-quinoline,
8-fluoro-3-(4-fluoro-benzenesulfonyl)-4-(3-fluoro-phenyl)-quinoline,
8-fluoro-3-(4-fluoro-benzenesulfonyl)-4-(3 -methoxy-phenyl)-quinoline,
4-(4-chloro-phenyl)-6-fluoro-3-(4-methoxy-benzenesulfonyl)-quinoline,
4-(4-chloro-phenyl)-3-(3,4-dimethyl-benzenesulfonyl)-6-fluoro-quinoline,
4-(4-chloro-phenyl)-3-(3,5-difluoro-benzenesulfonyl)-7-fluoro-quinoline,
3-(3,5-difluoro-benzenesulfonyl)-7-fluoro-4-(4-fluoro-phenyl)-quinoline,
4-(4-chloro-phenyl)-3-(3-cyano-benzenesulfonyl)-7-fluoro-quinoline,
3-(3-cyano-benzenesulfonyl)-7-fluoro-4-(3-fluoro-phenyl)-quinoline,
3-(3-cyano-benzenesulfonyl)-7-fluoro-4-(4-fluoro-phenyl)-quinoline,
7-fluoro-3-(4-fluoro-benzenesulfonyl)-4-(4-fluoro-phenyl)-quinoline,
4-(4-chloro-phenyl)-7-fluoro-3-(3-fluoro-benzenesulfonyl)-quinoline,
4-(3-chloro-phenyl)-7-fluoro-3-(3-methoxy-benzenesulfonyl)-quinoline,
7-fluoro-4-(4-fluoro-phenyl)-3-(3-methoxy-benzenesulfonyl)-quinoline,
4-(4-chloro-phenyl)-3-(3,4-dimethyl-benzenesulfonyl)-7-fluoro-quinoline,
3-(3,4-dimethyl-benzenesulfonyl)-7-fluoro-4-(4-fluoro-phenyl)-quinoline,
4-(3-chloro-phenyl)-3-(3-chloro-4-methoxy-benzenesulfonyl)-7-fluoro-quinoline,
3-(3-chloro-4-methoxy-benzenesulfonyl)-7-fluoro-4-(3-fluoro-phenyl)-quinoline,
3-(3-chloro-4-methoxy-benzenesulfonyl)-7-fluoro-4-(4-fluoro-phenyl)-quinoline,
3-(3,5-dichloro-benzenesulfonyl)-8-fluoro-4-(4-fluoro-phenyl)-quinoline,
3-(3,5-difluoro-benzenesulfonyl)-8-fluoro-4-(3-fluoro-phenyl)-quinoline,
8-fluoro-3-(4-fluoro-benzenesulfonyl)-4-(4-methoxy-phenyl)-quinoline,
4-(3-chloro-phenyl)-8-fluoro-3-(4-methoxy-benzenesulfonyl)-quinoline,
4-(4-chloro-phenyl)-8-fluoro-3-(4-methoxy-benzenesulfonyl)-quinoline,
8-fluoro-4-(4-fluoro-phenyl)-3-(4-methoxy-benzenesulfonyl)-quinoline,
8-fluoro-3-(4-methoxy-benzenesulfonyl)-4-(4-methoxy-phenyl)-quinoline,
8-fluoro-3-(4-methoxy-benzenesulfonyl)-4-(3-methoxy-phenyl)-quinoline,
4-(3-chloro-phenyl)-8-fluoro-3-(3-fluoro-benzenesulfonyl)-quinoline,
4-(4-chloro-phenyl)-8-fluoro-3-(3-fluoro-benzenesulfonyl)-quinoline,
8-fluoro-3-(3-fluoro-benzenesulfonyl)-4-(3-fluoro-phenyl)-quinoline,
8-fluoro-3-(3-fluoro-benzenesulfonyl)-4-(4-methoxy-phenyl)-quinoline,
8-fluoro-3-(3-fluoro-benzenesulfonyl)-4-(3-methoxy-phenyl)-quinoline,
8-fluoro-3-(3-fluoro-benzenesulfonyl)-4-(4-fluoro-phenyl)-quinoline,
3-(3-cyano-benzenesulfonyl)-6-fluoro-4-(3-fluoro-phenyl)-quinoline,
3-(3-cyano-benzenesulfonyl)-6-fluoro-4-(3-methoxy-phenyl)-quinoline,
3-(3-cyano-benzenesulfonyl)-6-fluoro-4-(4-fluoro-phenyl)-quinoline,
4-(3-chloro-phenyl)-6-fluoro-3-(3-methoxy-benzenesulfonyl)-quinoline,
6-fluoro-4-(4-fluoro-phenyl)-3-(3-methoxy-benzenesulfonyl)-quinoline,
3-(3-chloro-4-methoxy-benzenesulfonyl)-6-fluoro-4-(4-methoxy-phenyl)-quinoline,
3-(3-chloro-4-methoxy-benzenesulfonyl)-6-fluoro-4-(3-methoxy-phenyl)-quinoline,
4-(4-chloro-phenyl)-7-fluoro-3-(4-fluoro-benzenesulfonyl)-quinoline,
3-(3,4-dimethyl-benzenesulfonyl)-7-fluoro-4-(4-methoxy-phenyl)-quinoline,
3-(3-chloro-4-methoxy-benzenesulfonyl)-4-(4-chloro-phenyl)-6-fluoro-quinoline,
3-(3-chloro-4-methoxy-benzenesulfonyl)-6-fluoro-4-(3-fluoro-phenyl)-quinoline,
3-(3,5-dichloro-benzenesulfonyl)-7-fluoro-4-(4-methoxy-phenyl)-quinoline,
3-(3,5-dichloro-benzenesulfonyl)-7-fluoro-4-(3-methoxy-phenyl)-quinoline,
3-(3,5-difluoro-benzenesulfonyl)-7-fluoro-4-(4-fluoro-phenyl)-quinoline,
4-(3-chloro-phenyl)-7-fluoro-3-(3-fluoro-4-methyl-benzenesulfonyl)-quinoline,
7-fluoro-3-(3-fluoro-4-methyl-benzenesulfonyl)-4-(3-fluoro-phenyl)-quinoline,
7-fluoro-3-(3-fluoro-4-methyl-benzenesulfonyl)-4-(4-fluoro-phenyl)-quinoline,
3-(3,5-difluoro-benzenesulfonyl)-7-fluoro-4-(3-fluoro-phenyl)-quinoline,
3-(3,5-difluoro-benzenesulfonyl)-7-fluoro-4-(4-methoxy-phenyl)-quinoline,
3-(3,5-difluoro-benzenesulfonyl)-7-fluoro-4-(3-methoxy-phenyl)-quinoline,
4-(3-chloro-phenyl)-3-(3-cyano-benzenesulfonyl)-7-fluoro-quinoline,
3-(3-cyano-benzenesulfonyl)-7-fluoro-4-(4-methoxy-phenyl)-quinoline,
3-(3-cyano-benzenesulfonyl)-7-fluoro-4-(3-methoxy-phenyl)-quinoline,
3-(3-chloro-4-methyl-benzenesulfonyl)-7-fluoro-4-(3-fluoro-phenyl)-quinoline,
3-(3,4-difluoro-benzenesulfonyl)-7-fluoro-4-(3-fluoro-phenyl)-quinoline,
3-(3,4-difluoro-benzenesulfonyl)-7-fluoro-4-(4-methoxy-phenyl)-quinoline,
3-(3,4-difluoro-benzenesulfonyl)-7-fluoro-4-(3-methoxy-phenyl)-quinoline,
3-(3,4-difluoro-benzenesulfonyl)-7-fluoro-4-(4-fluoro-phenyl)-quinoline,
3-(3-chloro-4-fluoro-benzenesulfonyl)-4-(4-chloro-phenyl)-7-fluoro-quinoline,
3-(3-chloro-4-fluoro-benzenesulfonyl)-7-fluoro-4-(3-fluoro-phenyl)-quinoline,
3-(3-chloro-4-fluoro-benzenesulfonyl)-7-fluoro-4-(4-methoxy-phenyl)-quinoline,
3-(3-chloro-4-fluoro-benzenesulfonyl)-7-fluoro-4-(4-fluoro-phenyl)-quinoline,
3-(3,5-dichloro-benzenesulfonyl)-8-fluoro-4-(3-fluoro-phenyl)-quinoline,
3-(3,5-dichloro-benzenesulfonyl)-8-fluoro-4-(4-methoxy-phenyl)-quinoline,
3-(3,5-dichloro-benzenesulfonyl)-8-fluoro-4-(3-methoxy-phenyl)-quinoline,
4-(3-chloro-phenyl)-8-fluoro-3-(3-fluoro-4-methyl-benzenesulfonyl)-quinoline,
4-(4-chloro-phenyl)-8-fluoro-3-(3-fluoro-4-methyl-benzenesulfonyl)-quinoline,
8-fluoro-3-(3-fluoro-4-methyl-benzenesulfonyl)-4-(3-fluoro-phenyl)-quinoline,
8-fluoro-3-(3-fluoro-4-methyl-benzenesulfonyl)-4-(4-methoxy-phenyl)-quinoline,
8-fluoro-3-(3-fluoro-4-methyl-benzenesulfonyl)-4-(3-methoxy-phenyl)-quinoline,
8-fluoro-3-(3-fluoro-4-methyl-benzenesulfonyl)-4-(4-fluoro-phenyl)-quinoline,
4-(3-chloro-phenyl)-3-(3,5-difluoro-benzenesulfonyl)-8-fluoro-quinoline,
4-(4-chloro-phenyl)-3-(3,5-difluoro-benzenesulfonyl)-8-fluoro-quinoline,
3-(3,5-difluoro-benzenesulfonyl)-8-fluoro-4-(4-methoxy-phenyl)-quinoline,
3-(3,5-difluoro-benzenesulfonyl)-8-fluoro-4-(3-methoxy-phenyl)-quinoline,
3-(3,5-difluoro-benzenesulfonyl)-8-fluoro-4-(4-fluoro-phenyl)-quinoline,
4-(4-chloro-phenyl)-3-(3,4-difluoro-benzenesulfonyl)-8-fluoro-quinoline,
3-(3,4-difluoro-benzenesulfonyl)-8-fluoro-4-(3-fluoro-phenyl)-quinoline,
3-(3,4-difluoro-benzenesulfonyl)-8-fluoro-4-(4-methoxy-phenyl)-quinoline,
3-(3,4-difluoro-benzenesulfonyl)-8-fluoro-4-(4-fluoro-phenyl)-quinoline,
3-(3,5-dichloro-benzenesulfonyl)-6-fluoro-4-(3-fluoro-phenyl)-quinoline,
3-(3,5-dichloro-benzenesulfonyl)-6-fluoro-4-(4-methoxy-phenyl)-quinoline,
3-(3,5-dichloro-benzenesulfonyl)-6-fluoro-4-(3-methoxy-phenyl)-quinoline,
4-(3-chloro-phenyl)-6-fluoro-3-(3-fluoro-4-methyl-benzenesulfonyl)-quinoline,
6-fluoro-3-(3-fluoro-4-methyl-benzenesulfonyl)-4-(3-fluoro-phenyl)-quinoline,
6-fluoro-3-(3-fluoro-4-methyl-benzenesulfonyl)-4-(4-methoxy-phenyl)-quinoline,
6-fluoro-3-(3-fluoro-4-methyl-benzenesulfonyl)-4-(3-methoxy-phenyl)-quinoline,
6-fluoro-3-(3-fluoro-4-methyl-benzenesulfonyl)-4-(4-fluoro-phenyl)-quinoline,
4-(3-chloro-phenyl)-3-(3-cyano-benzenesulfonyl)-6-fluoro-quinoline,
4-(4-chloro-phenyl)-3-(3-cyano-benzenesulfonyl)-6-fluoro-quinoline,
3-(3-cyano-benzenesulfonyl)-6-fluoro-4-(4-methoxy-phenyl)-quinoline,
3-(3-chloro-4-methyl-benzenesulfonyl)-4-(3-chloro-phenyl)-6-fluoro-quinoline,
3-(3-chloro-4-methyl-benzenesulfonyl)-4-(4-chloro-phenyl)-6-fluoro-quinoline,
3-(3-chloro-4-methyl-benzenesulfonyl)-6-fluoro-4-(3-fluoro-phenyl)-quinoline,
3-(3-chloro-4-methyl-benzenesulfonyl)-6-fluoro-4-(3-methoxy-phenyl)-quinoline,
3-(3-chloro-4-methyl-benzenesulfonyl)-6-fluoro-4-(4-fluoro-phenyl)-quinoline,
4-(4-chloro-phenyl)-6-fluoro-3-(3-methoxy-benzenesulfonyl)-quinoline,
6-fluoro-3-(3-methoxy-benzenesulfonyl)-4-(3-methoxy-phenyl)-quinoline,
3-(3,4-dimethyl-benzenesulfonyl)-6-fluoro-4-(3-fluoro-phenyl)-quinoline,
3-(3,4-dimethyl-benzenesulfonyl)-6-fluoro-4-(4-methoxy-phenyl)-quinoline,
3-(3,4-dimethyl-benzenesulfonyl)-6-fluoro-4-(4-fluoro-phenyl)-quinoline,
3-(3,4-difluoro-benzenesulfonyl)-6-fluoro-4-(3-fluoro-phenyl)-quinoline,
3-(3,4-difluoro-benzenesulfonyl)-6-fluoro-4-(4-fluoro-phenyl)-quinoline,
3-(3-chloro-4-fluoro-benzenesulfonyl)-4-(4-chloro-phenyl)-6-fluoro-quinoline,
3-(3-chloro-4-fluoro-benzenesulfonyl)-6-fluoro-4-(3-methoxy-phenyl)-quinoline,
3-(3-chloro-4-fluoro-benzenesulfonyl)-6-fluoro-4-(4-fluoro-phenyl)-quinoline,
4-(3-chloro-phenyl)-3-(3,5-dichloro-benzenesulfonyl)-7-fluoro-quinoline,
4-(4-chloro-phenyl)-3-(3,5-dichloro-benzenesulfonyl)-7-fluoro-quinoline,
4-(3-chloro-phenyl)-3-(3,5-dichloro-benzenesulfonyl)-8-fluoro-quinoline,
4-(4-chloro-phenyl)-3-(3, 5-dichloro-benzenesulfonyl)-8-fluoro-quinoline,
4-(3-chloro-phenyl)-3-(3,5-dichloro-benzenesulfonyl)-6-fluoro-quinoline,
4-(4-chloro-phenyl)-3-(3,5-dichloro-benzenesulfonyl)-6-fluoro-quinoline,
6-fluoro-3-(4-methoxy-benzenesulfonyl)-4-(4-methoxy-phenyl)-quinoline,
7-chloro-4-(4-chloro-phenyl)-3-(3,5-difluoro-benzenesulfonyl)-quinoline,
7-chloro-4-(4-chloro-phenyl)-3-(3,4-difluoro-benzenesulfonyl)-quinoline,
7-chloro-4-(4-chloro-phenyl)-3-(3-cyano-benzenesulfonyl)-quinoline,
7-chloro-3-(3,5-dichloro-benzenesulfonyl)-4-(4-fluoro-phenyl)-quinoline,
7-chloro-3-(4-fluoro-benzenesulfonyl)-4-(4-fluoro-phenyl)-quinoline,
7-chloro-3-(3-fluoro-benzenesulfonyl)-4-(4-fluoro-phenyl)-quinoline,
7-chloro-3-(3,4-difluoro-benzenesulfonyl)-4-(4-fluoro-phenyl)-quinoline,
7-chloro-3-(3-chloro-4-fluoro-benzenesulfonyl)-4-(4-fluoro-phenyl)-quinoline and
7-chloro-3-(3-cyano-5-fluoro-benzenesulfonyl)-4-(4-fluoro-phenyl)-quinoline
6-chloro-3-(3-chloro-4-fluoro-benzenesulfonyl)-4-(4-chloro-phenyl)-7-fluoro-quinoline,
6-chloro-3-(3-chloro-4-fluoro-benzenesulfonyl)-7-fluoro-4-(4-fluoro-phenyl)-quinoline,
3-(3-chloro-4-fluoro-benzenesulfonyl)-7-cyano-4-(2-fluoro-phenyl)-quinoline,
7-chloro-3-(3,4-difluoro-benzenesulfonyl)-8-fluoro-4-(3-fluoro-phenyl)-quinoline,
7-chloro-3-(3-chloro-4-fluoro-benzenesulfonyl)-4-(4-fluoro-phenyl)-8-fluoro-quinoline,
7-chloro-3-(3-chloro-4-fluoro-benzenesulfonyl)-8-fluoro-4-(3-fluoro-phenyl)-quinoline,
7-chloro-4-(4-chloro-phenyl)-3-(3,4-difluoro-benzenesulfonyl)-8-fluoro-quinoline,
7-chloro-3-(3-chloro-4-fluoro-benzenesulfonyl)-8-fluoro-4-(4-fluoro-phenyl)-quinoline.
3-(3-cyano-4-fluoro-benzenesulfonyl)-4-(3-fluoro-phenyl)-quinoline,
3-(3-cyano-5-fluoro-benzenesulfonyl)-4-(3-fluoro-phenyl)-quinoline,
3-(3-chloro-4-fluoro-benzenesulfonyl)-4-(4-chloro-phenyl)-8-fluoro-quinoline,
3-(3-chloro-4-fluoro-benzenesulfonyl)-8-fluoro-4-(2-fluoro-phenyl)-quinoline,
3-(3-chloro-4-fluoro-benzenesulfonyl)-8-fluoro-4-(3-fluoro-phenyl)-quinoline,
3-(3-cyano-4-fluoro-benzenesulfonyl)-4-(4-fluoro-phenyl)-quinoline,
3-(3-chloro-4-fluoro-benzenesulfonyl)-8-fluoro-4-(4-fluoro-phenyl)-quinoline,
3-(3-cyano-benzenesulfonyl)-8-fluoro-4-(2-fluoro-phenyl)-quinoline,
3-(3-cyano-benzenesulfonyl)-4-(3-fluoro-phenyl)-quinoline,
3-(3-cyano-benzenesulfonyl)-8-fluoro-4-(3-fluoro-phenyl)-quinoline,
3-(3-cyano-4-fluoro-benzenesulfonyl)-7-fluoro-4-(3-fluoro-phenyl)-quinoline,
4-(3-chloro-phenyl)-3-(3-cyano-4-fluoro-benzenesulfonyl)-quinoline,
3-(3-cyano-5-fluoro-benzenesulfonyl)-7-fluoro-4-(4-fluoro-phenyl)-quinoline,
7-chloro-3-(3-cyano-4-fluoro-benzenesulfonyl)-4-(3-fluoro-phenyl)-quinoline,
3-(3,5-dichloro-benzenesulfonyl)-4-(3,4-difluoro-phenyl)-8-fluoro-quinoline,
7-chloro-3-(3-cyano-5-fluoro-benzenesulfonyl)-4-(3-fluoro-phenyl)-quinoline,
3-(3-cyano-benzenesulfonyl)-8-fluoro-4-(4-fluoro-phenyl)-quinoline,
3-(3,5-dichloro-benzenesulfonyl)-8-fluoro-4-(2-fluoro-phenyl)-quinoline,
3-(3-chloro-4-fluoro-benzenesulfonyl)-7-fluoro-4-(2-fluoro-phenyl)-quinoline,
4-(4-chloro-phenyl)-3-(3-cyano-benzenesulfonyl)-8-fluoro-quinoline,
4-(3-chloro-phenyl)-3-(3-cyano-S-fluoro-benzenesulfonyl)-quinoline,
7-chloro-3-(3-cyano-benzenesulfonyl)-4-(2-fluoro-phenyl)-quinoline,
3-(3-cyano-5-fluoro-benzenesulfonyl)-7-fluoro-4-(3-fluoro-phenyl)-quinoline,
3-(3-chloro-4-fluoro-benzenesulfonyl)-4-(3-chloro-phenyl)-8-fluoro-quinoline,
3-(3-cyano-benzenesulfonyl)-7-fluoro-4-(2-fluoro-phenyl)-quinoline,
3-(3-cyano-5-fluoro-benzenesulfonyl)-4-(3,4-difluoro-phenyl)-7-fluoro-quinoline,
3-(3-cyano-4-fluoro-benzenesulfonyl)-4-(3,4-dichloro-phenyl)-quinoline,
7-chloro-3-(3-chloro-4-fluoro-benzenesulfonyl)-4-(2-fluoro-phenyl)-quinoline,
7-chloro-3-(3-cyano-benzenesulfonyl)-4-(3-fluoro-phenyl)-quinoline,
4-(3-chloro-phenyl)-3-(3-cyano-4-fluoro-benzenesulfonyl)-7-fluoro-quinoline,
3-(3,4-difluoro-benzenesulfonyl)-4-(3,5-difluoro-phenyl)-8-fluoro-quinoline,
3-(3,4-difluoro-benzenesulfonyl)-4-(4-fluoro-phenyl)-quinoline,
3-(3,4-difluoro-benzenesulfonyl)-4-(3,4-difluoro-phenyl)-8-fluoro-quinoline,
3-(3,5-dichloro-benzenesulfonyl)-4-(3,4-difluoro-phenyl)-7-fluoro-quinoline,
3-(3,4-difluoro-benzenesulfonyl)-4-(3-fluoro-phenyl)-quinoline,
7-chloro-3-(3-cyano-benzenesulfonyl)-4-(4-fluoro-phenyl)-quinoline,
7-chloro-4-(3-chloro-phenyl)-3-(3-cyano-4-fluoro-benzenesulfonyl)-quinoline,
3-(3,4-difluoro-benzenesulfonyl)-8-fluoro-4-(2-fluoro-phenyl)-quinoline,
4-(3-chloro-phenyl)-3-(3-cyano-5-fluoro-benzenesulfonyl)-7-fluoro-quinoline,
3-(3-cyano-benzenesulfonyl)-4-(3,5-difluoro-phenyl)-7-fluoro-quinoline,
4-(3-chloro-phenyl)-3-(3-cyano-benzenesulfonyl)-8-fluoro-quinoline,
4-(4-chloro-phenyl)-3-(3,4-difluoro-benzenesulfonyl)-7-fluoro-quinoline,
7-chloro-3-(3,4-dichloro-benzenesulfonyl)-4-(3,4-difluoro-phenyl)-quinoline,
3-(3,5-dicyano-benzenesulfonyl)-7-fluoro-4-(4-fluoro-phenyl)-quinoline,
7-chloro-3-(3-chloro-5-fluoro-benzenesulfonyl)-4-(3-chloro-phenyl)-quinoline,
3-(3,4-difluoro-benzenesulfonyl)-4-(3,4-difluoro-phenyl)-7-fluoro-quinoline,
7-chloro-4-(3-chloro-phenyl)-3-(3,5-dichloro-benzenesulfonyl)-8-fluoro-quinoline,
7-chloro-4-(3-chloro-phenyl)-3-(3-cyano-benzenesulfonyl)-8-fluoro-quinoline,
7-chloro-4-(3-chloro-phenyl)-3-(3-cyano-benzenesulfonyl)-quinoline,
7-chloro-3-(3,5-dichloro-benzenesulfonyl)-4-(2-fluoro-phenyl)-quinoline,
3-(3,5-dicyano-benzenesulfonyl)-4-(3,4-difluoro-phenyl)-7-fluoro-quinoline,
3-(3,5-dichloro-benzenesulfonyl)-7-fluoro-4-(2-fluoro-phenyl)-quinoline,
3-(3-chloro-4-fluoro-benzenesulfonyl)-4-(3-chloro-phenyl)-7-fluoro-quinoline,
4-(3-chloro-phenyl)-3-(3,4-difluoro-benzenesulfonyl)-8-fluoro-quinoline,
4-(3,4-dichloro-phenyl)-3-(3,4-difluoro-benzenesulfonyl)-quinoline,
3-(3,4-difluoro-benzenesulfonyl)-7-fluoro-4-(2-fluoro-phenyl)-quinoline,
7-chloro-3-(3-chloro-4-fluoro-benzenesulfonyl)-4-(3,5-difluoro-phenyl)-quinoline,
7-chloro-4-(3-chloro-phenyl)-3-(3,5-dichloro-benzenesulfonyl)-quinoline,
7-amino-3-(3,4-difluoro-benzenesulfonyl)-4-(3-fluoro-phenyl)-quinoline,
4-(3-chloro-phenyl)-3-(3-cyano-benzenesulfonyl)-quinoline,
3-(3-cyano-5-fluoro-benzenesulfonyl)-4-(3,4-difluoro-phenyl)-quinoline,
3-(3-cyano-S-fluoro-benzenesulfonyl)-7-fluoro-4-(4-fluoro-phenyl)-quinoline,
4-(4-chloro-phenyl)-3-(3-cyano-5-fluoro-benzenesulfonyl)-7-fluoro-quinoline,
7-chloro-3-(3-chloro-5-fluoro-benzenesulfonyl)-4-(4-chloro-phenyl)-quinoline,
7-chloro-3-(3-chloro-5-fluoro-benzenesulfonyl)-4-(4-fluoro-phenyl)-quinoline,
3-(3-chloro-5-fluoro-benzenesulfonyl)-4-(4-fluoro-phenyl)-quinoline,
3-(3-chloro-5-fluoro-benzenesulfonyl)-4-(4-chloro-phenyl)-quinoline,
3-(3-chloro-4-fluoro-benzenesulfonyl)-4-(4-chloro-phenyl)-quinoline,
3-(3-chloro-4-fluoro-benzenesulfonyl)-4-(4-chloro-phenyl)-7-fluoro-quinoline,
3-(3-chloro-4-fluoro-benzenesulfonyl)-7-fluoro-4-(4-fluoro-phenyl)-quinoline,
7-chloro-3-(3-chloro-4-fluoro-benzenesulfonyl)-4-(4-fluoro-phenyl)-quinoline,
7-chloro-3-(3-chloro-4-fluoro-benzenesulfonyl)-4-(4-chloro-phenyl)-quinoline,
7-amino-3-(3-chloro-5-fluoro-benzenesulfonyl)-4-(4-fluoro-phenyl)-quinoline,
7-amino-3-(3-chloro-5-fluoro-benzenesulfonyl)-4-(3-fluoro-phenyl)-quinoline.
and/or salts and/or hydrates and/or solvates thereof.

3. A process for the preparation of a compound of formula (I): 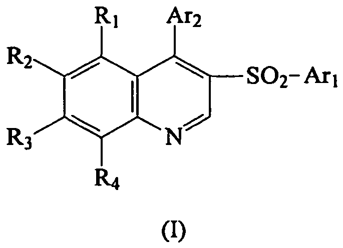 wherein Ar₁, Ar₂, R₁, R₂, R₃ and R₄ are as defined in claim 1, and/or salts and/or hydrates and/or solvates thereof,
a,
al. by reacting a compound of formula (II): 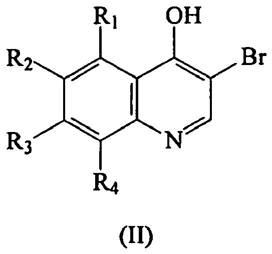 wherein R₁, R₂, R₃ and R₄ are as defined above for a compound of formula (I), with a compound of formula (III):
Ar₁-S⁻ M⁺ (III)
wherein Ar₁ is as defined above for compounds of formula (I), M is selected from alkali metals or alkaline-earth metals, to give a compound of formula (IV): 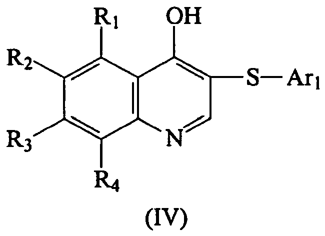 wherein Ar₁, R₁, R₂, R₃ and R₄ and are as defined above for compounds of formula (I), thereafter oxidizing a compound of formula (IV) to obtain a compound of formula (V): 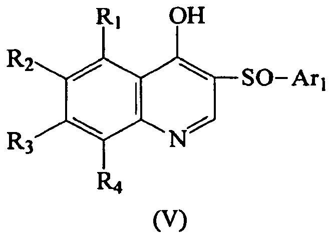 wherein Ar₁, R₁, R₂, R₃ and R₄ are as defined above for compounds of formula (I), thereafter oxidizing a compound of formula (V) to obtain a compound of formula (VI): 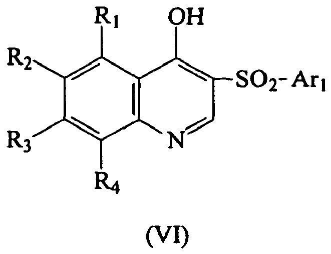 wherein Ar₁, R₁, R₂, R₃ and R₄ are as defined above for compounds of formula (I), or
a2.
by reacting a compound of formula (IX):
Ar₁-SO₂Na (IX)
wherein Ar₁ is as defined above for a compound of formula (I), with α-halogen-acetic acid esters of formula (X):
Hlg-CH₂-COOR₅ (X)
wherein Hlg is halogen and R₅ is an ethyl or methyl group, to obtain a compound of formula (XI):
Ar₁-SO₂-CH₂-COOR₅ (XI)
wherein Ar₁ is as defined above for a compound of formula (I) and R₅ is as defined above for compounds of formula (X), thereafter reacting a compound of formula (XI) with a trialkyl orthoformate of formula (XII):
CH(OR₆)₃ (XII)
wherein R₆ is an ethyl or methyl group, to obtain a compound of formula (XIII): 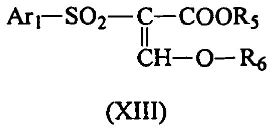 wherein Ar₁ is as defined above for a compound of formula (I), R₅ is as defined above for compounds of formula (X) and R₆ is as defined above for compounds of formula (XII), thereafter reacting a compound of formula (XIII) with an aniline derivative of formula (XIV): 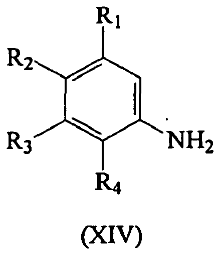 wherein R₁, R₂, R₃ and R₄ are as defined above for a compound of formula (I), to obtain a compound of formula (VI): 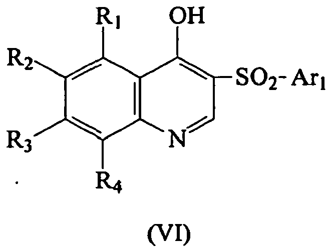 wherein Ar₁, R₁, R₂, R₃ and R₄ are as defined above for a compound of formula (I),
thereafter converting a compound of formula (VI) to a compound of formula (VII): 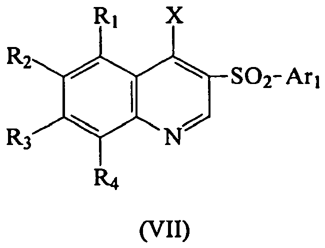 wherein Ar₁, R₁, R₂, R₃ and R₄ are as defined above for compounds of formula (I), X is selected from chloro, bromo, benzenesulfonyloxy, 4-fluoro-benzenesulfonyloxy, 4-methyl-benzenesulfonyloxy, methanesulfonyloxy or trifluoromethanesulfonyloxy groups,
thereafter reacting the obtained compound of formula (VII) with a boronic acid derivative of formula (VIII):
Ar₂-B(OH)₂ (VIII)
wherein Ar₂ is as defined above for a compound of formula (I), in the presence of base and catalyst in a solvent,
and optionally thereafter forming salts and/or hydrates and/or solvates of compounds of formula (I), or
b, interconverting one compound of formula (I), wherein Ar₁, Ar₂, R₁, R₂, R₃ and R₄ are as defined above for the formula (I), to a different compound of formula (I), wherein Ar₁, Ar₂, R₁, R₂, R₃ and R₄ are as defined as described above for the formula (I),
and optionally thereafter forming salts and/or hydrates and/or solvates of compounds of formula (I).

4. A pharmaceutical formulation comprising a therapeutically effective amount of a compound of formula (I) as defined in claim 1, and/or physiologically acceptable salts and/or hydrates and/or solvates thereof in association with one or more physiologically acceptable diluents, excipients and/or inert carriers.

5. A pharmaceutical formulation as defined in claim 4 for use in a method of preventing and/or treating a disorder selected from psychiatric disorders, neurological disorders, chronic and acute pain, neuromuscular dysfunctions of the lower urinary tract, gastrointestinal reflux disease, irritable bowel syndrome, substance abuse and withdrawal.

6. A compound of formula (I) as defined in claim 1 or 2 and/or physiologically acceptable salts and/or hydrates and/or solvates thereof for use in a method of treating and/or preventing a disease selected from psychiatric disorders, neurological disorders, chronic and acute pain, neuromuscular dysfunctions of the lower urinary tract, gastrointestinal reflux disease, irritable bowel syndrome, substance abuse and withdrawal.

## Patentansprüche

1. Verbindung der Formel (I): 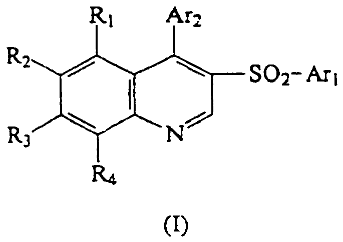 worin Ar₁ eine Phenyl- oder Thienylgruppe, optional substituiert mit einem oder mehreren Substituenten ausgewählt aus Wasserstoff, Fluor, Chlor, Cyano, Methyl, Methoxy, darstellt;
Ar₂ Phenyl, substituiert mit einem oder mehreren Substituenten ausgewählt aus Fluor, Chlor, Cyano, Methyl, Methoxy, darstellt;
R₁, R₂, R₃ und R₄ unabhängig voneinander einen Substituenten darstellen, der aus Wasserstoff, Fluor, Chlor, Cyano, Methyl, Methoxy, Hydroxy, Trifluormethyl, Amino, Methylamino, Dimethylamino, Aminomethyl, Methylaminomethyl, Dimethylaminomethyl ausgewählt ist, und/oder Salzen und/oder Hydraten und/oder Solvaten davon.

2. Verbindung gemäß Anspruch 1 ausgewählt aus der Gruppe bestehend aus
4-(4-Chlorphenyl)-3-(4-methylbenzolsulfonyl)-chinolin,
7-Chlor-3-(4-chlorbenzolsulfonyl)-4-(4-fluorphenyl)-chinolin,
8-Chlor-4-(3-chlorphenyl)-3-(3,4-dichlorbenzolsulfonyl)-chinolin,
7-Fluor-3-(4-fluorbenzolsulfonyl)-4-(3-fluorphenyl)-chinolin,
4-(4-Chlorphenyl)-7-fluor-3-(4-methoxybenzolsulfonyl)-chinolin,
7-Fluor-3-(4-methoxybenzolsulfonyl)-4-(4-methoxyphenyl)-chinolin,
7-Fluor-3-(3-fluorbenzolsulfonyl)-4-(3-fluorphenyl)-chinolin,
7-Fluor-3-(3-fluorbenzolsulfonyl)-4-(4-fluorphenyl)-chinolin,
4-(3-Chlorphenyl)-3-(3,4-dimethylbenzolsulfonyl)-7-fluorchinolin,
3-(3,4-Dimethylbenzolsulfonyl)-7-fluor-4-(3-fluorphenyl)-chinolin,
3-(3,4-Dimethylbenzolsulfonyl)-7-fluor-4-(3-methoxyphenyl)-chinolin,
4-(3-Chlorphenyl)-8-fluor-3-(4-fluorbenzolsulfonyl)-chinolin,
4-(4-Chlorphenyl)-8-fluor-3-(4-fluorbenzolsulfonyl)-chinolin,
8-Fluor-3-(4-fluorbenzolsulfonyl)-4-(3-fluorphenyl)-chinolin,
8-Fluor-3-(4-fluorbenzolsulfonyl)-4-(3-methoxyphenyl)-chinolin,
4-(4-Chlorphenyl)-6-fluor-3-(4-methoxybenzolsulfonyl)-chinolin,
4-(4-Chlorphenyl)-3-(3,4-dimethylbenzolsulfonyl)-6-fluorchinolin,
4-(4-Chlorphenyl)-3-(3,5-difluorbenzolsulfonyl)-7-fluorchinolin,
3-(3,5-Difluorbenzolsulfonyl)-7-fluor-4-(4-fluorphenyl)-chinolin,
4-(4-Chlorphenyl)-3-(3-cyanobenzolsulfonyl)-7-fluorchinolin,
3-(3-Cyanobenzolsulfonyl)-7-fluor-4-(3-fluorphenyl)-chinolin,
3-(3-Cyanobenzolsulfonyl)-7-fluor-4-(4-fluorphenyl)-chinolin,
7-Fluor-3-(4-fluorbenzolsulfonyl)-4-(4-fluorphenyl)-chinolin,
4-(4-Chlorphenyl)-7-fluor-3-(3-fluorbenzolsulfonyl)-chinolin,
4-(3-Chlorphenyl)-7-fluor-3-(3-methoxybenzolsulfonyl)-chinolin,
7-Fluor-4-(4-fluorbenzolsulfonyl)-3-(3-methoxybenzolsulfonyl)-chinolin,
4-(4-Chlorphenyl)-3-(3,4-dimethylbenzolsulfonyl)-7-fluorchinolin,
3-(3,4-Dimethylbenzolsulfonyl)-7-fluor-4-(4-fluorphenyl)-chinolin,
4-(3-Chlorphenyl)-3-(3-chlor-4-methoxybenzolsulfonyl)-7-fluorchinolin,
3-(3-Chlor-4-methoxybenzolsulfonyl)-7-fluor-4-(3-fluorphenyl)-chinolin,
3-(3-Chlor-4-methoxybenzolsulfonyl)-7-fluor-4-(4-fluorphenyl)-chinolin,
3-(3,5-Dichlorbenzolsulfonyl)-8-fluor-4-(4-fluorphenyl)-chinolin,
3-(3,5-Difluorbenzolsulfonyl)-8-fluor-4-(3-fluorphenyl)-chinolin,
8-Fluor-3-(4-fluorbenzolsulfonyl)-4-(4-methoxyphenyl)-chinolin,
4-(3-Chlorphenyl)-8-fluor-3-(4-methoxybenzolsulfonyl)-chinolin,
4-(4-Chlorphenyl)-8-fluor-3-(4-methoxybenzolsulfonyl)-chinolin,
8-Fluor-4-(4-fluorphenyl)-3-(4-methoxybenzolsulfonyl)-chinolin,
8-Fluor-3-(4-methoxybenzolsulfonyl)-4-(4-methoxyphenyl)-chinolin,
8-Fluor-3-(4-methoxybenzolsulfonyl)-4-(3-methoxyphenyl)-chinolin,
4-(3-Chlorphenyl)-8-fluor-3-(3-fluorbenzolsulfonyl)-chinolin,
4-(4-Chlorphenyl)-8-fluor-3-(3-fluorbenzolsulfonyl)-chinolin,
8-Fluor-3-(3-fluorbenzolsulfonyl)-4-(3-fluorphenyl)-chinolin,
8-Fluor-3-(3-fluorbenzolsulfonyl)-4-(4-methoxyphenyl)-chinolin,
8-Fluor-3-(3-fluorbenzolsulfonyl)-4-(3-methoxyphenyl)-chinolin,
8-Fluor-3-(3-fluorbenzolsulfonyl)-4-(4-fluorphenyl)-chinolin,
3-(3-Cyanobenzolsulfonyl)-6-fluor-4-(3-fluorphenyl)-chinolin,
3-(3-Cyanobenzolsulfonyl)-6-fluor-4-(3-methoxyphenyl)-chinolin,
3-(3-Cyanobenzolsulfonyl)-6-fluor-4-(4-fluorphenyl)-chinolin,
4-(3-Chlorphenyl)-6-fluor-3-(3-methoxybenzolsulfonyl)-chinolin,
6-Fluor-4-(4-fluorphenyl)-3-(3-methoxybenzolsulfonyl)-chinolin,
3-(3-Chlor-4-methoxybenzolsulfonyl)-6-fluor-4-(4-methoxyphenyl)-chinolin,
3-(3-Chlor-4-methoxybenzolsulfonyl)-6-fluor-4-(3-methoxyphenyl)-chinolin,
4-(4-Chlorphenyl)-7-fluor-3-(4-fluorbenzolsulfonyl)-chinolin,
3-(3,4-Dimethylbenzolsulfonyl)-7-fluor-4-(4-methoxyphenyl)-chinolin,
3-(3-Chlor-4-methoxybenzolsulfonyl)-4-(4-chlorphenyl)-6-fluor-chinolin,
3-(3-Chlor-4-methoxybenzolsulfonyl)-6-fluor-4-(3-fluorphenyl)-chinolin,
3-(3,5-Dichlorbenzolsulfonyl)-7-fluor-4-(4-methoxyphenyl)-chinolin,
3-(3,5-Dichlorbenzolsulfonyl)-7-fluor-4-(3-methoxyphenyl)-chinolin,
3-(3,5-Difluorbenzolsulfonyl)-7-fluor-4-(4-fluorphenyl)-chinolin,
4-(3-Chlorphenyl)-7-fluor-3-(3-fluor-4-methylbenzolsulfonyl)-chinolin,
7-Fluor-3-(3-fluor-4-methylbenzolsulfonyl)-4-(3-fluorphenyl)-chinolin,
7-Fluor-3-(3-fluor-4-methylbenzolsulfonyl)-4-(4-fluorphenyl)-chinolin,
3-(3,5-Difluorbenzolsulfonyl)-7-fluor-4-(3-fluorphenyl)-chinolin,
3-(3,5-Difluorbenzolsulfonyl)-7-fluor-4-(4-methoxyphenyl)-chinolin,
3-(3,5-Difluorbenzolsulfonyl)-7-fluor-4-(3-methoxyphenyl)-chinolin,
4-(3-Chlorphenyl)-3-(3-cyanobenzolsulfonyl)-7-fluorchinolin,
3-(3-Cyanobenzolsulfonyl)-7-fluor-4-(4-methoxyphenyl)-chinolin,
3-(3-Cyanobenzolsulfonyl)-7-fluor-4-(3-methoxyphenyl)-chinolin,
3-(3-Chlor-4-methylbenzolsulfonyl)-7-fluor-4-(3-fluorphenyl)-chinolin,
3-(3,4-Difluorbenzolsulfonyl)-7-fluor-4-(3-fluorphenyl)-chinolin,
3-(3,4-Difluorbenzolsulfonyl)-7-fluor-4-(4-methoxyphenyl)-chinolin,
3-(3,4-Difluorbenzolsulfonyl)-7-fluor-4-(3-methoxyphenyl)-chinolin,
3-(3,4-Difluorbenzolsulfonyl)-7-fluor-4-(4-fluorphenyl)-chinolin,
3-(3-Chlor-4-fluorbenzolsulfonyl)-4-(4-chlorphenyl)-7-fluorchinolin,
3-(3-Chlor-4-fluorbenzolsulfonyl)-7-fluor-4-(3-fluorphenyl)-chinolin,
3-(3-Chlor-4-fluorbenzolsulfonyl)-7-fluor-4-(4-methoxyphenyl)-chinolin,
3-(3-Chlor-4-fluorbenzolsulfonyl)-7-fluor-4-(4-fluorphenyl)-chinolin,
3-(3,5-Dichlorbenzolsulfonyl)-8-fluor-4-(3-fluorphenyl)-chinolin,
3-(3,5-Dichlorbenzolsulfonyl)-8-fluor-4-(4-methoxyphenyl)-chinolin,
3-(3,5-Dichlorbenzolsulfonyl)-8-fluor-4-(3-methoxyphenyl)-chinolin,
4-(3-Chlorphenyl)-8-fluor-3-(3-fluor-4-methylbenzolsulfonyl)-chinolin,
4-(4-Chlorphenyl)-8-fluor-3-(3-fluor-4-methylbenzolsulfonyl)-chinolin,
8-Fluor-3-(3-fluor-4-methylbenzolsulfonyl)-4-(3-fluorphenyl)-chinolin,
8-Fluor-3-(3-fluor-4-methylbenzolsulfonyl)-4-(4-methoxyphenyl)-chinolin,
8-Fluor-3-(3-fluor-4-methylbenzolsulfonyl)-4-(3-methoxyphenyl)-chinolin,
8-Fluor-3-(3-fluor-4-methylbenzolsulfonyl)-4-(4-fluorphenyl)-chinolin,
4-(3-Chlorphenyl)-3-(3,5-difluorbenzolsulfonyl)-8-fluorchinolin,
4-(4-Chlorphenyl)-3-(3,5-difluorbenzolsulfonyl)-8-fluorchinolin,
3-(3,5-Difluorbenzolsulfonyl)-8-fluor-4-(4-methoxyphenyl)-chinolin,
3-(3,5-Difluorbenzolsulfonyl)-8-fluor-4-(3-methoxyphenyl)-chinolin,
3-(3,5-Difluorbenzolsulfonyl)-8-fluor-4-(4-fluorphenyl)-chinolin,
4-(4-Chlorphenyl)-3-(3,4-difluorbenzolsulfonyl)-8-fluorchinolin,
3-(3,4-Difluorbenzolsulfonyl)-8-fluor-4-(3-fluorphenyl)-chinolin,
3-(3,4-Difluorbenzolsulfonyl)-8-fluor-4-(4-methoxyphenyl)-chinolin,
3-(3,4-Difluorbenzolsulfonyl)-8-fluor-4-(4-fluorphenyl)-chinolin,
3-(3,5-Dichlorbenzolsulfonyl)-6-fluor-4-(3-fluorphenyl)-chinolin,
3-(3,5-Dichlorbenzolsulfonyl)-6-fluor-4-(4-methoxyphenyl)-chinolin,
3-(3,5-Dichlorbenzolsulfonyl)-6-fluor-4-(3-methoxyphenyl)-chinolin,
4-(3-Chlorphenyl)-6-fluor-3-(3-fluor-4-methylbenzolsulfonyl)-chinolin,
6-Fluor-3-(3-fluor-4-methylbenzolsulfonyl)-4-(3-fluorphenyl)-chinolin,
6-Fluor-3-(3-fluor-4-methylbenzolsulfonyl)-4-(4-methoxyphenyl)-chinolin,
6-Fluor-3-(3-fluor-4-methylbenzolsulfonyl)-4-(3-methoxyphenyl)-chinolin,
6-Fluor-3-(3-fluor-4-methylbenzolsulfonyl)-4-(4-fluorphenyl)-chinolin,
4-(3-Chlorphenyl)-3-(3-cyanobenzolsulfonyl)-6-fluorchinolin,
4-(4-Chlorphenyl)-3-(3-cyanobenzolsulfonyl)-6-fluorchinolin,
3-(3-Cyanobenzolsulfonyl)-6-fluor-4-(4-methoxyphenyl)-chinolin,
3-(3-Chlor-4-methylbenzolsulfonyl)-4-(3-chlorphenyl)-6-fluorchinolin,
3-(3-Chlor-4-methylbenzolsulfonyl)-4-(4-chlorphenyl)-6-fluorchinolin,
3-(3-Chlor-4-methylbenzolsulfonyl)-6-fluor-4-(3-fluorphenyl)-chinolin,
3-(3-Chlor-4-methylbenzolsulfonyl)-6-fluor-4-(3-methoxyphenyl)-chinolin,
3-(3-Chlor-4-methylbenzolsulfonyl)-6-fluor-4-(4-fluorphenyl)-chinolin,
4-(4-Chlorphenyl)-6-fluor-3-(3-methoxybenzolsulfonyl)-chinolin,
6-Fluor-3-(3-methoxybenzolsulfonyl)-4-(3-methoxyphenyl)-chinolin,
3-(3,4-Dimethylbenzolsulfonyl)-6-fluor-4-(3-fluorphenyl)-chinolin,
3-(3,4-Dimethylbenzolsulfonyl)-6-fluor-4-(4-methoxyphenyl)-chinolin,
3-(3,4-Dimethylbenzolsulfonyl)-6-fluor-4-(4-fluorphenyl)-chinolin,
3-(3,4-Difluorbenzolsulfonyl)-6-fluor-4-(3-fluorphenyl)-chinolin,
3-(3,4-Difluorbenzolsulfonyl)-6-fluor-4-(4-fluorphenyl)-chinolin,
3-(3-Chlor-4-fluorbenzolsulfonyl)-4-(4-chlorphenyl)-6-fluorchinolin,
3-(3-Chlor-4-fluorbenzolsulfonyl)-6-fluor-4-(3-methoxyphenyl)-chinolin,
3-(3-Chlor-4-fluorbenzolsulfonyl)-6-fluor-4-(4-fluorphenyl)-chinolin,
4-(3-Chlorphenyl)-3-(3,5-dichlorbenzolsulfonyl)-7-fluorchinolin,
4-(4-Chlorphenyl)-3-(3,5-dichlorbenzolsulfonyl)-7-fluorchinolin,
4-(3-Chlorphenyl)-3-(3,5-dichlorbenzolsulfonyl)-8-fluorchinolin,
4-(4-Chlorphenyl)-3-(3,5-dichlorbenzolsulfonyl)-8-fluorchinolin,
4-(3-Chlorphenyl)-3-(3,5-dichlorbenzolsulfonyl)-6-fluorchinolin,
4-(4-Chlorphenyl)-3-(3,5-dichlorbenzolsulfonyl)-6-fluorchinolin,
6-Fluor-3-(4-methoxybenzolsulfonyl)-4-(4-methoxyphenyl)-chinolin,
7-Chlor-4-(4-chlorphenyl)-3-(3,5-difluorbenzolsulfonyl)-chinolin,
7-Chlor-4-(4-chlorphenyl)-3-(3,4-difluorbenzolsulfonyl)-chinolin,
7-Chlor-4-(4-chlorphenyl)-3-(3-cyanobenzolsulfonyl)-chinolin,
7-Chlor-3-(3,5-chlorbenzolsulfonyl)-4-(4-fluorphenyl)-chinolin,
7-Chlor-3-(4-fluorbenzolsulfonyl)-4-(4-fluorphenyl)-chinolin,
7-Chlor-3-(3-fluorbenzolsulfonyl)-4-(4-fluorphenyl)-chinolin,
7-Chlor-3-(3,4-difluorbenzolsulfonyl)-4-(4-fluorphenyl)-chinolin,
7-Chlor-3-(3-chlor-4-fluorbenzolsulfonyl)-4-(4-fluorphenyl)-chinolin und
7-Chlor-3-(3-cyano-5-fluorbenzolsulfonyl)-4-(4-fluorphenyl)-chinolin,
6-Chlor-3-(3-chlor-4-fluorbenzolsulfonyl)-4-(4-chlorphenyl)-7-fluorchinolin,
6-Chlor-3-(3-chlor-4-fluorbenzolsulfonyl)-7-fluor-4-(4-fluorphenyl)-chinolin,
3-(3-Chlor-4-fluorbenzolsulfonyl)-7-cyano-4-(2-fluorphenyl)-chinolin,
7-Chlor-3-(3,4-difluorbenzolsulfonyl)-8-fluor-4-(3-fluorphenyl)-chinolin,
7-Chlor-3-(3-chlor-4-fluorbenzolsulfonyl)-4-(4-fluorphenyl)-8-fluorchinolin,
7-Chlor-3-(3-chlor-4-fluorbenzolsulfonyl)-8-fluor-4-(3-fluorphenyl)-chinolin,
7-Chlor-4-(4-chlorphenyl)-3-(3,4-difluorbenzolsulfonyl)-8-fluorchinolin,
7-Chlor-3-(3-chlor-4-fluorbenzolsulfonyl)-8-fluor-4-(4-fluorphenyl)-chinolin,
3-(3-Cyano-4-fluorbenzolsulfonyl)-4-(3-fluorphenyl)-chinolin,
3-(3-Cyano-5-fluorbenzolsulfonyl)-4-(3-fluorphenyl)-chinolin,
3-(3-Chlor-4-fluorbenzolsulfonyl)-4-(4-chlorphenyl)-8-fluorchinolin,
3-(3-Chlor-4-fluorbenzolsulfonyl)-8-fluor-4-(2-fluorphenyl)-chinolin,
3-(3-Chlor-4-fluorbenzolsulfonyl)-8-fluor-4-(3-fluorphenyl)-chinolin,
3-(3-Cyano-4-fluorbenzolsulfonyl)-4-(4-fluorphenyl)-chinolin,
3-(3-Chlor-4-fluorbenzolsulfonyl)-8-fluor-4-(4-fluorphenyl)-chinolin,
3-(3-Cyanobenzolsulfonyl)-8-fluor-4-(2-fluorphenyl)-chinolin,
3-(3-Cyanobenzolsulfonyl)-4-(3-fluorphenyl)-chinolin,
3-(3-Cyanobenzolsulfonyl)-8-fluor-4-(3-fluorphenyl)-chinolin,
3-(3-Cyano-4-fluorbenzolsulfonyl)-7-fluor-4-(3-fluorphenyl)-chinolin,
4-(3-Chlorphenyl)-3-(3-cyano-4-fluorbenzolsulfonyl)-chinolin,
3-(3-Cyano-5-fluorbenzolsulfonyl)-7-fluor-4-(4-fluorphenyl)-chinolin,
7-Chlor-3-(3-cyano-4-fluorbenzolsulfonyl)-4-(3-fluorphenyl)-chinolin,
3-(3,5-Dichlorbenzolsulfonyl)-4-(3,4-difluorphenyl)-8-fluorchinolin,
7-Chlor-3-(3-cyano-5-fluorbenzolsulfonyl)-4-(3-fluorphenyl)-chinolin,
3-(3-Cyanobenzolsulfonyl)-8-fluor-4-(4-fluorphenyl)-chinolin,
3-(3,5-Dichlorbenzolsulfonyl)-8-fluor-4-(2-fluorphenyl)-chinolin,
3-(3-Chlor-4-fluorbenzolsulfonyl)-7-fluor-4-(2-fluorphenyl)-chinolin,
4-(4-Chlorphenyl)-3-(3-cyanobenzolsulfonyl)-8-fluorchinolin,
4-(3-Chlorphenyl)-3-(3-cyano-5-fluorbenzolsulfonyl)-chinolin,
7-Chlor-3-(3-cyanobenzolsulfonyl)-4-(2-fluorphenyl)-chinolin,
3-(3-Cyano-5-fluorbenzolsulfonyl)-7-fluor-4-(3-fluorphenyl)-chinolin,
3-(3-Chlor-4-fluorbenzolsulfonyl)-4-(3-chlorphenyl)-8-fluorchinolin,
3-(3-Cyanobenzolsulfonyl)-7-fluor-4-(2-fluorphenyl)-chinolin,
3-(3-Cyano-5-fluorbenzolsulfonyl)-4-(3,4-difluorphenyl)-7-fluorchinolin,
3-(3-Cyano-4-fluorbenzolsulfonyl)-4-(3,4-dichlorphenyl)-chinolin,
7-Chlor-3-(3-chlor-4-fluorbenzolsulfonyl)-4-(2-fluorphenyl)-chinolin,
7-Chlor-3-(3-cyanobenzolsulfonyl)-4-(3-fluorphenyl)-chinolin,
4-(3-Chlorphenyl)-3-(3-cyano-4-fluorbenzolsulfonyl)-7-fluorchinolin,
3-(3,4-Difluorbenzolsulfonyl)-4-(3,5-difluorphenyl)-8-fluorchinolin,
3-(3,4-Difluorbenzolsulfonyl)-4-(4-fluorphenyl)-chinolin,
3-(3,4-Difluorbenzolsulfonyl)-4-(3,4-difluorphenyl)-8-fluorchinolin,
3-(3,5-Difluorbenzolsulfonyl)-4-(3,4-difluorphenyl)-7-fluorchinolin,
3-(3,4-Difluorbenzolsulfonyl)-4-(3-fluorphenyl)-chinolin,
7-Chlor-3-(3-cyanobenzolsulfonyl)-4-(4-fluorphenyl)-chinolin,
7-Chlor-4-(3-chlorphenyl)-3-(3-cyano-4-fluorbenzolsulfonyl)-chinolin,
3-(3,4-Difluorbenzolsulfonyl)-8-fluor-4-(2-fluorphenyl)-chinolin,
4-(3-Chlorphenyl)-3-(3-cyano-5-fluorbenzolsulfonyl)-7-fluorchinolin,
3-(3-Cyanobenzolsulfonyl)-4-(3,5-difluorphenyl)-7-fluorchinolin,
4-(3-Chlorphenyl)-3-(3-cyanobenzolsulfonyl)-8-fluorchinolin,
4-(4-Chlorphenyl)-3-(3,4-difluorbenzolsulfonyl)-7-fluorchinolin,
7-Chlor-3-(3,4-dichlorbenzolsulfonyl)-4-(3,4-difluorphenyl)-chinolin,
3-(3,5-Dicyanobenzolsulfonyl)-7-fluor-4-(4-fluorphenyl)-chinolin,
7-Chlor-3-(3-chlor-5-fluorbenzolsulfonyl)-4-(3-chlorphenyl)-chinolin,
3-(3,4-Difluorbenzolsulfonyl)-4-(3,4-difluorphenyl)-7-fluorchinolin,
7-Chlor-4-(3-chlorphenyl)-3-(3,5-dichlorbenzolsulfonyl)-8-fluorchinolin,
7-Chlor-4-(3-chlorphenyl)-3-(3-cyanobenzolsulfonyl)-8-fluorchinolin,
7-Chlor-4-(3-chlorphenyl)-3-(3-cyanobenzolsulfonyl)-chinolin,
7-Chlor-3-(3,5-dichlorbenzolsulfonyl)-4-(2-fluorphenyl)-chinolin,
3-(3,5-Dicyanobenzolsulfonyl)-4-(3,4-difluorphenyl)-7-fluorchinolin,
3-(3,5-Dichlorbenzolsulfonyl)-7-fluor-4-(2-fluorphenyl)-chinolin,
3-(3-Chlor-4-fluor-benzolsulfonyl)-4-(3-chlorphenyl)-7-fluorchinolin,
4-(3-Chlorphenyl)-3-(3,4-difluorbenzolsulfonyl)-8-fluorchinolin,
4-(3,4-Dichlorphenyl)-3-(3,4-difluorbenzolsulfonyl)-chinolin,
3-(3,4-Difluorbenzolsulfonyl)-7-fluor-4-(2-fluorphenyl)-chinolin,
7-Chlor-3-(3-chlor-4-fluor-benzolsulfonyl)-4-(3,5-difluorphenyl)-chinolin,
7-Chlor-4-(3-chlorphenyl)-3-(3,5-dichlorbenzolsulfonyl)-chinolin,
7-Amino-3-(3,4-difluorbenzolsulfonyl)-4-(3-fluorphenyl)-chinolin,
4-(3-Chlorphenyl)-3-(3-cyanobenzolsulfonyl)-chinolin,
3-(3-Cyano-5-fluorbenzolsulfonyl)-4-(3,4-difluorphenyl)-chinolin,
3-(3-Cyano-5-fluorbenzolsulfonyl)-7-fluor-4-(4-fluorphenyl)-chinolin,
4-(4-Chlorphenyl)-3-(3-cyano-5-fluorbenzolsulfonyl)-7-fluorchinolin,
7-Chlor-3-(3-chlor-5-fluorbenzolsulfonyl)-4-(4-chlorphenyl)-chinolin,
7-Chlor-3-(3-chlor-5-fluorbenzolsulfonyl)-4-(4-fluorphenyl)-chinolin,
3-(3-Chlor-5-fluorbenzolsulfonyl)-4-(4-fluorphenyl)-chinolin,
3-(3-Chlor-5-fluorbenzolsulfonyl)-4-(4-chlorphenyl)-chinolin,
3-(3-Chlor-4-fluorbenzolsulfonyl)-4-(4-chlorphenyl)-chinolin,
3-(3-Chlor-4-fluorbenzolsulfonyl)-4-(4-chlorphenyl)-7-fluorchinolin,
3-(3-Chlor-4-fluorbenzolsulfonyl)-7-fluor-4-(4-fluorphenyl)-chinolin,
7-Chlor-3-(3-chlor-4-fluorbenzolsulfonyl)-4-(4-fluorphenyl)-chinolin,
7-Chlor-3-(3-chlor-4-fluorbenzolsulfonyl)-4-(4-chlorphenyl)-chinolin,
7-Amino-3-(3-chlor-5-fluorbenzolsulfonyl)-4-(4-fluorphenyl)-chinolin,
7-Amino-3-(3-chlor-5-fluorbenzolsulfonyl)-4-(3-fluorphenyl)-chinolin
und/oder Salze und/oder Hydrate und/oder Solvate davon.

3. Verfahren zur Herstellung einer Verbindung der Formel (I) : 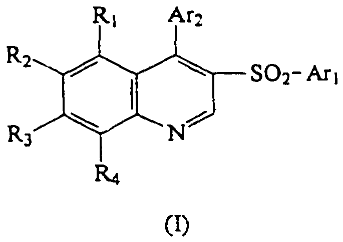 worin Ar₁, Ar₂, R₁, R₂, R₃ und R₄ wie in Anspruch 1 definiert sind, und/oder Salzen und/oder Hydraten und/oder Solvaten davon,
a,
a1. Reagieren einer Verbindung der Formel (II): 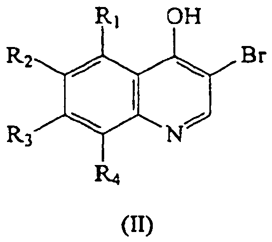 worin R₁, R₂, R₃ und R₄ wie oben definiert für eine Verbindung der Formel (I) sind, mit einer Verbindung der Formel (III):
Ar₁-S⁻M⁺ (III)
worin Ar₁ wie oben definiert für Verbindungen der Formel (I) ist, M aus Alkalimetallen oder Erdalkalimetallen ausgewählt ist, um eine Verbindung der Formel (IV) zu ergeben: 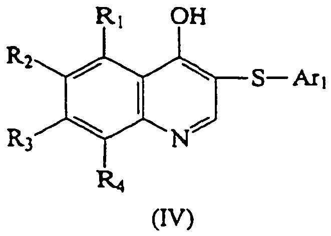 worin Ar₁, R₁, R₂, R₃ und R₄ wie oben definiert für eine Verbindung der Formel (I) sind, danach Oxidieren einer Verbindung der Formel (IV), um eine Verbindung der Formel (V) zu erhalten: 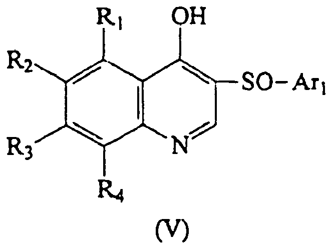 worin Ar₁, R₁, R₂, R₃ und R₄ wie oben definiert für Verbindungen der Formel (I) sind, danach Oxidieren einer Verbindung der Formel (V), um eine Verbindung der Formel (VI) zu erhalten: 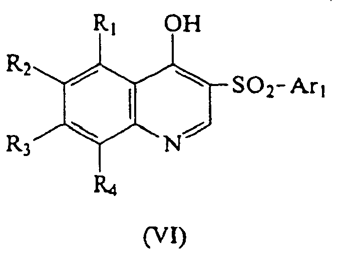 worin Ar₁, R₁, R₂, R₃ und R₄ wie oben definiert für Verbindungen der Formel (I) sind, oder
a2. Reagieren einer Verbindung der Formel (IX):
Ar₁-SO₂Na (IX)
worin Ar₁ wie oben definiert für eine Verbindung der Formel (I) ist, mit α-Halogen-Essigsäureestern der Formel (X) :
Hlg-CH₂-COOR₅ (X)
worin Hlg ein Halogen ist und R₅ eine Ethyl- oder Methylgruppe, um eine Verbindung der Formel (XI) zu erhalten:
Ar₁-SO₂-CH₂-COOR₅ (XI)
worin Ar₁ wie oben definiert für eine Verbindung der Formel (I) ist und R₅ wie oben definiert für Verbindungen der Formel (X) ist, danach Reagieren einer Verbindung der Formel (XI) mit einem Trialkylorthoformiat der Formel (XII):
CH(OR₆)₃ (XII)
worin R₆ eine Ethyl- oder Methylgruppe ist, um eine Verbindung der Formel (XIII) zu erhalten: 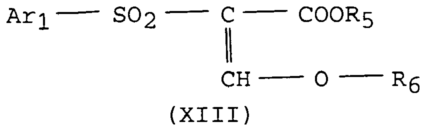 worin Ar₁ wie oben definiert für eine Verbindung der Formel (I) ist, R₅ wie oben definiert für Verbindungen der Formel (X) ist und R₆ wie oben definiert für Verbindungen der Formel (XII) ist, danach Reagieren einer Verbindung der Formel (XIII) mit einem Anilinderivat der Formel (XIV): 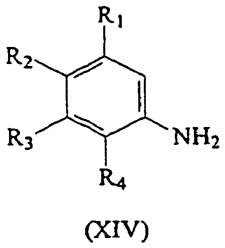 worin R₁, R₂, R₃ und R₄ wie oben definiert für eine Verbindung der Formel (I) sind, um eine Verbindung der Formel (VI) zu erhalten: 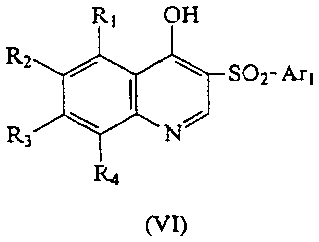 worin Ar₁, R₁, R₂, R₃ und R₄ wie oben definiert für eine Verbindung der Formel (I) sind,
danach Umsetzen einer Verbindung der Formel (VI) zu einer Verbindung der Formel (VII): 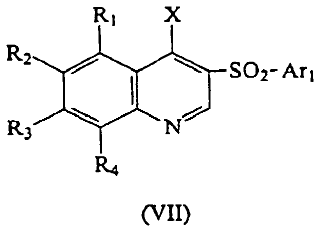 worin Ar₁, R₁, R₂, R₃ und R₄ wie oben definiert für Verbindungen der Formel (I) sind, X ausgewählt ist aus Chlor, Brom, Benzolsulfonyloxy-, 4-Fluorbenzolsulfonyloxy-, 4-Methylbenzolsulfonyloxy-, Methansulfonyloxy- oder Trifluormethansulfonyloxygruppen,
danach Reagieren der erhaltenen Verbindung der Formel (VII) mit einem Borsäurederivat der Formel (VIII):
Ar₂-B(OH)₂ (VIII)
worin Ar₂ wie oben definiert für eine Verbindung der Formel (I) ist, in Gegenwart einer Base und eines Katalysators in einem Lösungsmittel,
und danach optional Bildung von Salzen und/oder Hydraten und/oder Solvaten der Verbindungen der Verbindungen der Formel (I) oder
b. Umsetzen einer Verbindung der Formel (I), worin Ar₁, Ar₂, R₁, R₂, R₃ und R₄ wie oben definiert für die Formel (I) sind, zu einer unterschiedlichen Verbindung der Formel (I), worin Ar₁, Ar₂, R₁, R₂, R₃ und R₄ wie oben definiert für die Formel (I) sind, und danach optional Bilden von Salzen und/oder Hydraten und/oder Solvaten der Verbindungen der Formel (I).

4. Pharmazeutische Formulierung, umfassend eine therapeutisch wirksame Menge einer Verbindung der Formel (I) wie definiert in Anspruch 1, und/oder physiologisch annehmbaren Salzen und/oder Hydraten und/oder Solvaten davon zusammen mit einem oder mehreren physiologisch annehmbaren Verdünnern, Hilfsstoffen und/oder inerten Trägern.

5. Pharmazeutische Formulierung wie definiert in Anspruch 4 für die Verwendung in einem Verfahren zur Vermeidung und/oder Behandlung einer Krankheit, die ausgewählt ist aus psychiatrischen Krankheiten, neurologischen Krankheiten, chronischem und akutem Schmerz, neuromuskulären Disfunktionen der unteren Harnwege, gastrointestinaler Rückflusskrankheit, Reizdarmsyndrom, Suchtmittelmissbrauch und Entzug.

6. Verbindung der Formel (I) wie definiert in Anspruch 1 oder 2 und/oder physiologisch annehmbaren Salzen und/oder Hydraten und/oder Solvaten davon für die Verwendung in einem Verfahren zur Behandlung und/oder Vermeidung einer Krankheit, die ausgewählt ist aus psychiatrischen Krankheiten, neurologischen Krankheiten, chronischem und akutem Schmerz, neuromuskulären Disfunktionen der unteren Harnwege, gastrointestinaler Rückflusskrankheit, Reizdarmsyndrom, Suchtmittelmissbrauch und Entzug.

## Revendications

1. Composé de formule (I) : 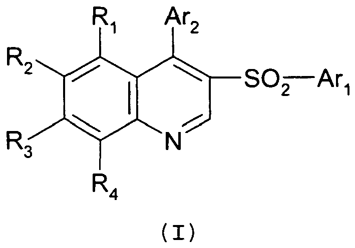 dans laquelle
Ar₁ représente un groupe phényle ou thiényle, éventuellement substitué par un ou plusieurs substituants choisis parmi l'hydrogène, fluoro, chloro, cyano, méthyle, méthoxy ;
Ar₂ représente un phényle substitué par ou plusieurs substituants choisis parmi fluoro, chloro, cyano, méthyle, méthoxy ;
R₁, R₂, R₃ et R₄ représentent indépendamment un substituant choisi parmi l'hydrogène, fluoro, chloro, cyano, méthyle, méthoxy, hydroxy, trifluorométhyle, amino, méthylamino, diméthylamino, aminométhyle, méthylaminométhyle, diméthylaminométhyle,
et/ou ses sels et/ou hydrates et/ou solvates.

2. Composé selon la revendication 1, choisi dans le groupe constitué par les suivants :
4-(4-chlorophényl)-3-(4-méthylbenzènesulfonyl)quinoline,
7-chloro-3-(4-chlorobenzènesulfonyl)-4-(4-fluorophényl)-quinoline,
8-chloro-4-(3-chlorophényl)-3-(3,4-dichlorobenzêne-sulfonyl)quinoline,
7-fluoro-3-(4-fluorobenzènesulfonyl)-4-(3-fluorophényl)-quinoline,
4-(4-chlorophényl)-7-fluoro-3-(4-méthoxybenzènesulfonyl)-quinoline,
7-fluoro-3-(4-méthoxybenzènesulfonyl)-4-(4-méthoxyphényl)-quinoline,
7-fluoro-3-(3-fluorobenzènesulfonyl)-4-(3-fluorophényl)-quinoline,
7-fluoro-3-(3-fluorobenzènesulfonyl)-4-(4-fluorophényl)-quinoline,
4-(3-chlorophényl)-3-(3,4-diméthylbenzènesulfonyl)-7-fluoroquinoline,
3-(3,4-diméthylbenzènesulfonyl)-7-fluoro-4-(3-fluorophényl)quinoline,
3-(3,4-diméthylbenzènesulfonyl)-7-fluoro-4-(3-méthoxy-phényl)quinoline,
4-(3-chlorophényl)-8-fluoro-3-(4-fluorobenzènesulfonyl)-quinoline,
4-(4-chlorophényl)-8-fluoro-3-(4-fluorobenzènesulfonyl)-quinoline,
8-fluoro-3-(4-fluorobenzènesulfonyl)-4-(3-fluorophényl)-quinoline,
8-fluoro-3-(4-fluorobenzènesulfonyl)-4-(3-méthoxyphényl)-quinoline,
4-(4-chlorophényl)-6-fluoro-3-(4-méthoxybenzènesulfonyl)-quinoline,
4-(4-chlorophényl)-3-(3,4-diméthylbenzènesulfonyl)-6-fluoroquinoline,
4-(4-chlorophényl)-3-(3,5-difluorobenzènesulfonyl)-7-fluoroquinoline,
3-(3,5-difluorobenzènesulfonyl)-7-fluoro-4-(4-fluorophényl)quinoline,
4-(4-chlorophényl)-3-(3-cyanobenzènesulfonyl)-7-fluoro-quinoline,
3-(3-cyanobenzènesulfonyl)-7-fluoro-4-(3-fluorophényl)-quinoline,
3-(3-cyanobenzènesulfonyl)-7-fluoro-4-(4-fluorophényl)-quinoline,
7-fluoro-3-(4-fluorobenzènesulfonyl)-4-(4-fluorophényl)-quinoline,
4-(4-chlorophényl)-7-fluoro-3-(3-fluorobenzènesulfonyl)-quinoline,
4-(3-chlorophényl)-7-fluoro-3-(3-méthoxybenzènesulfonyl)-quinoline,
7-fluoro-4-(4-fluorophênyl)-3-(3-méthoxybenzènesulfonyl)-quinoline,
4-(4-chlorophényl)-3-(3,4-diméthylbenzènesulfonyl)-7-fluoroquinoline,
3-(3,4-diméthylbenzènesulfonyl)-7-fluoro-4-(4-fluorophényl)quinoline,
4-(3-chlorophényl)-3-(3-chloro-4-méthoxybenzènesulfonyl)-7-fluoroquinoline,
3-(3-chloro-4-méthoxybenzènesulfonyl)-7-fluoro-4-(3-fluorophényl)quinoline,
3-(3-chloro-4-méthoxybenzènesulfonyl)-7-fluoro-4-(4-fluorophényl)quinoline,
3-(3,5-dichlorobenzènesulfonyl)-8-fluoro-4-(4-fluorophényl)quinoline,
3-(3,5-difluorobenzènesulfonyl)-8-fluoro-4-(3-fluorophényl)quinoline,
8-fluoro-3-(4-fluorobenzènesulfonyl)-4-(4-méthoxyphényl)-quinoline,
4-(3-chlorophényl)-8-fluoro-3-(4-méthoxybenzènesulfonyl)-quinoline,
4-(4-chlorophényl)-8-fluoro-3-(4-méthoxybenzènesulfonyl)-quinoline,
8-fluoro-4-(4-fluorophényl)-3-(4-méthoxybenzènesulfonyl)-quinoline,
8-fluoro-3-(4-méthoxybenzènesulfonyl)-4-(4-méthoxyphényl)-quinoline,
8-fluoro-3-(4-méthoxybenzènesulfonyl)-4-(3-méthoxyphényl)-quinoline,
4-(3-chlorophényl)-8-fluoro-3-(3-fluorobenzènesulfonyl)-quinoline,
4-(4-chlorophényl)-8-fluoro-3-(3-fluorobenzènesulfonyl)-quinoline,
8-fluoro-3-(3-fluorobenzènesulfonyl)-4-(3-fluorophényl)-quinoline,
8-fluoro-3-(3-fluorobenzènesulfonyl)-4-(4-méthoxyphényl)-quinoline,
8-fluoro-3-(3-fluorobenzènesulfonyl)-4-(3-méthoxyphényl)-quinoline,
8-fluoro-3-(3-fluorobenzènesulfonyl)-4-(4-fluorophényl)-quinoline,
3-(3-cyanobenzènesulfonyl)-6-fluoro-4-(3-fluorophényl)-quinoline,
3-(3-cyanobenzènesulfonyl)-6-fluoro-4-(3-méthoxyphényl)-quinoline,
3- (3-cyanobenzènesulfonyl) -6-fluoro-4- (4-fluorophényl) - quinoline,
4-(3-chlorophényl)-6-fluoro-3-(3-méthoxybenzènesulfonyl)-quinoline,
6-fluoro-4-(4-fluorophényl)-3-(3-méthoxybenzènesulfonyl)-quinoline,
3-(3-chloro-4-méthoxybenzènesulfonyl)-6-fluoro-4-(4-méthoxyphényl)quinoline,
3-(3-chloro-4-méthoxybenzènesulfonyl)-6-fluoro-4-(3-méthoxyphényl)quinoline,
4-(4-chlorophényl)-7-fluoro-3-(4-fluorobenzènesulfonyl)-quinoline,
3-(3,4-diméthylbenzènesulfonyl)-7-fluoro-4-(4-méthoxy-phényl)quinoline,
3-(3-chloro-4-méthoxybenzènesulfonyl)-4-(4-chlorophényl)-6-fluoroquinoline,
3-(3-chloro-4-méthoxybenzènesulfonyl)-6-fluoro-4-(3-fluorophényl)quinoline,
3-(3,5-dichlorobenzènesulfonyl)-7-fluoro-4-(4-méthoxy-phényl)quinoline,
3-(3,5-dichlorobenzènesulfonyl)-7-fluoro-4-(3-méthoxy-phényl)quinoline,
3-(3,5-difluorobenzènesulfonyl)-7-fluoro-4-(4-fluorophényl)quinoline,
4-(3-chlorophényl)-7-fluoro-3-(3-fluoro-4-méthylbenzène-sulfonyl)quinoline,
7-fluoro-3-(3-fluoro-4-méthylbenzènesulfonyl)-4-(3-fluorophényl)quinoline,
7-fluoro-3-(3-fluoro-4-méthylbenzènesulfonyl)-4-(4-fluorophényl)quinoline,
3- (3, 5-difluorobenzènesulfonyl)-7-fluoro-4- (3-fluorophényl)quinoline,
3-(3,5-difluorobenzènesulfonyl)-7-fluoro-4-(4-méthoxy-phényl)quinoline,
3- (3, 5-difluorobenzènesulfonyl) -7-fluoro-4- (3-méthoxy-phényl)quinoline,
4-(3-chlorophényl)-3-(3-cyanobenzènesulfonyl)-7-fluoro-quinoline,
3-(3-cyanobenzènesulfonyl)-7-fluoro-4-(4-méthoxyphényl)-quinoline,
3-(3-cyanobenzènesulfonyl)-7-fluoro-4-(3-méthoxyphényl)-quinoline,
3-(3-chloro-4-méthylbenzènesulfonyl)-7-fluoro-4-(3-fluorophényl)quinoline,
3-(3,4-difluorobenzènesulfonyl)-7-fluoro-4-(3-fluorophényl)quinoline,
3-(3,4-difluorobenzènesulfonyl)-7-fluoro-4-(4-méthoxy-phényl)quinoline,
3-(3,4-difluorobenzènesulfonyl)-7-fluoro-4-(3-méthoxy-phényl)quinoline,
3- (3, 4-difluorobenzènesulfonyl) -7-fluoro-4- (4-fluorophényl)quinoline,
3-(3-chloro-4-fluorobenzènesulfonyl)-4-(4-chlorophényl)-7-fluoroquinoline,
3-(3-chloro-4-fluorobenzènesulfonyl)-7-fluoro-4-(3-fluorophényl)quinoline,
3-(3-chloro-4-fluorobenzènesulfonyl)-7-fluoro-4-(4-méthoxyphényl)quinoline,
3-(3-chloro-4-fluorobenzènesulfonyl)-7-fluoro-4-(4-fluorophényl)quinoline,
3-(3,5-dichlorobenzènesulfonyl)-8-fluoro-4-(3-fluorophényl)quinoline,
3-(3,5-dichlorobenzènesulfonyl)-8-fluoro-4-(4-méthoxy-phényl)quinoline,
3-(3,5-dichlorobenzènesulfonyl)-8-fluoro-4-(3-méthoxy-phényl)quinoline,
4-(3-chlorophényl)-8-fluoro-3-(3-fluoro-4-méthylbenzène-sulfonyl)quinoline,
4-(4-chlorophényl)-8-fluoro-3-(3-fluoro-4-méthylbenzène-sulfonyl)quinoline,
8-fluoro-3-(3-fluoro-4-méthylbenzènesulfonyl)-4-(3-fluorophényl)quinoline,
8-fluoro-3-(3-fluoro-4-méthylbenzènesulfonyl)-4-(4-méthoxyphényl)quinoline,
8-fluoro-3-(3-fluoro-4-méthylbenzènesulfonyl)-4-(3-méthoxyphényl)quinoline,
8-fluoro-3-(3-fluoro-4-méthylbenzènesulfonyl)-4-(4-fluorophényl)quinoline,
4-(3-chlorophényl)-3-(3,5-difluorobenzènesulfonyl)-8-fluoroquinoline,
4-(4-chlorophényl)-3-(3,5-difluorobenzènesulfonyl)-8-fluoroquinoline,
3-(3,5-difluorobenzènesulfonyl)-8-fluoro-4-(4-méthoxy-phényl)quinoline,
3-(3,5-difluorobenzènesulfonyl)-8-fluoro-4-(3-méthoxy-phényl)quinoline,
3-(3,5-difluorobenzènesulfonyl)-8-fluoro-4-(4-fluorophényl)quinoline,
4-(4-chlorophényl)-3-(3,4-difluorobenzènesulfonyl)-8-fluoroquinoline,
3-(3,4-difluorobenzènesulfonyl)-8-fluoro-4-(3-fluorophényl)quinoline,
3-(3,4-difluorobenzènesulfonyl)-8-fluoro-4-(4-méthoxy-phényl)quinoline,
3-(3,4-difluorobenzènesulfonyl)-8-fluoro-4-(4-fluorophényl)quinoline,
3-(3,5-dichlorobenzènesulfonyl)-6-fluoro-4-(3-fluorophényl)quinoline,
3-(3,5-dichlorobenzènesulfonyl)-6-fluoro-4-(4-méthoxy-phényl)quinoline,
3-(3,5-dichlorobenzènesulfonyl)-6-fluoro-4-(3-méthoxy-phényl)quinoline,
4-(3-chlorophényl)-6-fluoro-3-(3-fluoro-4-méthylbenzène-sulfonyl)quinoline,
6-fluoro-3-(3-fluoro-4-méthylbenzènesulfonyl)-4-(3-fluorophényl)quinoline,
6-fluoro-3-(3-fluoro-4-méthylbenzènesulfonyl)-4-(4-méthoxyphényl)quinoline,
6-fluoro-3-(3-fluoro-4-méthylbenzènesulfonyl)-4-(3-méthoxyphényl)quinoline,
6-fluoro-3-(3-fluoro-4-méthylbenzènesulfonyl)-4-(4-fluorophényl)quinoline,
4-(3-chlorophényl)-3-(3-cyanobenzènesulfonyl)-6-fluoro-quinoline,
4-(4-chlorophényl)-3-(3-cyanobenzènesulfonyl)-6-fluoro-quinoline,
3-(3-cyanobenzènesulfonyl)-6-fluoro-4-(4-méthoxyphényl)-quinoline,
3-(3-chloro-4-méthylbenzènesulfonyl)-4-(3-chlorophényl)-6-fluoroquinoline,
3-(3-chloro-4-méthylbenzènesulfonyl)-4-(4-chlorophényl)-6-fluoroquinoline,
3-(3-chloro-4-méthylbenzènesulfonyl)-6-fluoro-4-(3-fluorophényl)quinoline,
3-(3-chloro-4-méthylbenzènesulfonyl)-6-fluoro-4-(3-méthoxyphényl)quinoline,
3-(3-chloro-4-méthylbenzènesulfonyl)-6-fluoro-4-(4-fluorophényl)quinoline,
4-(4-chlorophényl)-6-fluoro-3-(3-méthoxybenzènesulfonyl)-quinoline,
6-fluoro-3-(3-méthoxybenzènesulfonyl)-4-(3-méthoxyphényl)-quinoline,
3-(3,4-diméthylbenzènesulfonyl)-6-fluoro-4-(3-fluorophényl)quinoline,
3-(3,4-diméthylbenzènesulfonyl)-6-fluoro-4-(4-méthoxy-phényl)quinoline,
3-(3,4-diméthylbenzènesulfonyl)-6-fluoro-4-(4-fluorophényl)quinoline,
3-(3,4-difluorobenzènesulfonyl)-6-fluoro-4-(3-fluorophényl)quinoline,
3-(3,4-difluorobenzènesulfonyl)-6-fluoro-4-(4-fluorophényl)quinoline,
3-(3-chloro-4-fluorobenzènesulfonyl)-4-(4-chlorophényl)-6-fluoroquinoline,
3-(3-chloro-4-fluorobenzènesulfonyl)-6-fluoro-4-(3-méthoxyphényl)quinoline,
3-(3-chloro-4-fluorobenzènesulfonyl)-6-fluoro-4-(4-fluorophényl)quinoline,
4-(3-chlorophényl)-3-(3,5-dichlorobenzènesulfonyl)-7-fluoroquinoline,
4-(4-chlorophényl)-3-(3,5-dichlorobenzènesulfonyl)-7-fluoroquinoline,
4-(3-chlorophényl)-3-(3,5-dichlorobenzènesulfonyl)-8-fluoroquinoline,
4-(4-chlorophényl)-3-(3,5-dichlorobenzènesulfonyl)-8-fluoroquinoline,
4-(3-chlorophényl)-3-(3,5-dichlorobenzênesulfonyl)-6-fluoroquinoline,
4-(4-chlorophényl)-3-(3,5-dichlorobenzènesulfonyl)-6-fluoroquinoline,
6-fluoro-3-(4-méthoxybenzènesulfonyl)-4-(4-méthoxyphényl)-quinoline,
7-chloro-4-(4-chlorophényl)-3-(3,5-difluorobenzènesulfonyl)quinoline,
7-chloro-4-(4-chlorophényl)-3-(3,4-difluorobenzènesulfonyl)quinoline,
7-chloro-4-(4-chlorophényl)-3-(3-cyanobenzènesulfonyl)-quinoline,
7-chloro-3-(3,5-dichlorobenzènesulfonyl)-4-(4-fluorophényl)quinoline,
7-chloro-3-(4-fluorobenzènesulfonyl)-4-(4-fluorophényl)-quinoline,
7-chloro-3-(3-fluorobenzènesulfonyl)-4-(4-fluorophényl)-quinoline,
7-chloro-3-(3,4-difluorobenzènesulfonyl)-4-(4-fluorophényl)quinoline,
7-chloro-3-(3-chloro-4-fluorobenzènesulfonyl)-4-(4-fluorophényl)quinoline et
7-chloro-3-(3-cyano-5-fluorobenzènesulfonyl)-4-(4-fluorophényl)quinoline,
6-chloro-3-(3-chloro-4-fluorobenzènesulfonyl)-4-(4-chloro-phényl)-7-fluoroquinoline,
6-chloro-3-(3-chloro-4-fluorobenzènesulfonyl)-7-fluoro-4-(4-fluorophényl) quinoline,
3-(3-chloro-4-fluorobenzènesulfonyl)-7-cyano-4-(2-fluorophényl)quinoline,
7-chloro-3-(3,4-difluorobenzènesulfonyl)-8-fluoro-4-(3-fluorophényl)quinoline,
7-chloro-3-(3-chloro-4-fluorobenzènesulfonyl)-4-(4-fluorophényl)-8-fluoroquinoline,
7-chloro-3-(3-chloro-4-fluorobenzènesulfonyl)-8-fluoro-4-(3-fluorophényl)quinoline,
7-chloro-4-(4-chlorophényl)-3-(3,4-difluorobenzènesulfonyl)-8-fluoroquinoline,
7-chloro-3-(3-chloro-4-fluorobenzènesulfonyl)-8-fluoro-4-(4-fluorophényl)quinoline,
3-(3-cyano-4-fluorobenzènesulfonyl)-4-(3-fluorophényl)-quinoline,
3-(3-cyano-5-fluorobenzènesulfonyl)-4-(3-fluorophényl)-quinoline,
3-(3-chloro-4-fluorobenzènesulfonyl)-4-(4-chlorophényl)-8-fluoroquinoline,
3-(3-chloro-4-fluorobenzènesulfonyl)-8-fluoro-4-(2-fluorophényl)quinoline,
3-(3-chloro-4-fluorobenzênesulfonyl)-8-fluoro-4-(3-fluorophényl)quinoline,
3-(3-cyano-4-fluorobenzènesulfonyl)-4-(4-fluorophényl-)quinoline,
3-(3-chloro-4-fluorobenzènesulfonyl)-8-fluoro-4-(4-fluorophényl)quinoline,
3-(3-cyanobenzènesulfonyl)-8-fluoro-4-(2-fluorophényl)-quinoline,
3-(3-cyanobenzènesulfonyl)-4-(3-fluorophényl)quinoline,
3-(3-cyanobenzènesulfonyl)-8-fluoro-4-(3-fluorophényl)-quinoline,
3-(3-cyano-4-fluorobenzènesulfonyl)-7-fluoro-4-(3-fluorophényl)quinoline,
4-(3-chlorophényl)-3-(3-cyano-4-fluorobenzènesulfonyl)-quinoline,
3-(3-cyano-5-fluorobenzènesulfonyl)-7-fluoro-4-(4-fluorophényl)quinoline,
7-chloro-3-(3-cyano-4-fluorobenzènesulfonyl)-4-(3-fluorophényl)quinoline,
3-(3,5-dichlorobenzènesulfonyl)-4-(3,4-difluorophényl)-8-fluoroquinoline,
7-chloro-3-(3-cyano-5-fluorobenzènesulfonyl)-4-(3-fluorophényl)quinoline,
3-(3-cyanobenzènesulfonyl)-8-fluoro-4-(4-fluorophényl)-quinoline,
3-(3,5-dichlorobenzènesulfonyl)-8-fluoro-4-(2-fluorophényl)quinoline,
3-(3-chloro-4-fluorobenzènesulfonyl)-7-fluoro-4-(2-fluorophényl)quinoline,
4-(4-chlorophényl)-3-(3-cyanobenzènesulfonyl)-8-fluoro-quinoline,
4-(3-chlorophényl)-3-(3-cyano-5-fluorobenzènesulfonyl)-quinoline,
7-chloro-3-(3-cyanobenzènesulfonyl)-4-(2-fluorophényl)-quinoline,
3-(3-cyano-5-fluorobenzènesulfonyl)-7-fluoro-4-(3-fluorophényl)quinoline,
3-(3-chloro-4-fluorobenzènesulfonyl)-4-(3-chlorophényl)-8-fluoroquinoline,
3-(3-cyanobenzènesulfonyl)-7-fluoro-4-(2-fluorophényl)-quinoline,
3-(3-cyano-5-fluorobenzènesulfonyl)-4-(3,4-difluoro-phényl)-7-fluoroquinoline,
3-(3-cyano-4-fluorobenzènesulfonyl)-4-(3,4-dichloro-phényl)quinoline,
7-chloro-3-(3-chloro-4-fluorobenzènesulfonyl)-4-(2-fluorophényl)quinoline,
7-chloro-3-(3-cyanobenzènesulfonyl)-4-(3-fluorophényl)-quinoline,
4-(3-chlorophényl)-3-(3-cyano-4-fluorobenzènesulfonyl)-7-fluoroquinoline,
3-(3,4-difluorobenzènesulfonyl)-4-(3,5-difluorophényl)-8-fluoroquinoline,
3-(3,4-difluorobenzènesulfonyl)-4-(4-fluorophényl)-quinoline,
3-(3,4-difluorobenzènesulfonyl)-4-(3,4-difluorophényl)-8-fluoroquinoline,
3-(3,5-dichlorobenzènesulfonyl)-4-(3,4-difluorophényl)-7-fluoroquinoline,
3-(3,4-difluorobenzènesulfonyl)-4-(3-fluorophényl)-quinoline,
7-chloro-3-(3-cyanobenzènesulfonyl)-4-(4-fluorophényl)-quinoline,
7-chloro-4-(3-chlorophényl)-3-(3-cyano-4-fluorobenzène-sulfonyl)quinoline,
3-(3,4-difluorobenzènesulfonyl)-8-fluoro-4-(2-fluorophényl)quinoline,
4-(3-chlorophényl)-3-(3-cyano-5-fluorobenzènesulfonyl)-7-fluoroquinoline,
3-(3-cyanobenzènesulfonyl)-4-(3,5-difluorophényl)-7-fluoroquinoline,
4-(3-chlorophényl)-3-(3-cyanobenzènesulfonyl)-8-fluoro-quinoline,
4-(4-chlorophényl)-3-(3,4-difluorobenzènesulfonyl)-7-fluoroquinoline,
7-chloro-3-(3,4-dichlorobenzènesulfonyl)-4-(3,4-difluoro-phényl)quinoline,
3-(3,5-dicyanobenzènesulfonyl)-7-fluoro-4-(4-fluorophényl)quinoline,
7-chloro-3-(3-chloro-5-fluorobenzènesulfonyl)-4-(3-chloro-phényl)quinoline,
3-(3,4-difluorobenzênesulfonyl)-4-(3,4-difluorophényl)-7-fluoroquinoline,
7-chloro-4-(3-chlorophényl)-3-(3,5-dichlorobenzène-sulfonyl)-8-fluoroquinoline,
7-chloro-4-(3-chlorophényl)-3-(3-cyanobenzènesulfonyl)-8-fluoroquinoline,
7-chloro-4-(3-chlorophényl)-3-(3-cyanobenzènesulfonyl)-quinoline,
7-chloro-3-(3,5-dichlorobenzènesulfonyl)-4-(2-fluorophényl)quinoline,
3-(3,5-dicyanobenzènesulfonyl)-4-(3,4-difluorophényl)-7-fluoroquinoline,
3-(3,5-dichlorobenzènesulfonyl)-7-fluoro-4-(2-fluorophényl)quinoline,
3-(3-chloro-4-fluorobenzènesulfonyl)-4-(3-chlorophényl)-7-fluoroquinoline,
4-(3-chlorophényl)-3-(3,4-difluorobenzènesulfonyl)-8-fluoroquinoline,
4-(3,4-dichlorophényl)-3-(3,4-difluorobenzènesulfonyl)-quinoline,
3-(3,4-difluorobenzènesulfonyl)-7-fluoro-4-(2-fluorophényl)quinoline,
7-chloro-3-(3-chloro-4-fluorobenzènesulfonyl)-4-(3,5-difluorophényl)quinoline,
7-chloro-4-(3-chlorophényl)-3-(3,5-dichlorobenzène-sulfonyl)quinoline,
7-amino-3-(3,4-difluorobenzènesulfonyl)-4-(3-fluorophényl)quinoline,
4-(3-chlorophényl)-3-(3-cyanobenzènesulfonyl)quinoline,
3-(3-cyano-5-fluorobenzènesulfonyl)-4-(3,4-difluoro-phényl)quinoline,
3-(3-cyano-5-fluorobenzènesulfonyl)-7-fluoro-4-(4-fluorophényl)quinoline,
4-(4-chlorophényl)-3-(3-cyano-5-fluorobenzènesulfonyl)-7-fluoroquinoline,
7-chloro-3-(3-chloro-5-fluorobenzènesulfonyl)-4-(4-chlorophényl)quinoline,
7-chloro-3-(3-chloro-5-fluorobenzènesulfonyl)-4-(4-fluorophényl)quinoline,
3-(3-chloro-5-fluorobenzènesulfonyl)-4-(4-fluorophényl)-quinoline,
3-(3-chloro-5-fluorobenzènesulfonyl)-4-(4-chlorophényl)-quinoline,
3-(3-chloro-4-fluorobenzènesulfonyl)-4-(4-chlorophényl)-quinoline,
3-(3-chloro-4-fluorobenzènesulfonyl)-4-(4-chlorophényl)-7-fluoroquinoline,
3-(3-chloro-4-fluorobenzènesulfonyl)-7-fluoro-4-(4-fluorophényl)quinoline,
7-chloro-3-(3-chloro-4-fluorobenzènesulfonyl)-4-(4-fluorophényl)quinoline,
7-chloro-3-(3-chloro-4-fluorobenzênesulfonyl)-4-(4-chloro-phényl)quinoline,
7-amino-3-(3-chloro-5-fluorobenzènesulfonyl)-4-(4-fluorophényl)quinoline,
7-amino-3-(3-chloro-5-fluorobenzènesulfonyl)-4-(3-fluorophényl)quinoline,
et/ou leurs sels et/ou hydrates et/ou solvates.

3. Procédé pour la préparation d'un composé de formule (I) : 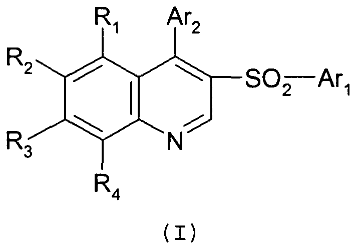 dans laquelle Ar₁, Ar₂, R₁, R₂, R₃ et R₄ sont tels que définis dans la revendication 1, et/ou de ses sels et/ou hydrates et/ou solvates,
a.
a1. par réaction d'un composé de formule (II) : 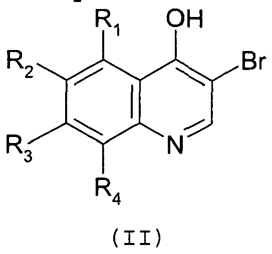 dans laquelle R₁, R₂, R₃ et R₄ sont tels que définis ci-dessus à propos du composé de formule (I), avec un composé de formule (III) :
Ar₁-S⁻M⁺ (III)
dans laquelle Ar₁ est tel que défini ci-dessus à propos des composés de formule (I), M est choisi parmi les métaux alcalins et les métaux alcalino-terreux, pour former un composé de formule (IV) : 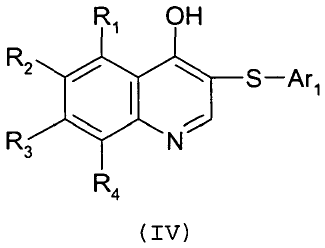 dans laquelle Ar₁, R₁, R₂, R₃ et R₄ sont tels que définis ci-dessus à propos des composés de formule (I), puis oxydation du composé de formule (IV) pour former un composé de formule (V) : 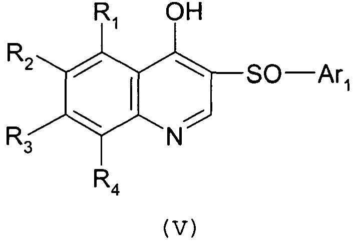 dans laquelle Ar₁, R₁, R₂, R₃ et R₄ sont tels que définis ci-dessus à propos des composés de formule (I), puis oxydation du composé de formule (V) pour former un composé de formule (VI) : 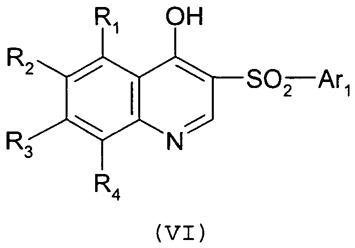 dans laquelle Ar₁, R₁, R₂, R₃ et R₄ sont tels que définis ci-dessus à propos des composés de formule (I), ou
a2. par réaction d'un composé de formule (IX) :
Ar₁-SO₂Na (IX)
dans laquelle Ar₁ est tel que défini ci-dessus à propos des composés de formule (I), avec un ester d'acide α-halogénoacétique de formule (X) :
Hlg-CH₂-COOR₅ (X)
dans laquelle Hlg est un halogène et R₅ est un groupe éthyle ou méthyle, pour former un composé de formule (XI) :
Ar₁-SO₂-CH₂-COOR₅ (XI)
dans laquelle Ar₁ est tel que défini ci-dessus à propos du composé de formule (I) et R₅ est tel que défini ci-dessus à propos des composés de formule (X), ensuite réaction du composé de formule (XI) avec un orthoformiate de trialkyle de formule (XII) :
CH(OR₆)₃ (XII)
dans laquelle R₆ est un groupe éthyle ou méthyle, pour former un composé de formule (XIII) : 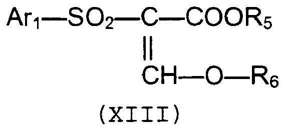 dans laquelle Ar₁ est tel que défini ci-dessus à propos du composé de formule (I), R₅ est tel que défini ci-dessus à propos des composés de formule (X), et R₆ est tel que défini ci-dessus à propos des composés de formule (XII), ensuite réaction du composé de formule (XIII) avec un dérivé d'aniline de formule (XIV) : 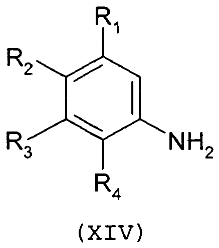 dans laquelle R₁, R₂, R₃ et R₄ sont tels que définis ci-dessus à propos du composé de formule (I), pour former un composé de formule (VI) : 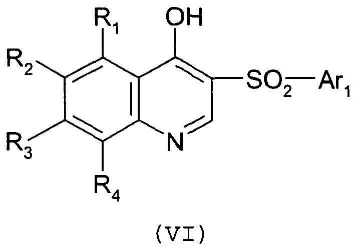 dans laquelle Ar₁, R₁, R₂, R₃ et R₄ sont tels que définis ci-dessus à propos du composé de formule (I),
ensuite conversion du composé de formule (VI) en un composé de formule (VII) : 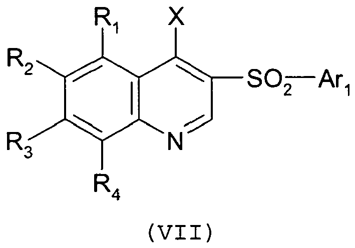 dans laquelle Ar₁, R₁, R₂, R₃ et R₄ sont tels que définis ci-dessus à propos des composés de formule (I), X est choisi parmi les groupes chloro, bromo, benzènesulfonyloxy, 4-fluorobenzènesulfonyloxy, 4-méthylbenzènesulfonyloxy, méthanesulfonyloxy et trifluoro-méthanesulfonyloxy,
ensuite réaction du composé obtenu de formule (VII) avec un dérivé d'acide boronique de formule (VIII) :
Ar₂-B(OH)₂ (VIII)
dans laquelle Ar₂ est tel que défini ci-dessus à propos du composé de formule (I), en présence d'une base et d'un catalyseur dans un solvant,
et éventuellement ensuite formation de sels et/ou hydrates et/ou solvates de composés de formule (I), ou b. par interconversion d'un composé de formule (I), dans laquelle Ar₁, Ar₂, R₁, R₂, R₃ et R₄ sont tels que définis ci-dessus à propos de la formule (I), en un composé différent de formule (I), dans laquelle Ar₁, Ar₂, R₁, R₂, R₃ et R₄ sont tels que définis ci-dessus à propos de la formule (I),
et éventuellement ensuite formation de sels et/ou hydrates et/ou solvates de composés de formule (I).

4. Formulation pharmaceutique comprenant une quantité efficace, du point de vue thérapeutique, d'un composé de formule (I) tel que défini dans la revendication 1, et/ou de sels physiologiquement acceptables et/ou hydrates et/ou solvates de celui-ci, en association avec un ou plusieurs diluants, excipients et/ou véhicules inertes physiologiquement acceptables.

5. Formulation pharmaceutique telle que définie dans la revendication 4, pour une utilisation dans un procédé pour prévenir et/ou traiter un trouble choisi parmi les troubles psychiatriques, les troubles neurologiques, la douleur chronique ou aiguë, les dysfonctionnements neuromusculaires des voies urinaires inférieures, les maladies de reflux gastro-intestinal, le syndrome du côlon irritable, la toxicomanie et la désintoxication.

6. Composé de formule (I) tel que défini dans la revendication 1 ou 2 et/ou ses sels physiologiquement acceptables et/ou hydrates et/ou solvates, pour une utilisation dans un procédé pour prévenir et/ou traiter un trouble choisi parmi les troubles psychiatriques, les troubles neurologiques, la douleur chronique ou aiguë, les dysfonctionnements neuromusculaires des voies urinaires inférieures, les maladies de reflux gastro-intestinal, le syndrome du côlon irritable, la toxicomanie et la désintoxication.
